# EUROPEAN PATENT APPLICATION

(11) **EP 1 226 752 A1**
(43) Date of publication of application: **31.07.2002**
(21) Application number: 00971708.3
(22) Date of filing: 01.11.2000
(51) Int. Cl.: A01K 67/027, G01N 33/50, G01N 33/15, C12N 5/10, C12N 15/09, C12N 15/63, C12Q 1/02

(54) **TRANSGENIC NON-HUMAN MAMMALS FOR MONITORING CHANGE IN CALCIUM ION CONCENTRATION IN CELLS**

(30) Priority: 05.11.1999 JP 31451299
(71) Applicant: KYOWA HAKKO KOGYO CO., LTD., Chiyoda-ku, Tokyo 100-8185 (JP)
(72) Inventor: MORI, Katsuhiro, Kyowa Hakko Kogyo Ltd, Machida, Tokyo 194-8533 (JP); SATO, Mitsuo, Kyowa Hakko Kogyo Ltd, Machida-shi, Tokyo 194-8533 (JP); SEKINE, Susumu, Kyowa Hakko Kogyo Ltd, Machida-shi, Tokyo 194-8533 (JP)
(74) Representative: Sternagel, Fleischer, Godemeyer & Partner Patentanwälte
(86) International application number: JP0007681
(87) International publication number: WO0133957

(57) **Abstract**

The present invention provides a transgenic non-human mammal which has DNA encoding a foreign reporter protein for monitoring changes in intracellular calcium ion concentration and expresses the reporter protein in one or more types of non-neuronal cells; a method for producing the above transgenic non-human mammal; a method for detecting changes in intracellular calcium ion concentration using the above transgenic non-human mammal; DNA having promoter activity which is used for production of the transgenic non-human mammal; and the method of use of the DNA. The non-human mammal of the present invention expresses the DNA in one or more tissues or organs selected from the group consisting of epithelial tissue, connective tissue, adipose tissue, cartilage tissue, bone tissue, muscle tissue, intraoral tissue, blood tissue, bone marrow, cardiovascular organ, lymphatic organ, teeth, digestive tractl, liver, gallbladder, pancreas, respiratory organ, urinary organ, genital organ, endocrine organ, sense organ and nail, and can be used for evaluation of pharmacological effect and activity of physiologically active substances (for example, drug), novel gene products having unknown functions and the like in these tissues or organs.

SEQUENCE LISTING
<110> KYOWA HAKKO KOGYO CO., LTD.
<120> A transgenic non-human mammalian animal for monitoring the change of
the calcium ion concentration in cells
<130> A01394MA
<150> JP 11-314512
<151> 1999-11-5
<160> 7

<210> 1
<211> 5581
<212> DNA
<213> Artificial Sequence
<220>
<223> Description of Artificial Sequence: DNA constructed from  DNAs with
different origin
<220>
<222> (1)..(48)
<223> Synthetic DNA containing loxP sequence
<220>
<222> (48)..(56)
<223> DNA originated from pKO plasmid
<220>
<222> (56)..(3732)
<223> DNA containing PGK promoter and hprt gene
<220>
<222> (3732)..(3741)

<223> DNA originated from pKO plasmid

<220>

<222> (3741)..(3787)

<223> Synthetic DNA containing loxP sequence

<220>

<222> (3787)..(3830)

<223> DNA originated from pKO plasmid

<220>

<221> promoter

<222> (3830)..(5558)

<223> DNA containing CAG promoer

<220>

<222> (5558)..(5575)

<223> DNA originated from pBluescritIIKS(+) plasmid

<400> 1

ggtaccgcgg ccgcataact tcgtataatg tatgctatac gaagttatct cgaggggcgc 60

gccctaccgg gtaggggagg cgcttttccc aaggcagtct ggagcatgcg ctttagcagc 120

cccgctgggc acttggcgct acacaagtgg cctctggcct cgcacacatt ccacatccac 180

cggtaggcgc caaccggctc cgttctttgg tggccccttc gcgccacctt ctactcctcc 240

cctagtcagg aagttccccc ccgccccgca gctcgcgtcg tgcaggacgt gacaaatgga 300

agtagcacgt ctcactagtc tcgtgcagat ggacagcacc gctgagcaat ggaagcgggt 360

aggcctttgg ggcagcggcc aatagcagct ttggctcctt cgctttctgg gctcagaggc 420

tgggaagggg tgggtccggg ggcgggctca ggggcgggct caggggcggg gcgggcgccc 480

gaaggtcctc cggaagcccg gcattctgca cgcttcaaaa gcgcacgtct gccgcgctgt 540

tctcctcttc ctcatctccg ggcctttcga cctgcagggg ggggggggtc ttgctgcgcc 600

tccgcctcct cctctgctcc gccaccggct tcctcctcct gaacaatcag cccgcgcgcc 660

ggccggctcc gttatggcga cccgcagccc tggcgtcgtg attagtgatg atgaaccagg 720

ttatgacctt gatttatttt gcatacctaa tcattatgct gaggatttgg aaagggtgtt 780
tattccccat ggactaatta tggacaggta agtaagatct taaaatgagg ttttttactt 840
tttcttgtgt taatttcaaa catcagcagc tgttctgagt acttgctatt tgaacataaa 900
ctaggccaac ttattaaata actgatgctt tctaaaatct tctttattaa aaataaaaga 960
ggagggcctt actaattact tagtatcagt tgtggtatag tgggactctg tagggaccag 1020
aacaaagtaa acattgaagg gagatggaag aaggaactct agccagagtc ttgcatttct 1080
cagtcctaaa cagggtaatg gactggggct gaatcacatg aaggcaaggt cagattttta 1140
ttattatgca catctagctt gaaaattttc tgttaagtca attacagtga aaaaccttac 1200
ctggtattga atgcttgcat tgtatgtctg gctattctgt gtttttattt taaaattata 1260
atatcaaaat atttgtgtta taaaatattc taactatgga ggccataaac aagaagacta 1320
aagttctctc ctttcagcct tctgtacaca tttcttctca agcactggcc tatgcatgta 1380
tactatatgc aaaagtacat atatacattt atattttaac gtatgagtat agttttaaat 1440
gttattggac acttttaata ttagtgtgtc tagagctatc taatatattt taaaggttgc 1500
atagcattct gtcttatgga gataccataa ctgatttaac cagtccacta ttgatagaca 1560
ctattttgtt cttaccgact gtactagaag aaacattctt ttacatgttt ggtacttgtt 1620
cagctttatt caagtggaat ttctgggtca aggggaaaga gtttattgaa tattttggta 1680
ttgccaaatt ttcctctaag aagttgaatc attttatact cctgatgtta tatgagagta 1740
cctttctctt cacaatttgt ctcttttttt tttttttttg agacaaggtc tctgttgccc 1800
aggctggggt gcagtgcagc agaatgatca cagttcactg cagtctcaac ctcctgggtt 1860
caagcgatcc ttccacctca gcctcctgag tagctgggac tataggtgtg cgccaccaat 1920
cccagctaat atttttattt tgtagaaaca gggttcgcca tgttacccag cctcccaaag 1980
tgctgggatt acaggcatga gccactggcc cagtttatac agtctctctt aatatggtat 2040
attatccaga aaatttcatt taatcagaac ctgccagtct gataggtgaa aatggtatct 2100
tgtttttatt tgcatttaaa aaaaattatg atagtggtat gcttggtttt tttgaaggta 2160
tcaaattttt taccttatga aacatgaggg caaaggatgt gttacgtgga agatttaaaa 2220
aaaattttta atgcattttt ttgagacaag gtcttgctct attgtccagg ctggagtgca 2280
gtggcacaat cacagttcac tccagcctca acatcctgca ctaaagtgat tttcccacct 2340

cacttctcaa gtagctggga ctacaggtac atgctaccat gcctggctaa tttttttttt 2400
tttgcaggca tggggtctca ctatattgcc caggttggtg tggaagttta atgactaaga 2460
ggtgtttgtt ataaagttta atgtatgaaa ctttctatta aattcctgat tttatttctg 2520
taggactgga ctgaacgtct tgctcgagat gtgatgaagg agatgggggg ccatcacatt 2580
gtagccctct gtgtgctcaa ggggggctat aaattctttg ctgacctgct ggattacatc 2640
aaagcactga atagaaatag tgatagatcc attcctatga ctgtagattt tatcagactg 2700
aagagctatt gtaatgacca gtcaacaggg gacataaaag taattggtgg agatgatctc 2760
tcaactttaa ctggaaagaa tgtcttgatt gtggaagata taattgacac tggcaaaaca 2820
atgcagactt tgctttcctt ggtcaggcag tataatccaa agatggtcaa ggtcgcaagc 2880
ttgctggtga aaaggacccc acgaagtgtt ggatataagc cagactttgt tggatttgaa 2940
attccagaca agtttgttgt aggatatgcc cttgactata atgaatactt cagggatttg 3000
aatcatgttt gtgtcattag tgaaactgga aaagcaaaat acaaagccta agatgagagt 3060
tcaagttgag tttggaaaca tctggagtcc tattgacatc gccagtaaaa ttatcaatgt 3120
tctagttctg tggccatctg cttagtagag ctttttgcat gtatcttcta agaattttat 3180
ctgttttgta ctttagaaat gtcagttgct gcattcccta aactgtttat ttgccctatg 3240
agcctataga ctatcagttc cctttgggcg gattgttgtt taacttgtaa atgaaaaaat 3300
tctcttaaac cacagcacta ttgagtgaaa cattgaactc atatctgtaa ggaaataaag 3360
agaagatata ttagtttttt aattggtatt ttaattttta tatatgcagg aaagaataga 3420
agtgattgaa tattgttaat tataccaccg tgtgttagaa aagtaagaag cagtcaattt 3480
tcacatcaaa gacagcatct aagaagtttt gttctgtcct ggaattattt tagtagtgtt 3540
tcagtaatgt tgactgtatt ttccaacttg ttcaaattat taccagtgaa tctttgtcag 3600
cagttccctt ttaaatgcaa atcaataaat tcccaaaaat ttaaaaaaaa aaaaaaaaaa 3660
aaaaaaaaaa aaaaaaaaaa aaaaaaaaaa aaaaaaaaaa aagtaccttc tgaggcggaa 3720
agaaccagcc tggcgcgccg ggatctataa cttcgtataa tgtatgctat acgaagttat 3780
ggatccatcg atcctagaga attcctagac cgcggctaga cccggggggg tcgacattga 3840
ttattgacta gttattaata gtaatcaatt acggggtcat tagttcatag cccatatatg 3900
gagttccgcg ttacataact tacggtaaat ggcccgcctg gctgaccgcc caacgacccc 3960

cgcccattga cgtcaataat gacgtatgtt cccatagtaa cgccaatagg gactttccat 4020
tgacgtcaat gggtggacta tttacggtaa actgcccact tggcagtaca tcaagtgtat 4080
catatgccaa gtacgccccc tattgacgtc aatgacggta aatggcccgc ctggcattat 4140
gcccagtaca tgaccttatg ggactttcct acttggcagt acatctacgt attagtcatc 4200
gctattacca tgggtcgagg tgagccccac gttctgcttc actctcccca tctccccccc 4260
ctccccaccc ccaattttgt atttatttat tttttaatta ttttgtgcag cgatgggggc 4320
gggggggggg ggggcgcgcg ccaggcgggg cggggcgggg cgaggggcgg ggcggggcga 4380
ggcggagagg tgcggcggca gccaatcaga gcggcgcgct ccgaaagttt ccttttatgg 4440
cgaggcggcg gcggcggcgg ccctataaaa agcgaagcgc gcggcgggcg ggagtcgctg 4500
cgttgccttc gccccgtgcc ccgctccgcg ccgcctcgcg ccgcccgccc cggctctgac 4560
tgaccgcgtt actcccacag gtgagcgggc gggacggccc ttctcctccg ggctgtaatt 4620
agcgcttggt ttaatgacgg ctcgtttctt ttctgtggct gcgtgaaagc cttaaagggc 4680
tccgggaggg ccctttgtgc gggggggagc ggctcggggg gtgcgtgcgt gtgtgtgtgc 4740
gtggggagcg ccgcgtgcgg cccgcgctgc ccggcggctg tgagcgctgc gggcgcggcg 4800
cggggctttg tgcgctccgc gtgtgcgcga ggggagcgcg gccgggggcg gtgccccgcg 4860
gtgcgggggg gctgcgaggg gaacaaaggc tgcgtgcggg gtgtgtgcgt gggggggtga 4920
gcagggggtg tgggcgcggc ggtcgggctg taaccccccc ctgcaccccc ctccccgagt 4980
tgctgagcac ggcccggctt cgggtgcggg gctccgtgcg gggcgtggcg cggggctcgc 5040
cgtgccgggc ggggggtggc ggcaggtggg ggtgccgggc ggggcggggc cgcctcgggc 5100
cggggagggc tcgggggagg ggcgcggcgg ccccggagcg ccggcggctg tcgaggcgcg 5160
gcgagccgca gccattgcct tttatggtaa tcgtgcgaga gggcgcaggg acttcctttg 5220
tcccaaatct ggcggagccg aaatctggga ggcgccgccg caccccctct agcgggcgcg 5280
ggcgaagcgg tgcggcgccg gcaggaagga aatgggcggg gagggccttc gtgcgtcgcc 5340
gcgccgccgt ccccttctcc atctccagcc tcggggctgc cgcaggggga cggctgcctt 5400
cgggggggac ggggcagggc ggggttcggc ttctggcgtg tgaccggcgg ctctagagcc 5460
tctgctaacc atgttcatgc cttcttcttt ttcctacagc tcctgggcaa cgtgctggtt 5520
gttgtgctgt ctcatcattt tggcaaagaa ttgatttatc gataagcttg atatcgaatt 5580

c 5581

<210> 2
<211> 27
<212> DNA
<213> Artificial Sequence
<220>
<223> Description of Artificial Sequence: Synthetic DNA
<400> 2
cttgtcgaca tgcaggtctc tgtcacg 27

<210> 3
<211> 28
<212> DNA
<213> Artificial Sequence
<220>
<223> Description of Artificial Sequence: Synthetic DNA
<400> 3
cttgtcgaca ctagttctct gtcatact 28

<210> 4
<211> 44
<212> DNA
<213> Artificial Sequence
<220>
<223> Description of Artificial Sequence: Synthetic DNA
<400> 4

cgcggccgca taacttcgta taatgtatgc tatacgaagt tatc 44

<210> 5
<211> 52
<212> DNA
<213> Artificial Sequence
<220>
<223> Description of Artificial Sequence: Synthetic DNA
<400> 5
tcgagataac ttcgtatagc atacattata cgaagttatg cggccgcggt ca 52

<210> 6
<211> 47
<212> DNA
<213> Artificial Sequence
<220>
<223> Description of Artificial Sequence: Synthetic DNA
<400> 6
gatctataac ttcgtataat gtatgctata cgaagttatg gatccat 47

<210> 7
<211> 45
<212> DNA
<213> Artificial Sequence
<220>
<223> Description of Artificial Sequence: Synthetic DNA
<400> 7

cgatggatcc ataacttcgt atagcataca ttatacgaag ttata 45

## Description

### TECHNICAL FIELD

The present invention relates to a transgenic non-human mammal. More particularly, the present invention relates to a transgenic non-human mammal which is useful as an animal for pharmacological evaluation and expresses, in one or more tissues or organs, DNA encoding a foreign reporter protein for monitoring changes in intracellular calcium ion concentration. Further, the present invention relates to a method for producing the above transgenic non-human mammal, a method for using animals produced by the above method, DNA having promoter activity to be used in the above method, and a method of use thereof.

### BACKGROUND ART

Many cells compose a living body, and these cells lives while closely communicating with each other. Physiologically active substances, such as hormones, neurotransmitters and cell growth factors are involved in such close intercellular communication. Many of the physiologically active substances transduce signal by changing the intracellular calcium ion concentration.

The role of calcium ion as an intracellular signal transducing substance was observed in the skeletal muscle by Ebashi et al. in the 1960s (Ann. Rev. Physiol., 38, 293, 1976). Thereafter, Ozawa et al. showed that calcium ion is involved not only in muscle contraction, but also in other enzyme reactions (J. Biochem., 61, 531, 1967). Today, calcium ion has been shown to be involved in various physiological phenomena including excitement of nerve (Ann. Rev. Physiol., 60, 327, 1998), secretion of physiologically active substances from the secretory gland (Cell Calcium., 24, 367, 1998), and cell growth or differentiation (Cellular Signalling, 9, 483, 1997). Regarding the mechanism of incorporation of calcium ion into the cytoplasm, Michell has suggested that the metabolic turnover of phosphatidylinositol plays an important role in changing cytoplasmic calcium ion concentration (Biochim. Biophys. Acta, 415, 81, 1975). Subsequently, the presence of inositol 1, 4, 5-triphosphate (IP3) receptors in the endoplasmic reticulum that is an intracellular pool of calcium ion and the mechanism of releasing calcium ion via the receptor were revealed, so that calcium ion has been shown to be an intracellular signal transducing substance, called a "second messenger" (Nature, 342, 32, 1989).

Accordingly, since dynamic changes in intracellular calcium ion are involved in many physiological phenomena, precise measuring and monitoring of in vivo changes in calcium ion concentration are very important in the fields of medicine and the pharmaceutical development.

Examples of methods for measuring intracellular calcium ion concentration include 1) a method using a calcium indicator, such as fura-2 or indo-1 (Nature, 290, 527, 1981; J. Cell. Biol., 94, 325, 1982), 2) a method using Ca²⁺-sensitive microelectrode [Detection and Measurement of Free Ca²⁺ in cells (Elsevier, North-Holland, Amsterdam, 1979)], 3) a method using fluorescence resonance energy transfer (FRET), and 4) a method using aequorin, a calcium ion-sensitive photoprotein (Biochem. Biophys. Res. Commun., 29, 229, 1967).

A measurement method using a calcium indicator is the most widely used method. However, because these indicators are fluorescent substances, a problem exists due to the necessity of irradiating excitation light for measurement. That is, biological samples contain self-fluorescent substances, such as a serum component, so that the effect of the fluorescence of these substances must be considered (J. Biol. Chem., 260, 3440, 1985). Further, intracellular NADH and NADPH are also known to emit self fluorescence (Saibo Kogaku, Cell Technology, 17, 584, 1998). Furthermore, it is difficult to measure intracellular calcium ion for biological samples (for example, organs or tissues being cultured intact) which have a structure too complex to be irradiated with excitation light.

The method using Ca²⁺-sensitive microelectrode also has a constitutional constraint, that is, the necessity of insertion within a cell for measurement.

As a method using fluorescence energy transfer, a method using Cameleon, a chimera protein in which two types of fluorescent proteins are bound via calmodulin and M13 peptide, has been developed (Nature, 388, 882, 1997). However, irradiation with excitation light is necessary in the measurement, and a problem similar to that arising in measurement with calcium indicators has been pointed out (Saibo Kogaku, Cell Technology, 17, 584, 1998).

Next, the method using aequorin, a calcium ion-sensitive photoprotein, will be described. Aequorin is a bioluminescent protein complex which exists around the umbrella of luminous Aequorea (*Aequorea Victoria*), and was purified by Shimomura et al. in 1962 (J. Cell Comp. Physiol., 59, 223, 1962; Methods in Enzymol., 57, 271, 1978). Aequorin is a complex consisting of apoaequorin which is a protein portion, coelenterazine which is a luminous substrate, and a molecular oxygen. Aequorin is a protein complex which emits light upon binding with calcium ion (Pharmacol. Rev., 28, 1, 1976; Biochem. Biophys. Res. Comm., 211, 359, 1995). In 1985, Charbonneau et al. determined the amino acid sequence of apoaequorin (Biochemistry, 24, 6762, 1985). Cormier et al. (Biochem. Biophys. Res. Comm., 126, 1259, 1985; Japanese Published Unexamined Patent Application No. 86/224989) and Inouye et al. (Proc. Natl. Acad. Sci. USA, 82, 3154, 1985; Japanese Published Unexamined Patent Application No. 86/135586) have cloned an apoaequorin gene and revealed the nucleotide sequence thereof. Subsequently, a mutant which emits stronger light (Proc. Natl. Acad. Sci. USA, 86, 80, 1989; United States Patent No. 5,541,309), a mutant with enhanced sensitivity to calcium ion (Biochemical Journal, 228, 745, 1985), a mutant with improved stability against heat and freeze-drying (Japanese Published Unexamined Patent Application No. 97/278796) and a mutant modified to localize in the endoplasmic reticulum (EMBO J., 14, 5467, 1995) have been produced.

Aequorin is used for measuring calcium ion concentration because it emits light upon binding with calcium ion. For example, a method involving direct injection of apoaequorin protein within a cell (Biochem. J., 248, 313, 1987), and a method involving expression of apoaequorin gene in a culture cell (J. Biol. Chem., 270, 9896, 1995) have been used. However, in these methods, samples which is subjected to measurement of calcium ion concentration are limited to cells which can be cultured in vitro for a certain period of time. Moreover, due to the limited efficiency of introduction of a protein or a gene, it is difficult to inject or express apoaequorin in all the cells of biological samples under culturing. Particularly, it is difficult to introduce apoaequorin into a biological sample consisting of organs or tissues under culturing.

It is also known that cells in the state composing a part of the living body in vivo differ from cells under culturing in vitro after isolation from the living body in their physiological functions and responses to stimulations from outside (Biochem. Biophys. Res. Commun., 161, 385, 1989). Thus, the original function of cells is preferably evaluated using cells in the state composing the living body, and development of a method that allows examination of the function of a cell in an environment which reflects conditions closer to the in vivo state is sought.

As a means to solve the above problems, production of a transgenic individual has been attempted. For example, the mechanism of a neuropeptide function has been analyzed using a fly to which apoaequorin was introduced (J. Cell Sci., 110, 1683, 1997). Regarding plants, a transgenic plant containing a gene introduced therein which comprises an apoaequorin gene ligated downstream of 35S promoter of cauliflower mosaic virus has been produced, and the changes in intracellular calcium ion concentration resulting from contact or low temperature shock have been observed (Nature, 352, 524, 1991). Regarding mammals, a transgenic mouse containing a gene introduced therein which comprises an apoaequorin gene ligated downstream of a neuron-specific promoter, that is, enolase promoter, has been produced, and it has been showed that measurement of changes in intracellular calcium ion concentration in nerve cells by drug response is possible (United States Patent No. 5,714,666).

When a transgenic individual is produced, a transgene is inserted at random on the chromosome of the individual, so that an individual difference occurs in the efficiency and distribution of expression of the transgene. For example, it has been reported that cells which do not express transgenes are observed even when gene introduction is performed using the promoter of β actin gene, which is thought to be expressed constitutively as a house-keeping gene in each cell (Development, 106, 37, 1989). In addition, expression in the organs is not always the same among individuals, even when gene introduction is performed using a promoter (hereinafter referred to as CAG promoter) obtained by fusing an enhancer of IE (immediate early) gene of human cytomegalovirus with a chicken β actin promoter which is known to exhibit enhanced promoter activity (Develop. Growth Differ., 37, 455, 1995; FEBS Letters, 407, 313, 1997). Moreover, with respect to toxicity and physiological dysfunction caused when apoaequorin is expressed over a long period of time in the living tissue other than the nerve in mammals, no findings have been reported. In producing a transgenic animal, it is also necessary to consider the influence caused by disruption of an existing gene residing at a position to which a transgene is inserted. If individuals differ from each other in terms of the insertion position of a transgene on the chromosome, such individuals are treated as different lines having different genetic backgrounds. When a candidate medicament is pharmacologically evaluated, inbred strain animals having a uniform genetic background are often used as models. Therefore, if a transgenic animal which stably expresses a transgene in increased numbers of tissues or organs can be produced, a variety of tissues, organs or cells which share the same genetic background and are useful for measuring changes in intracellular calcium ion concentration can be supplied.

There have been various reports on a method for producing transgenic animals.

In 1980, Gordon et al. produced a transgenic mouse by directly injecting DNA into a fertilized egg (Proc. Natl. Acad. Sci. USA, 77, 7380, 1980). Since then, using a similar method, production of a transgenic zebra fish (Development, 109, 577, 1990), a transgenic chicken (Bio/Technology, 12, 60, 1994), a transgenic rat (Nature, 344, 541, 1990), a transgenic rabbit (Nature, 315, 680, 1985), and a transgenic pig (Immunol. Immunopathol., 17, 303, 1987) have been reported. That is, the method for producing a transgenic animal by directly injecting DNA into a fertilized egg has been shown to be a highly generalized method.

Further, since the establishment of mouse embryonic stem cells (Nature, 292, 154, 1981; Proc. Natl. Acad. Sci. USA, 78, 7634, 1981), effective methods for establishing embryonic stem cells, for example, a method for establishing non-mouse embryonic stem cells (United States Patent No. 5,453,357;United States Patent No. 5,670,372), have been studied. Thus, embryonic stem cells of a rat (Dev. Biol., 163, 288, 1994), chicken (United States Patent No. 5,340,740; United States Patent No. 5,656,479), pig (Reprod. Fertil. Dev., 6, 563, 1994), monkey (Proc. Natl. Acad. Sci. USA, 92, 7844, 1996), and human (Science, 282, 1145, 1998; Proc. Natl. Acad. Sci. USA, 95, 13726, 1998) have been established. Gene introduction into these embryonic stem cells can be performed in a manner similar to standard techniques for gene introduction into culture cells. After introduction of a target gene, a transgenic animal can be produced by techniques such as an aggregation chimera method, and an injection chimera method [Manipulating the Mouse Embryo, A Laboratory Manual, Second Edition, Cold Spring Harbor Laboratory Press (1994) (hereinafter abbreviated as Manipulating Mouse Embryo, 2^{nd} ed.); Gene Targeting, A Practical Approach, IRL Press at Oxford University Press (1993); Bio Manual Series 8, Gene Targeting, Production of Mutant Mouse Using ES Cells, YODOSHA (1995); Developmental Engineering Experimental Manual, How to Produce Transgenic Mouse, KODANSHA Ltd. (1987)].

Moreover, since Wilmut et al. produced a cloned mammal for the first time, that is, a cloned sheep named Dolly derived from the nucleus of a somatic cell (Nature, 385, 810, 1997) in 1997, the production of a cloned cow using the nucleus of a fetal cell (Science, 280, 1256, 1998), a cloned cow using the nuclei of skin, muscle, auricle, oviduct and cumulus cells (Protein, Nucleic Acid, Enzyme, 44, 892, 1999), a cloned goat (Nature Biotechnology, 17, 456, 1999), a cloned mouse using the nucleus of a cumulus cell (Nature, 394, 369, 1998), and a cloned mouse using a cell derived from the tail of a male mouse (Nature Genetics, 22, 127, 1999) have been reported. Gene introduction into somatic cells or embryo-derived cells can be performed in a manner similar to standard techniques for gene introduction into culture cells. A transgenic animal has been produced by transplanting a nucleus having a target gene introduced therein into an enucleated egg (Science, 280, 1256, 1998; Science, 278, 2130, 1997).

Further, advances in the microfertilization technique have revealed that a spermatid originally lacking fertility can have fertility, so that individuals have been obtained using a mouse spermatocyte (Biol. Reprod., 53, 855, 1995), mouse spermatid (Proc. Natl. Acad. Sci. USA, 91, 7460, 1994), rabbit spermatid (J. Assist. Reprod. Genet., 11, 335, 1994), bull spermatid (Hum. Reprod., 11, 824, 1996), and human spermatid (New Eng. J. Med., 333, 525, 1995) as male gametes. Furthermore, a transgenic mouse having a target foreign gene introduced into the chromosome has been produced by mixing a freeze-dried, or frozen and thawed, sperm with the foreign gene, followed by microfertilization (Science, 284, 1180, 1999).

When an egg cell is used, a general method for producing a transgenic animal (Manipulating Mouse Embryo, 2^{nd} ed.) involves allowing eight-cell-stage embryos to be infected with retrovirus. However, it has been reported that performing retrovirus-mediated introduction of a gene into an unfertilized egg at the metaphase of the second meiotic division enables production of a transgenic cow more efficiently compared to conventional techniques (Proc. Natl. Acad. Sci. USA, 95, 14028, 1998).

There have been various reports on differentiation from embryonic stem cells to various cells. When an embryonic stem cell is subcutaneously transplanted to an individual isogenic with the embryonic stem cell, teratoma composed of multiple tissues is induced (Manipulating Mouse Embryo, 2^{nd} ed). In in vitro culturing of embryonic stem cells, it has been reported that the embryonic stem cells are induced to differenciate to endodermal cells, ectodermal cells, mesodermal cells, blood cells, endothelial cells, cartilage cells, skeletal muscle cells, smooth muscle cells, cardiac muscle cells, neuronal cells, glial cells, epidermal cells, melanocytes, and keratinocytes (Reprod. Fertil. Dev., 10, 31, 1998).

As described above, some methods for producing a transgenic animal are known, however, nothing has been reported thus far about production of a transgenic animal which has DNA encoding a foreign reporter protein for monitoring changes in intracellular calcium ion concentration, and expresses the reporter protein in tissues other than the nerve.

### DISCLOSURE OF THE INVENTION

A problem to be solved by the present invention is to provide a transgenic non-human mammal which has DNA encoding a foreign reporter protein for monitoring changes in intracellular calcium ion concentration, and which expresses the reporter protein in one or more tissues or organs. Another problem to be solved by the present invention is to provide a method for producing the above transgenic non-human mammal, a method for detecting changes in intracellular calcium ion concentration by using the above transgenic non-human mammal, DNA having promoter activity to be used for the above production method, and a method of use of the DNA.

The transgenic non-human mammal of the present invention is considered to be useful for pharmacological evaluation of various physiologically active substances. In particular, it is considered to be useful for examining drug responses and the like in cells which are difficult to culture in vitro while keeping their physiological functions, and in tissues or organs having a complex intercellular network.

The present inventors have produced a transgenic non-human mammal by searching for a DNA sequence having promoter activity which promotes high expression of a transgene in various tissues and organs, and introducing DNA, which is ligated to said DNA sequence and encodes a reporter protein for monitoring changes in intracellular calcium ion concentration, into a mammalian egg or embryonic stem cell. As a result of having thoroughly studied expression of the product of a transgene in various tissues and organs of the produced transgenic non-human mammal, the present inventors have succeeded in producing a transgenic non-human mammal which expresses a target transgene in at least one or more non-neuronal cells (for example, cells of the tissues and organs, including epithelial tissue, connective tissue, adipose tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail), thereby completing the present invention.

Thus, the present invention relates to the following (1) to (61).
(1) A transgenic non-human mammal which has DNA encoding a foreign reporter protein for monitoring changes in intracellular calcium ion concentration, and expresses the reporter protein in one or more types of non-neuronal cells.
(2) The transgenic non-human mammal according to (1) wherein the non-neuronal cells are cells which compose a site selected from the group consisting of epithelial tissue, connective tissue, adipose tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail.
(3) The transgenic non-human mammal according to (1) or (2) wherein the DNA encoding a foreign reporter protein is DNA selected from the group consisting of the following (a), (b), (c) and (d):
   (a) DNA encoding apoaequorin;
   (b) DNA encoding a protein which comprises an amino acid sequence derived from the amino acid sequence of apoaequorin by deletion, substitution, or addition of one or more amino acids, and which is capable of emitting light upon binding with calcium ion;
   (c) DNA encoding a protein which comprises an amino acid sequence having 50% or more homology with the amino acid sequence of apoaequorin, and which is capable of emitting light upon binding with calcium ion;
   (d) DNA capable of hybridizing to any one of the DNAs of (a) to (c) under stringent conditions, and encoding a protein which is capable of emitting light upon binding with calcium ion;
   (e) DNA encoding cameleon;
   (f) DNA encoding a protein which comprises an amino acid sequence derived from the amino acid sequence of cameleon by deletion, substitution, or addition of one or more amino acids, and which is capable of causing fluorescence resonance energy transfer (FRET) upon binding with calcium ion;
   (g) DNA encoding a protein which comprises an amino acid sequence having 50% or more homology with the amino acid sequence of cameleon, and which is capable of causing fluorescence resonance energy transfer (FRET) upon binding with calcium ion;
   (h) DNA capable of hybridizing to any one of the DNAs of (e), (f) and (g) under stringent conditions, and encoding a protein which is capable of causing fluorescence resonance energy transfer (FRET) upon binding with calcium ion.
(4) The transgenic non-human mammal according to any one of (1) to (3) wherein the DNA encoding a foreign reporter protein is ligated to DNA having promoter activity that exists upstream of the DNA.
(5) The transgenic non-human mammal according to (4) wherein the DNA having promoter activity is any one of the following DNAs of (a) to (i):
   (a) DNA having SE promoter sequence;
   (b) DNA having CAG promoter sequence;
   (c) DNA having PGK promoter sequence;
   (d) DNA having a sequence in which hprt gene is inserted between two types of promoters;
   (e) DNA in which loxP, promoter, hprt gene, loxP and promoter are linked in this order;
   (f) DNA having a nucleotide sequence represented by SEQ ID NO: 1;
   (g) DNA comprising a nucleotide sequence derived from the nucleotide sequence of any one of the DNAs of (a) to (f) by deletion, substitution, or addition of one or more nucleotides and having promoter activity;
   (h) DNA comprising a nucleotide sequence which has 50% or more homology with the nucleotide sequence of any one of the DNAs of (a) to (f) and having promoter activity;
   (i) DNA capable of hybridizing to any one of the DNAs of (a) to (f) under stringent conditions and having promoter activity;
(6) The transgenic non-human mammal according to any one of (1) to (5), which is a non-human mammal selected from the group consisting of a mouse, rat, guinea pig, hamster, rabbit, dog, cat, sheep, pig, goat, cow and monkey.
(7) The transgenic non-human mammal according to any one of (1) to (6), which is a non-human mammal having a mutated gene on the chromosome.
(8) A method for producing the transgenic non-human mammal according to any one of (1) to (7), which comprises the steps of constructing a transgene which comprises DNA encoding a foreign reporter protein and DNA having promoter activity located upstream of the DNA; microinjecting the transgene into a fertilized egg of a non-human mammal; transplanting the obtained egg to the oviduct or uterus of a pseudopregnant female non-human mammal; breeding the transplanted animal; and from the offsprings, selecting individuals having the transgene.
(9) A method for producing the transgenic non-human mammal according to any one of (1) to (7), which comprises the steps of constructing a transgene which comprises DNA encoding a foreign reporter protein and DNA having promoter activity located upstream of the DNA; introducing the transgene into an embryonic stem cell of a non-human mammal; introducing the embryonic stem cell into a fertilized egg of a non-human mammal; transplanting the obtained egg to the oviduct or uterus of a pseudopregnant female non-human mammal; breeding the transplanted animal; and from the offsprings, selecting individuals having the transgene.
(10) A method for producing the transgenic non-human mammal according to any one of (1) to (7), which comprises the steps of constructing a transgene which comprises DNA encoding a foreign reporter protein and DNA having promoter activity located upstream of the DNA; introducing the transgene into a cell of a non-human mammal; introducing the nucleus of the cell into an unfertilized and enucleated egg of a mammal; transplanting the obtained egg to the oviduct or uterus of a pseudopregnant female non-human mammal; breeding the transplanted animal; and from the offsprings, selecting individuals having the transgene.
(11) A method for producing the transgenic non-human mammal according to any one of (1) to (7), which comprises the steps of constructing a transgene which comprises DNA encoding a foreign reporter protein and DNA having promoter activity located upstream of the DNA; allowing a sperm of a non-human mammal to incorporate the transgene; allowing the sperm to fertilize an unfertilized egg; transplanting the obtained egg to the oviduct or uterus of a pseudopregnant female non-human mammal; breeding the transplanted animal; and from the offsprings, selecting individuals having the transgene.
(12) A method for producing the transgenic non-human mammal according to any one of (1) to (7), which comprises the steps of constructing a transgene which comprises DNA encoding a foreign reporter protein and DNA having promoter activity located upstream of the DNA; inregrating the transgene into a retrovirus vector; infecting an egg cell with virus containing the retrovirus vector; transplanting the obtained egg to the oviduct or uterus of a pseudopregnant female non-human mammal; breeding the transplanted animal; and from the offsprings, selecting individuals having the transgene.
(13) An embryonic stem cell of a non-human mammal in which DNA encoding a foreign reporter protein is introduced, which is used for producing the transgenic non-human mammal according to any one of (1) to (7).
(14) A cell of the transgenic non-human mammal according to any one of (1) to (7).
(15) The cell according to (14), which is a cell selected from the group consisting of an embryonic stem cell, an egg and a sperm.
(16) A nucleus of the cell according to any one of (13) to (15).
(17) A method for detecting changes in intracellular calcium ion concentration by using a foreign reporter protein, wherein the transgenic non-human mammal according to any one of (1) to (7) expressing the foreign reporter protein, or the tissue, organ or cell of the animal, is used.
(18) The method according to (17), wherein the transgenic non-human mammal is a living transgenic non-human mammal.
(19) A method for detecting changes in intracellular calcium ion concentration by using a foreign reporter protein introduced into an embryonic stem cell, which comprises inducing differentiation of the embryonic stem cell according to (13) or (15), and detecting changes in intracellular calcium ion concentration of the cell obtained by the induction of differentiation.
(20) A method for screening a substance involved in the regulation of intracellular calcium ion concentration, which comprises contacting a test substance with the transgenic non-human mammal according to any one of (1) to (7) expressing a foreign reporter protein, or the tissue, organ or cell of the animal; and detecting changes in intracellular calcium ion concentration using the foreign reporter protein.
(21) A method for producing a transgenic non-human mammal wherein the cell according to (14) or (15) is used.
(22) A method for producing a cloned non-human mammal wherein the nucleus of the cell of (16) is used.
(23) A transgenic or cloned non-human mammal which is produced by the method according to (21) or (22).
(24) A method for producing a gene-modified mammal, which comprises mating the transgenic non-human mammal according to any one of (1) to (7) with an animal belonging to the same species but to a different line from the transgenic non-human mammal.
(25) A gene-modified mammal which is obtained by the method of (24).
(26) A method for detecting changes in intracellular calcium ion concentration using a foreign reporter protein, wherein the mammal according to (23) or (25) expressing the foreign reporter protein, or the tissue, organ or cell of the animal is used.
(27) The method according to (26), wherein the mammal is a living mammal.
(28) A method for screening a substance involved in the regulation of intracellular calcium ion concentration, which comprises contacting a test substance with the mammal according to (23) or (25) expressing a foreign reporter protein, or the tissue, organ or cell of the animal; and detecting changes in intracellular calcium ion concentration using the foreign reporter protein.
(29) DNA selected from the following (a) to (f):
   (a) DNA having a sequence in which hprt gene is inserted between two types of promoters;
   (b) DNA in which loxP, promoter, hprt gene, loxP and promoter are ligated in this order;
   (c) DNA having a nucleotide sequence represented by SEQ ID NO: 1;
   (d) DNA comprising a nucleotide sequence derived from the nucleotide sequence of any one of the DNAs of (a) to (c) by deletion, substitution, or addition of one or more nucleotides and having promoter activity;
   (e) DNA comprising a nucleotide sequence which has 50% or more homology with the nucleotide sequence of any one of the DNAs of (a) to (c) and having promoter activity;
   (f) DNA capable of hybridizing to any one of the DNAs of (a) to (c) under stringent conditions and having promoter activity.
(30) The DNA according to (29), wherein the DNA having a sequence in which hprt gene is inserted between two types of promoters is DNA having a sequence in which hprt gene is inserted between PGK promoter and CAG promoter.
(31) The DNA according to (29), wherein the DNA in which loxP, promoter, hprt gene, loxP and promoter are ligated in this order is DNA in which loxP, PGK promoter, hprt gene, loxP and CAG promoter are ligated in this order.
(32) The DNA according to any one of (29) to (31), which has, when integrated into a chromosome of a mammal, promoter activity higher than the respective promoter activity of loxP, promoter, and hprt gene that compose the DNA.
(33) The DNA according to (32) which has, when integrated into a chromosome of a mammal, promoter activity higher than the respective promoter activity of loxP, promoter, and hprt gene that compose the DNA, in a cell composing a site selected from the group consisting of epithelial tissue, connective tissue, adipose tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail.
(34) A transgenic non-human mammal, which has DNA wherein DNA encoding a foreign protein is ligated downstream of the DNA according to any one of (29) to (33).
(35) The transgenic non-human mammal according to (34), wherein the DNA encoding a foreign protein is DNA selected from the following (a) or (b):
   (a) DNA encoding a foreign reporter protein for monitoring changes in intracellular calcium ion concentration;
   (b) DNA encoding a foreign reporter protein for labeling a cell.
(36) The transgenic non-human mammal according to (35) wherein the DNA encoding a foreign reporter protein for monitoring changes in intracellular calcium ion concentration is DNA selected from the group consisting of the following (a), (b), (c), (d), (e), (f), (g) and (h):
   (a) DNA encoding apoaequorin;
   (b) DNA encoding a protein which comprises an amino acid sequence derived from the amino acid sequence of apoaequorin by deletion, substitution, or addition of one or more amino acids, and which is capable of emitting light upon binding with calcium ion;
   (c) DNA encoding a protein which comprises an amino acid sequence having 50% or more homology with the amino acid sequence of apoaequorin, and which is capable of emitting light upon binding with calcium ion;
   (d) DNA capable of hybridizing to any one of the DNAs of (a) to (c) under stringent conditions and encoding a protein which is capable of emitting light upon binding with calcium ion;
   (e) DNA encoding cameleon;
   (f) DNA encoding a protein which comprises an amino acid sequence derived from the amino acid sequence of cameleon by deletion, substitution, or addition of one or more amino acids, and which is capable of causing fluorescence resonance energy transfer (FRET) upon binding with calcium ion;
   (g) DNA encoding a protein which comprises an amino acid sequence having 50% or more homology with the amino acid sequence of cameleon, and which is capable of causing fluorescence resonance energy transfer (FRET) upon binding with calcium ion;
   (h) DNA capable of hybridizing to any one of the DNAs of (e) to (g) under stringent conditions, and encoding a protein which is capable of causing fluorescence resonance energy transfer (FRET) upon binding with calcium ion.
(37) The transgenic non-human mammal according to (35), wherein the DNA encoding a foreign reporter protein for labeling a cell is DNA selected from the following (a) or (b):
   (a) DNA encoding a fluorescent protein;
   (b) DNA encoding a photo- or chromogenic protein.
(38) The transgenic non-human mammal according, to (37), wherein the DNA encoding a fluorescent protein is DNA selected from the group consisting of the following (a), (b), (c), (d) and (e):
   (a) DNA encoding the green fluorescent protein (GFP) of jelly fish;
   (b) DNA encoding the red fluorescent protein (DsRed) of sea anemone;
   (c) DNA encoding a protein which comprises an amino acid sequence derived from the amino acid sequence of the fluorescent protein of (a) or (b) by deletion, substitution, or addition of one or more amino acids, and which is capable of emitting fluorescence;
   (d) DNA encoding a protein which comprises an amino acid sequence having 50% or more homology with the amino acid sequence of the fluorescent protein of (a) or (b), and which is capable of emitting fluorescence;
   (e) DNA capable of hybridizing to any one of the DNAs of (a) to (d) under stringent conditions and encoding a protein which is capable of emitting fluorescence.
(39) The transgenic non-human mammal according to (37), wherein the DNA encoding a photo- or chromogenic protein is DNA selected from the group consisting of the following (a), (b), (c), (d), (e), (f), (g) and (h):
   (a) DNA encoding luciferase of firefly;
   (b) DNA encoding luciferase of *Renilla*;
   (c) DNA encoding alkaline phosphatase:
   (d) DNA encoding β-galactosidase;
   (e) DNA encoding chroramphenicol acetyltransferase;
   (f) DNA encoding a protein which comprises an amino acid sequence derived from the amino acid sequence of the photo- or chromogenic protein according to any one of (a) to (e) by deletion, substitution, or addition of one or more amino acids, and which is capable of catalyzing light emission or color development by acting on any substrate;
   (g) DNA encoding a protein which comprises an amino acid sequence having 50% or more homology with the amino acid sequence of the photo- or chromogenic protein according to any one of (a) to (e), and which is capable of catalyzing light emission or color development by acting on any substrate;
   (h) DNA capable of hybridizing to any one of the DNAs of (a) to (g) under stringent conditions and encoding a protein which is capable of catalyzing light emission or color development by acting on any substrate.
(40) The transgenic non-human mammal according to any one of (34) to (39), which is selected from the group consisting of a mouse, rat, guinea pig, hamster, rabbit, dog, cat, sheep, pig, goat, cow and monkey.
(41) The transgenic non-human mammal according to any one of (34) to (40), which is a non-human mammal having a mutated gene on the chromosome.
(42) A method for producing the transgenic non-human mammal according to any one of (34) to (41), which comprises the steps of constructing a transgene which comprises DNA having DNA encoding a foreign protein ligated downstream of the DNA according to any one of (29) to (33); microinjecting the transgene into a fertilized egg of a non-human mammal; transplanting the obtained egg to the oviduct or uterus of a pseudopregnant female non-human mammal; breeding the transplanted animal; and from the offsprings, selecting individuals having the transgene.
(43) A method for producing the transgenic non-human mammal according to any one of (34) to (41), which comprises the steps of constructing a transgene which comprises DNA having DNA encoding a foreign protein ligated downstream of the DNA according to any one of (29) to (33); introducing the transgene into an embryonic stem cell of a non-human mammal; introducing the embryonic stem cell into a fertilized egg of a non-human mammal; transplanting the obtained egg to the oviduct or uterus of a pseudopregnant female non-human mammal; breeding the transplanted animal; and from the offsprings, selecting individuals having the transgene.
(44) A method for producing the transgenic non-human mammal according to any one of (34) to (41), which comprises the steps of constructing a transgene which comprises DNA having DNA encoding a foreign protein ligated downstream of the DNA according to any one of (29) to (33); introducing the transgene into a cell of a non-human mammal; introducing the nucleus of the cell into an unfertilized and enucleated egg of a mammal; transplanting the obtained egg to the oviduct or uterus of a pseudopregnant female non-human mammal; breeding the transplanted animal; and from the offsprings, selecting individuals having the transgene.
(45) A method for producing the transgenic non-human mammal according to any one of (34) to (41), which comprises the steps of constructing a transgene which comprises DNA having DNA encoding a foreign protein ligated downstream of the DNA according to any one of (29) to (33); allowing a sperm of a non-human mammal to incorporate the transgene; allowing the sperm to fertilize an unfertilized egg; transplanting the obtained egg to the oviduct or uterus of a pseudopregnant female non-human mammal; breeding the transplanted animal; and from the offsprings, selecting individuals having the transgene.
(46) A method for producing the transgenic non-human mammal according to any one of (34) to (41), which comprises the steps of constructing a transgene which comprises DNA having DNA encoding a foreign protein ligated downstream of the DNA according to any one of (29) to (33); intregrating the transgene into a retrovirus vector; infecting an egg cell with virus containing the retrovirus vector; transplanting the obtained egg to the oviduct or uterus of a pseudopregnant female non-human mammal; breeding the transplanted animal; and from the offsprings, selecting individuals having the transgene.
(47) An embryonic stem cell of a non-human mammal in which DNA encoding a foreign protein is introduced, which is used for producing the transgenic non-human mammal according to any one of (34) to (41).
(48) A cell of the transgenic non-human mammal according to any one of (34) to (41).
(49) The cell according to (48) which is a cell selected from the group consisting of an embryonic stem cell, an egg and a sperm.
(50) A nucleus of the cell according to any one of (47) to (49).
(51) A method for detecting changes in intracellular calcium ion concentration using a foreign reporter protein, wherein the transgenic non-human mammal according to any one of (35), (36), (40) and (41) expressing the foreign reporter gene, or the tissue, organ or cell of the animal is used.
(52) The method according to (51), wherein the transgenic non-human mammal is a living transgenic non-human mammal.
(53) A method for detecting changes in intracellular calcium ion concentration using a foreign reporter protein introduced in an embryonic stem cell, which comprises inducing differentiation of the embryonic stem cell according to (47) or (49), and detecting changes in calcium ion concentration in the cell obtained by the induction of differentiation.
(54) A method for screening a substance involved in the regulation of intracellular calcium ion concentration, which comprises contacting a test substance with the transgenic non-human mammal according to (35), (36), (40) or (41) expressing a foreign reporter protein, or the tissue, organ or cell of the animal; and detecting changes in intracellular calcium ion concentration using the foreign reporter protein.
(55) A method for detecting physiological changes, which comprises transplanting the tissue, organ or cell of the transgenic non-human mammal according to any one of (34) to (41) expressing the foreign reporter protein into another individual which does not immunologically reject the transplanted tissue, organ or cell; and detecting physiological changes in the transplanted tissue, organ or cell using the foreign reporter protein.
(56) A method for detecting physiological changes, which comprises transplanting an embryonic stem cell according to (47) or (49) or a cell induced to differentiate from the embryonic stem cell into another individual which does not immunologically reject the transplanted cell; and detecting physiological changes in the transplanted cell using the foreign reporter protein introduced in the embryonic stem cell.
(57) A method for producing a transgenic non-human mammal, which uses the cell according to (48) or (49).
(58) A method for producing a cloned non-human mammal, which uses the nucleus according to (50).
(59) A transgenic or a cloned non-human mammal which is produced by the method according to (57) or (58).
(60) A method for producing a gene-modified mammal, which comprises mating the transgenic non-human mammal according to any one of (34) to (41) with an animal belonging to the same species but to a different strain from the transgenic non-human mammal.
(61) A gene-modified mammal, which is obtained by the method according to (60).

### BRIEF DESCRIPTION OF DRAWINGS

Figure 1 shows the construction of a plasmid pAGE107AEQ.
Figure 2 shows the construction of a plasmid pBSAEQpA.
Figure 3 shows the construction of a plasmid pBSKS(+)CAG promoter.
Figure 4 shows the construction of a plasmid pCAG-AEQ.
Figure 5 shows the construction of a plasmid ploxp#1.
Figure 6 shows the construction of a plasmid ploxp.
Figure 7 shows the construction of a plasmid ploxpHPRT.
Figure 8 shows the construction of a plasmid ploxpHPRT2.
Figure 9 shows the construction of a plasmid pCAG-AEQ-pHPRTp.
Figure 10 shows the result of comparing promoter activity of each plasmid using as an index the amount of luminescence from aequorin by stimulation with A23187. Specifically, Fig. 10 shows the results when CHOdhfr (-) DG44 cells into which (1) pCAG-AEQ (2) pAGE107AEQ or (3) pBSAEQpA was respectively introduced were used. Further, RLU denotes the relative luminescence unit.
Figure 11 shows comparison of the expression level of apoaequorin of clone 21 transformed with plasmid pCAG-AEQ-pHPRTp and that of mutant clone 21 Δ hprt prepared by removing the expression unit of hprt gene from clone 21.
Figure 12 shows the result of analysis on expression of transgenes in the samples of the crystalline lens, cranial bone, nail and teeth prepared from the transgenic mouse of the present invention.
Figure 13 shows the result of measurement of physiologically active substances using the transgenic mouse of the present invention. Addition of (1) RPMI1640, (2) angiotensin II with a final concentration of 1 µmol/l, (3) bradykinin with a final concentration of 10 µmol/l, (4) A23187 with a final concentration of 1 µmol/l and (5) TritonX-100 with a final concentration of 2% were performed at each of the times indicated with an arrow.
Figure 14 shows the results of measurement of physiologically active substances using the transgenic mouse of the present invention. Addition of (1) RPMI1640, (2) carbachol with a final concentration of 10 µmol/l, (3) α methylserotonin with a final concentration of 10 µmol/l, (4) ATP with a final concentration of 100 µmol/l, (5) phenylephrine with a final concentration of 20 µmol/l, (6) A23187 with a final concentration of 1 µmol/l, and (7) TritonX-100 with a final concentration of 2% were performed at each of the times indicated with an arrow.
Figure 15 shows the result of measurement of physiologically active substances using the transgenic mouse of the present invention. Addition of (1) RPMI1640, (2) endothelin with a final concentration of 10 nmol/l, (3) calcitonin with a final concentration of 10 nmol/l, (4) A23187 with a final concentration of 1 µmol/l and (5) TritonX-100 with a final concentration of 2% were performed at each of the times indicated with an arrow.
Figure 16 shows the result of measurement of physiologically active substances using the transgenic mouse of the present invention. Addition of (1) RPMI1640, (2) phenylephrine with a final concentration of 20 µmol/l, (3) A23187 with a final concentration of 1 µmol/l and (4) TritonX-100 with a final concentration of 2% were performed at each of the times indicated with an arrow.
Figure 17 shows the results of measurement of physiologically active substances using the uterus prepared from the transgenic mouse of the present invention. Addition of RPMI1640 medium, carbachol (car) with a final concentration of 20 µmol/l, α methylserotonin (aMe-5HT) with a final concentration of 20 µmol/l, ATP with a final concentration of 100 µmol/l, phenylephrine (PE) with a final concentration of 20 µmol/l, A23187 with a final concentration of 1 µmol/l, and TritonX-100 with a final concentration of 2% were performed in order at each of the times indicated with an arrow.
Figure 18 shows the construction of plasmid pCAG-GFP-pHPRTp.

The embodiments and methods of the present invention will now be further described.

A foreign protein of the transgenic non-human mammal of the present invention and DNA encoding the protein are divided into the following two types:
(a) a foreign reporter protein for monitoring changes in intracellular calcium ion concentration, and DNA encoding the protein;
(b) a foreign reporter protein for labeling cells, and DNA encoding the protein.

In the present invention, the foreign reporter protein for monitoring changes in intracellular calcium ion concentration encompasses a reporter protein which is expressed within a cell, and can transduce signal about intracellular calcium ion concentration toward the outside of the cells by exhibiting any function such as luminescence, fluorescence or transcription in a manner depending on calcium ion. Examples of such a foreign reporter protein include apoaequorin and cameleon or variants thereof.

The DNA sequence of apoaequorin gene is known as has been reported by Cormier et al. (Biochem. Biophys. Res. Comm., 126, 1259, 1985; Japanese Published Unexamined Patent Application No. 86/224989) and mouye et al. (Proc. Natl. Acad. Sci. USA, 82, 3154, 1985; Japanese Published Unexamined Patent Application No. 86/135586). The DNA sequence of cameleon gene is known as has been reported by Miyawaki et al. (Nature, 388, 882, 1997).

Examples of DNA encoding a variant of apoaequorin protein include a DNA nucleotide sequence which is obtained by introducing variation (for example, mutation) into the original apoaequorin gene. Specific examples of DNA encoding apoaequorin protein or a variant thereof include the following DNA:
(a) DNA encoding apoaequorin;
(b) DNA encoding a protein which comprises an amino acid sequence derived from the amino acid sequence of apoaequorin by deletion, substitution or addition of one or more amino acids, and which is capable of emitting light upon binding with calcium ion;
(c) DNA encoding a protein which comprises an amino acid sequence which has 50% or more homology with the amino acid sequence of apoaequorin, and which is capable of emitting light upon binding with calcium ion;
(d) DNA capable of hybridizing to any one of the DNAs of (a) to (c) under stringent conditions, and encoding a protein capable of emitting light upon binding with calcium ion.

The above described "an amino acid sequence derived from the amino acid sequence of apoaequorin by deletion, substitution or addition of one or more amino acids" means an amino acid sequence which is derived from the amino acid sequence of wild type apoaequorin protein by deletion, substitution or addition ("addition" used in the present specification includes both insertion into the inside of a sequence, and addition to the outside of a sequence) of any number of amino acids, for example, 1 to 20, preferably 1 to 15, more preferably 1 to 10, further more preferably 1 to 5.

The above described "an amino acid sequence having 50% or more homology with the amino acid sequence of apoaequorin" means, when calculated with analysis software, such as BLAST (J.Mol.Biol., 215. 403, 1990) and FASTA (Methods in Enzymology, 183, 63-69, 1990), an amino acid sequence showing at least 50% or more, preferably 60% or more, more preferably 70% or more, further more preferably 80% or more, further more preferably 85% or more, further more preferably 90% or more, particularly preferably 95% or more, and most preferably 98% or more sequence identity at the amino acid level with wild type apoaequorin protein.

The above described "DNA capable of hybridizing under stringent conditions and encoding a protein capable of emitting light upon binding with calcium ion" means DNA which can be obtained by the colony hybridization method, plaque hybridization method, Southern blot hybridization method or the like using DNA encoding wild type apoaequorin as a probe. A specific example is DNA which can be identified by performing hybridization at 42°C to 68°C in the presence of 0.7 to 1.0 mol/l sodium chloride using a filter to which DNA derived from a colony or a plaque has been immobilized, and washing the filter at a temperature condition of 42°C to 68°C using a 0.1 to 2 x SSC solution (the composition of a 1 x SSC solution comprises 150 mmol/l sodium chloride and 15 mmol/l sodium citrate). Hybridization can be performed according to methods described in Molecular Cloning, A Laboratory Manual, Second Edition, Cold Spring Harbor Laboratory Press (1989) (hereinafter, abbreviated as Molecular Cloning, 2^{nd} ed.), Current Protocols in Molecular Biology, John Wiley & Sons (1987-1997) (hereinafter abbreviated as Current Protocols in Molecular Biology), DNA Cloning 1: Core Techniques, A Practical Approach, Second Edition, Oxford University (1995), or the like. Specific examples of DNAs capable of hybridizing as described above include, when calculated with BLAST, FAST or the like, those having at least 50% or more, preferably 60% or more, more preferably 70% or more, further more preferably 80% or more, further more preferably 85% or more, further more preferably 90% or more, particularly preferably 95% or more, and most preferably 98% or more homology with the nucleotide sequence of the DNA encoding wild type apoaequorin.

The above described "a protein capable of emitting light upon binding with calcium ion" means a protein which can produce light upon binding with calcium ion in the presence of coelenterazine, a luminogenic substrate, in a manner similar to wild type apoaequorin protein.

A method for obtaining a mutated apoaequorin gene may be any method known in the art, such as chemical synthesis, genetic engineering techniques, mutagenesis and the like. For example, the DNA of a mutated apoaequorin gene can be obtained based on the wild type apoaequorin gene.

Mutation can be introduced into a gene encoding wild type apoaequorin by various methods. Further, mutation may be introduced into a subcloned wild type apoaequorin gene fragment, or into the full-length wild type apoaequorin gene. For example, mutation can be introduced by a method which involves allowing a drug as a mutagen to contact with and to act on a wild type apoaequorin gene or on a recombinant DNA comprising a wild type apoaequorin gene; a method which involves irradiating ultraviolet ray; a genetic engineering technique; or the like.

A genetic engineering technique, the site-directed mutagnesis, is useful because it enables introduction of a specific mutation into a certain position. The site-directed mutagenesis can be performed according to methods described in Molecular Cloning, 2^{nd} ed., Current Protocols in Molecular Biology, Nucleic Acids Research, 10, 6487, 1982, Nucleic Acids Research, 12, 9441, 1984, Nucleic Acids Research, 13, 4431, 1985, Nucleic Acids Research, 13, 8749, 1985, Proc. Natl. Acad. Sci. USA, 79, 6409, 1982, Proc. Natl. Acad. Sci. USA, 82, 488, 1985, Gene, 34, 315, 1985, Gene, 102, 67, 1991, or the like.

When mutation is introduced into a partial fragment of wild type apoaequorin gene, preferably, the fragment is subsequently ligated to other fragments to form a full-length, mutated apoaequorin gene. The nucleotide sequence of the mutated apoaequorin gene obtained can be confirmed by a DNA sequencing method.

As previously described in the present specification, examples of apoaequorin variants that have been reported so far include a variant emitting stronger light (Proc. Natl. Acad. Sci. USA, 86, 80, 1989; United States Patent No. 5,541,309), a variant having enhanced sensitivity to calcium ion (Biochemical Journal, 228, 745, 1985), a variant having enhanced stability to heat or freeze-drying (Japanese Published Unexamined Patent Application No. 97/278796), a variant altered to be localized in endoplasmic reticulum (EMBO J., 14, 5467, 1995), and the like. Genes encoding these variants can also be used in the present invention.

Examples of DNA encoding a variant of cameleon protein include a DNA nucleotide sequence having variation (for example, mutation) in the original cameleon gene. Specific examples of DNA encoding cameleon protein or the variant thereof include the following DNA:
(e) DNA encoding cameleon;
(f) DNA encoding a protein which comprises an amino acid sequence derived from the amino acid sequence of cameleon by deletion, substitution or addition of one or more amino acids, and which is capable of causing fluorescence resonance energy transfer (FRET) upon binding with calcium ion;
(g) DNA encoding a protein which comprises an amino acid sequence having 50% or more homology with the amino acid sequence of cameleon, and which is capable of causing fluorescence resonance energy transfer (FRET) upon binding with calcium ion;
(h) DNA capable of hybridizing to any one of the DNAs of (e) to (g) under stringent conditions, and encoding a protein capable of causing fluorescence resonance energy transfer (FRET) upon binding with calcium ion.

The above described "an amino acid sequence derived from the amino acid sequence of cameleon by deletion, substitution or addition of one or more amino acids" means an amino acid sequence which is derived from the amino acid sequence of original cameleon protein by deletion, substitution or addition of any number of amino acids, for example, 1 to 20, preferably 1 to 15, more preferably 1 to 10, and further more preferably 1 to 5.

The above described "an amino acid sequence having 50% or more homology with the amino acid sequence of cameleon" means, when calculated with analytical software, such as BLAST (J.Mol.Biol., 215, 403, 1990) or FASTA (Methods in Enzymology, 183, 63-69, 1990), an amino acid sequence showing at least 50% or more, preferably 60% or more, more preferably 70% or more, further more preferably 80% or more, further more preferably 85% or more, further more preferably 90% or more, particularly preferably 95% or more, and most preferably 98% or more sequence identity at the amino acid level with original cameleon protein.

The above described "DNA capable of hybridizing under stringent conditions and encoding a protein capable of causing fluorescence resonance energy transfer (FRET) upon binding with calcium ion" means DNA which can be obtained by the colony hybridization method, plaque hybridization method, or the like using as a probe DNA encoding original cameleon. Specifically, such DNA can be identified by performing hybridization at 42°C to 68°C in the presence of 0.7 to 1.0 mol/l sodium chloride using a filter to which DNA derived from a colony or a plaque has been immobilized, and then washing the filter at a temperature condition of 42°C to 68°C using 0.1 to 2 x SSC solution (the composition of 1 x SSC solution comprises 150 mmol/l sodium chloride and 15 mmol/l sodium citrate). Hybridization can be performed according to methods described in Molecular Cloning, 2^{nd} ed., Current Protocols in Molecular Biology, DNA Cloning 1: Core Techniques, A Practical Approach, Second Edition, Oxford University (1995), or the like. Specific examples of DNA capable of hybridizing as described above include, when calculated with BLAST, FAST or the like, those having at least 50% or more, preferably 60% or more, more preferably 70% or more, further more preferably 80% or more, further more preferably 85% or more, further more preferably 90% or more, particularly preferably 95% or more, and most preferably 98% or more homology with the nucleotide sequence of the DNA encoding original cameleon.

The above described "a protein capable of causing fluorescence resonance energy transfer (FRET) upon binding with calcium ion" means a protein which can cause fluorescence resonance energy transfer by changing its three-dimensional structure upon binding with calcium ion in a manner similar to that of the original cameleon protein.

The gene sequence of a variant of cameleon protein as described above can be produced, in a manner similar to a technique to obtain a mutated apoaequorin gene, by any method known in the art, such as chemical synthesis, genetic engineering technique, site-directed mutagenesis, or the like.

Further, in the present invention, examples of a reporter protein for labeling cells include reporter proteins which are expressed within a cell, and can transduce signal about identification of labeled cells by exhibiting any function such as emitting fluorescence, or catalyzing color development or light emmision by acting on any substrate. Specific examples of such a reporter protein include a fluorescent protein, photo- or chromogenic protein, or the variants thereof.

Examples of DNA encoding fluorescent protein include green fluorescent proteins (GFP) derived from *Aequorea* (*Aequorea Victoria*), EGFP, EBFP, EYFP, GFPuv, which are commercially available from Clontech; red fluorescent proteins, DsRed derived from sea anemone (*Discosoma striata*); GFP Emerald, GFP Topaz, GFP Sapphire and GFP S65T which are available from Aurora Biosciences; fluorescent proteins described in Nature Biotech., 18, 313, (2000) or Nature Cell Biol., 2, 25, (2000), or the like.

Examples of DNA encoding a photo- or a chromogenic protein include luciferase which is derived from firefly (*Photinus pyralis*) and which is commercially available from Promega; luciferase derived from Renilla (*Renilla reniformis*); chloramphenicol acetyltransferase; alkaline phosphatase SEAP which is derived from human placenta and which is commercially available from Clontech; β-galactosidase; and the like.

An example of DNA encoding a variant of a fluorescent protein is a DNA nucleotide sequence which has variation (for example, mutation) in that of the gene of the original fluorescent protein. Specific examples of DNA encoding a fluorescent protein or the variant thereof include the DNA of the following (a) to (e):
(a) DNA encoding the green fluorescent protein (GFP) of jelly fish;
(b) DNA encoding the red fluorescent protein (DsRed) of sea anemone;
(c) DNA encoding a protein which comprises an amino acid sequence derived from the amino acid sequence of the fluorescent protein of (a) or (b) by deletion, substitution, or addition of one or more amino acids, and which is capable of emitting fluorescence;
(d) DNA encoding a protein which comprises an amino acid sequence having 50% or more homology with the amino acid sequence of the fluorescent protein of (a) or (b), and which is capable of emitting fluorescence;
(e) DNA capable of hybridizing to any one of the DNAs of (a) to (d) under stringent conditions and encoding a protein capable of emitting fluorescence.

The above described "an amino acid sequence derived from the amino acid sequence of a fluorescent protein by deletion, substitution or addition of one or more amino acids" means an amino acid sequence which is derived from the amino acid sequence of the original fluorescent protein by deletion, substitution or addition of any number of amino acids, for example, 1 to 20, preferably 1 to 15, more preferably 1 to 10, and further more preferably 1 to 5.

The above described "an amino acid sequence having 50% or more homology with the amino acid sequence of a fluorescent protein" means, when calculated with analytical software, such as BLAST (J.Mol.Biol., 215, 403, 1990) and FASTA (Methods in Enzymology, 183, 63-69, 1990), an amino acid sequence showing at least 50% or more, preferably 60% or more, more preferably 70% or more, further more preferably 80% or more, further more preferably 85% or more, further more preferably 90% or more, particularly preferably 95% or more, and most preferably 98% or more sequence identity at the amino acid level with the original fluorescent protein.

The above described "DNA capable of hybridizing under stringent conditions and encoding a protein capable of emitting fluorescence" means DNA which can be obtained by the colony hybridization method, plaque hybridization method, or the like using as a probe DNA encoding the original fluorescent protein. A specific example is DNA which can be identified by performing hybridization at 42°C to 68°C in the presence of 0.7 to 1.0 mol/l sodium chloride using a filter to which DNA derived from a colony or a plaque has been immobilized, and then washing the filter at a temperature condition of 42°C to 68°C using a 0.1 to 2 x SSC solution (the composition of a 1 x SSC solution comprises 150 mmol/l sodium chloride and 15 mmol/l sodium citrate). Hybridization can be performed according to methods described in Molecular Cloning, 2^{nd} ed., Current Protocols in Molecular Biology, DNA Cloning 1: Core Techniques, A Practical Approach, Second Edition, Oxford University (1995), or the like. A specific example of DNA capable of hybridizing is, when calculated with BLAST, FAST or the like, DNA comprising a nucleotide sequence having at least 50% or more, preferably 60% or more, more preferably 70% or more, further more preferably 80% or more, further more preferably 85% or more, further more preferably 90% or more, particularly preferably 95% or more, and most preferably 98% or more homology with the nucleotide sequence of the DNA encoding the original fluorescent protein.

The above described "a protein capable of emitting fluorescence" means a protein which can emit fluorescence when irradiated with excitation light in a manner similar to that of the original fluorescent protein.

The gene sequence of a variant of the fluorescent protein described above can be produced, in a manner similar to a technique to obtain a mutated apoaequorin gene, by any method known in the art, such as chemical synthesis, genetic engineering technique, site-directed mutagenesis, or the like.

An example of DNA encoding a variant of a photo- or a chromogenic protein is a DNA nucleotide sequence which has variation (for example, mutation) in that of the gene of the original photo- or chromogenic protein. Specific examples of DNA encoding a photo- or a chromogenic protein or the variant thereof include the DNA of the following (a) to (h):
(a) DNA encoding luciferase of firefly;
(b) DNA encoding luciferase of *Renilla*;
(c) DNA encoding alkaline phosphatase:
(d) DNA encoding β-galactosidase;
(e) DNA encoding chroramphenicol acetyltransferase;
(f) DNA encoding a protein which comprises an amino acid sequence derived from the amino acid sequence of the photo- or chromogenic protein according to any one of (a) to (e) by deletion, substitution, or addition of one or more amino acids, and which is capable of catalyzing light emission or color development by acting on any substrate;
(g) DNA encoding a protein which comprises an amino acid sequence having 50% or more homology with the amino acid sequence of a photo- or a chromogenic protein of any one of (a) to (e), and which is capable of catalyzing light emission or color development by acting on any substrate;
(h) DNA capable of hybridizing to any one of the DNAs of (a) to (g) under stringent conditions and encoding a protein which is capable of catalyzing light emission or color development by acting on any substrate.

The above described "an amino acid sequence derived from the amino acid sequence of a photo- or a chromogenic protein by deletion, substitution or addition of one or more amino acids" means an amino acid sequence which is derived from the amino acid sequence of an original photo- or chromogenic protein by deletion, substitution or addition of any number of amino acids, for example, 1 to 20, preferably 1 to 15, more preferably 1 to 10, and further more preferably 1 to 5.

The above described "an amino acid sequence having 50% or more homology with the amino acid sequence of a photo- or chromogenic protein" means, when calculated with analytical software, such as BLAST (J.Mol.Biol., 215, 403, 1990) and FASTA (Methods in Enzymology, 183, 63-69, 1990), an amino acid sequence showing at least 50% or more, preferably 60% or more, more preferably 70% or more, further more preferably 80% or more, further more preferably 85% or more, further more preferably 90% or more, particularly preferably 95% or more, and most preferably 98% or more sequence identity at the amino acid level with the original photo- or chromogenic protein.

The above described "DNA capable of hybridizing under stringent conditions and encoding a protein capable of catalyzing light emission or color development by acting on any substrate" means DNA which can be obtained by the colony hybridization method, plaque hybridization method, or the like using as a probe DNA encoding the original photo- or chromogenic protein. A specific example is DNA which can be identified by performing hybridization at 42°C to 68°C in the presence of 0.7 to 1.0 mol/l sodium chloride using a filter to which DNA derived from a colony or a plaque has been immobilized, and then washing the filter at a temperature condition of 42°C to 68°C using a 0.1 to 2 x SSC solution (the composition of a 1 x SSC solution comprises 150 mmol/l sodium chloride and 15 mmol/l sodium citrate). Hybridization can be performed according to methods described in Molecular Cloning, 2^{nd} ed., Current Protocols in Molecular Biology, DNA Cloning 1: Core Techniques, A Practical Approach, Second Edition, Oxford University (1995), or the like. A specific example of DNA capable of hybridizing as described above is, when calculated with BLAST, FASTA or the like, DNA comprising a nucleotide sequence having at least 50% or more, preferably 60% or more, more preferably 70% or more, further more preferably 80% or more, further more preferably 85% or more, further more preferably 90% or more, particularly preferably 95% or more, and most preferably 98% or more homology with the nucleotide sequence of the DNA encoding the original photo- or chromogenic protein.

The gene sequence of a variant of the photo- or chromogenic protein described above can be produced, in a manner similar to a technique to obtain a mutated apoaequorin gene, by any method known in the art, such as chemical synthesis, genetic engineering technique, site-directed mutagenesis, or the like.

The transgenic non-human mammal of the present invention expresses DNA encoding a foreign reporter protein for monitoring changes in intracellular calcium ion concentration in one or more types of non-neuronal cells, preferably in one or more, more preferably 2 or more, further more preferably 3 or more, further more preferably 5 or more, further more preferably 10 or more, particularly preferably 15 or more, and most preferably in all of the tissues or organs selected from the group consisting of epithelial tissue, connective tissue, adipose tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail.

In the present specification, each term of the description "epithelial tissue, connective tissue, adipose tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail" means the broadest sense thereof. Examples of epithelial tissue includes skin, and a hair root; the connective tissue includes skin, and a tail; the bone tissue includes the cranial bone; the muscle tissue includes the abdominal muscle, and diaphragm; the intraoral tissue includes the tongue; the blood tissue includes blood; the cardiovascular organ includes a blood vessel, and heart; the lymphatic organ includes thymus, and spleen; the digestive tract includes the esophagus, stomach, small intestine, and colon; the respiratory organ includes the lung; the urinary organ includes the kidney, and bladder; the genital organ includes the testis, seminal vesicle, penis, ovary, uterus, and oviduct; the endocrine organ includes the thyroid gland, and adrenal gland; and the sense organ includes ears, and crystalline lens, but are not limited thereto.

Examples of a promoter sequence which allows a target DNA to be expressed in a subject animal include a promoter sequence which enables expression of the DNA in at least one or more non-neuronal cells of a subject animal (for example, cells of one or more tissues or organs selected from a group consisting of epithelial tissue, connective tissue, adipose tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail).

Examples of a promoter used in the present invention include a promoter of a gene derived from a virus (such as a cytomegalovirus, Moloney leukemia virus, JC virus, mammary tumor virus, simian virus, and retrovirus); a promoter of a gene [for example, genes of albumin, insulin II, erythropoietin, endothelin, osteocalcin, muscle creatine kinase, platelet derived growth factor β, keratin K1, K10 and K14, collagen type I and II, atrial natriuretic factor, dopamine β-hydroxylase, endothelial receptor tyrosine kinase (generally abbreviated as Tie2), sodium potassium adenosine triphosphatase (generally abbreviated as Na, K-ATPase), neurofilament light chain, metallothionein I and IIA, tissue inhibitor of metalloproteinase 1, MHC class I antigen, (generally abbreviated as H-2L), smooth muscle α actin, polypeptide chain elongation factor 1α (EF-1α), β actin, α and (β myosin heavy chain, myosin light chain 1 and 2, myelin basic protein, serum amyloid P component, myoglobin, renin, glycolytic enzyme, heat shock protein and the like] derived from various mammals (such as a human, rabbit, dog, cat, guinea pig, hamster, rat and mouse) and birds (such as a chicken), and a promoter in which an enhancer sequence and a promoter sequence of various genes are fused (for example, genes of SRα promoter, CAG promoter and the like).

Cytomegalovirus promoter, human and chicken β actin promoters, and the like are preferably used in the present invention. In particular, a fusion promoter comprising an enhancer of IE (immediate early) gene of human cytomegalovirus and each of the above promoters is preferably used.

Specific examples of promoter sequences preferably used in the present invention include the following (a) to (i):
(a) DNA having SE promoter sequence;
(b) DNA having CAG promoter sequence;
(c) DNA having PGK promoter sequence;
(d)DNA having a sequence in which hprt gene is inserted between two types of promoters;
(e) DNA in which loxP, promoter, hprt gene, loxP and promoter are ligated in this order;
(f) DNA comprising a nucleotide sequence represented by SEQ ID NO: 1;
(g) DNA comprising a nucleotide sequence derived from the nucleotide sequence of any one of the DNAs of (a) to (f) by deletion, substitution, or addition of one or more nucleotides and having promoter activity;
(h) DNA comprising a nucleotide sequence which has 50% or more homology with the nucleotide sequence of any one of the DNAs of (a) to (f) and having promoter activity;
(i) DNA capable of hybridizing to any one of the DNAs of (a) to (f) under stringent conditions and having promoter activity.

The above (g) "a nucleotide sequence derived from the nucleotide sequence by deletion, substitution, or addition of one or more nucleotides" means a nucleotide sequence which is derived from the nucleotide sequence of the DNA by deletion, substitution or addition of any number of nucleotides, for example, 1 to 20, preferably 1 to 15, more preferably 1 to 10, and further more preferably 1 to 5.

The above described "a nucleotide sequence having 50% or more homology with the nucleotide sequence" means, when calculated with analysis software, such as BLAST, FASTA or the like, a nucleotide sequence having at least 50% or more, preferably 60% or more, more preferably 70% or more, further more preferably 80% or more, further more preferably 85% or more, further more preferably 90% or more, particularly preferably 95% or more, and most preferably 98% or more sequence identity at DNA level with the nucleotide sequence.

The above described "DNA capable of hybridizing under stringent conditions and having promoter activity" means DNA which can be obtained by the colony hybridization method, plaque hybridization method, Southern blot hybridization method, or the like using as a probe DNA having the nucleotide sequence of SEQ ID NO: 1. A specific example is DNA which can be identified by performing hybridization at 42°C to 68°C in the presence of 0.7 to 1.0 mol/l sodium chloride using a filter to which DNA derived from a colony or a plaque has been immobilized, and then washing the filter at a temperature condition of 42°C to 68°C using a 0.1 to 2 x SSC solution (the composition of a 1 x SSC solution comprises 150 mmol/l sodium chloride and 15 mmol/l sodium citrate). Hybridization can be performed according to methods described in Molecular Cloning, 2^{nd} ed., Current Protocols in Molecular Biology, DNA Cloning 1: Core Techniques, A Practical Approach, Second Edition, Oxford University (1995), or the like. A specific example of DNA capable of hybridizing as described above is, when calculated with FASTA or the like, DNA having at least 50% or more, preferably 60% or more, more preferably 70% or more, further more preferably 80% or more, further more preferably 85% or more, further more preferably 90% or more, particularly preferably 95% or more, and most preferably 98% or more homology with the nucleotide sequence of SEQ ID NO: 1.

The variant comprising a mutated sequence of SEQ ID NO: 1 described above can be produced, in a manner similar to a technique to obtain a mutated apoaequorin gene, by any method known in the art, such as chemical synthesis, genetic engineering technique, site-directed mutagenesis, or the like.

Any of the promoters described in the above (d) and (e) can be used so far as it has promoter activity. A preferred example is a combination of two types of promoters, PGK promoter and CAG promoter.

The transgenic animal of the present invention is produced by introducing a target gene into, for example, germinal cells or the like, such as an unfertilized egg, a fertilized egg, a sperm and the primordial germ cell thereof, preferably at the developmental stage of embryogenesis (more preferably, the stage of single cell or fertilized egg cell, and generally, at the stage of before eight-cell-stage) of a non-human mammal, using a calcium phosphate method, electric pulse method, lipofection method, aggregation method, microinjection method, particle gun method, DEAE-dextran method, virus vector method, or the like. Further, a transgenic animal can also be produced by introducing DNA encoding a foreign reporter protein for monitoring changes in intracellular calcium ion concentration into an embryonic stem cell by the above method for introducing a gene, and producing a chimera individual of the germ line introduced with a target gene by the aggregation chimera method, injection chimera method, or the like using the embryonic stem cell and a fertilized egg of a non-human mammal. Furthermore, a transgenic animal can also be produced by introducing DNA encoding a foreign reporter protein for monitoring changes in intracellular calcium ion concentration into a cell by the calcium phosphate method, electric pulse method, lipofection method, aggregation method, microinjection method, particle gun method, DEAE-dextran method, virus vector method, or the like, and transplanting the nucleus of the cell into an enucleated and unfertilized egg of a non-human mammal using an apparatus, such as a micromanipulater, to obtain a cloned individual.

Specific examples of the term "non-human mammal" of the present specification include a mouse, rat, guinea pig, hamster, rabbit, dog, cat, sheep, pig, goat, cow, monkey and the like. A preferred example is a rodent, such as a mouse, rat, guinea pig, hamster or rabbit. The most preferred example is a mouse.

The term "non-human mammal" of the present specification may be a non-human mammal having a transgene on the chromosome, such as various disease model animals.

The production method and the application of the transgenic non-human mammal of the present invention will now be described in detail.

### 1. Production method of a transgenic non-human mammal of the present invention

### (1) Preparation of transgene

To introduce DNA encoding a foreign reporter protein for monitoring changes in intracellular calcium ion concentration into a subject animal, it is generally advantageous to use a gene construct in which the DNA is ligated downstream of a promoter which enables expression of the DNA in a cell of the subject animal for introduction of the DNA. A gene to be introduced can be prepared, for example, by the following procedure using the method described in Molecular Cloning, 2^{nd} ed., Current Protocols in Molecular Biology, or the like.

A DNA fragment with an appropriate length containing a portion encoding the protein is prepared, as necessary, based on the full length complementary DNA of a gene to be introduced. Then, the DNA fragment or the full length complementary DNA is inserted downstream of the promoter of an appropriate expression vector, thereby constructing a recombinant vector.

Examples of an expression vector used herein for a gene to be introduced into an animal include a plasmid derived from *Escherichia coli,* a plasmid derived from *Bacillus subtilis,* a plasmid derived from yeast, a bacteriophage, such as λ phage, a retrovirus, such as Moloney leukemia virus, an animal virus, such as vaccinia virus or baculo virus, and the like. In particular, a plasmid derived from *Escherichia coli, a* plasmid derived from *Bacillus subtilis*, or a plasmid derived from yeast is preferably used, and the plasmid derived from *E. coli* is particularly preferred.

Specific examples of a promoter used in the present invention are as described above in the present specification.

An expression vector used in the present invention preferably has a sequence (generally referred to as a terminator) which terminates a target mRNA transcription in a transgenic mammal. For example, gene expression can be regurated using a sequence having the same terminator function, which is comprised in each gene derived from viruses, various mammals and birds. Preferably, SV40 terminator of simian virus, or the like, is often used. For the purpose of enabling higher expression of a target gene, a splicing signal and an enhancer region of each gene, and a part of an intron of an eukaryotic gene can be ligated 5' upstream of a promoter region, between the promoter region and translated region, or 3' downstream of the translated region, depending on the purpose.

As DNA (for example, apoaequorin gene) encoding a foreign reporter protein for monitoring changes in intracellular calcium ion concentration, commercially available DNA (for example, those manufactured by Molecular Probes) may be used, or the DNA may be prepared from a biological tissue [for example, luminous *Aequorea* (*Aequorea Victoria*)] as follows.

RNA is prepared from a biological tissue by a known method, and then a complementary DNA is produced.

Examples of a method for preparing a total RNA from a biological tissue include a guanidine thiocyanate cesium trifluoroacetate method (Methods in Enzymology, 154, 3, 1987), acid guanidine thiocyanate-phenol-chloroform (AGPC) method (Analytical Biochemistry, 162, 156, 1987; Jikken Igaku, Experimental Medicine, 9, 1937, 1991), and the like. Examples of a method for preparing mRNA as poly (A)⁺RNA from total RNA include an oligo (dT) immobilized cellulose column method (Molecular Cloning, 2^{nd} ed.). Further, mRNA can also be prepared using a kit, such as Fast Track mRNA Isolation Kit (Invitrogen) and Quick Prep mRNA Purification Kit (Pharmacia). Examples of a method for synthesizing complementary DNA include methods described in Molecular Cloning, 2^{nd} ed., Current Protocols in Molecular Biology, or the like, or a method using a commercial kit, such as a Superscript Plasmid System for cDNA Synthesis and Plasmid Cloning (Life Technologies), or ZAP-cDNA Synthesis Kit (STRATAGENE). Using the obtained complementary DNA as a template, DNA is amplified by the polymerase chain reaction method (PCR) [PCR Protocols, Academic Press (1990)], so that a gene containing a target translated region can be obtained. The nucleotide sequence of the obtained gene can be determined by analyzing by a generally employed method for sequence analysis, for example, the dideoxy method of Sanger et al. (Proc. Natl. Acad. Sci. USA, 74, 5463, 1977), or using a nucleotide sequence analyzer, such as an ABIPRISM377 DNA sequencer (PE Biosystems). Moreover, the mutated translated region for the above protein obtained from the tissue can be prepared by site-directed mutagenesis or the like described in Nucleic Acids Research, 10 6487, 1982, Proc. Natl. Acad. Sci. USA, 79, 6409, 1982, Gene, 34, 315, 1985, Nucleic Acids Research, 13, 4431, 1985, Proc. Natl. Acad. Sci. USA, 82, 488, 1985, and the like. All of these are materials that can be used for a transgenic animal. The translated region is ligated downstream (preferably, upstream of the transcription termination site) of the aforementioned promoter, as a gene construct that can be expressed in a transgenic animal, according to a standard genetic engineering technique, so that DNA in which DNA encoding a foreign reporter protein for monitoring changes in intracellular calcium ion concentration has been integrated can be prepared.

### (2) Production of transgenic non-human mammal

The transgenic non-human mammal of the present invention can be produced by introducing DNA encoding a foreign reporter protein for monitoring changes in intracellular calcium ion concentration into a subject animal. Specifically, the gene is introduced into a fertilized egg, embryonic stem cell, somatic cell, sperm, or unfertilized egg of a subject non-human mammal, so that a transgenic non-human mammal in which a DNA encoding a foreign reporter protein for monitoring changes in intracellular calcium ion concentration has been integrated into the chromosome of all the cells including germinal cells can be obtained. From the obtained individual animals, those in which the transgene product is present in at least one or more non-neuronal cells are selected, thereby producing the transgenic non-human mammal. The gene is introduced into a fertilized egg, embryonic stem cell, somatic cell, sperm, or unfertilized egg so as to ensure the presence of the gene on the chromosome of all the cells including germinal cells and somatic cells of the subject non-human mammal. The presence of the transgene in the germinal cell of the produced animal after introduction of the gene means that the progeny of the produced animal have the transgene in all germinal cells and somatic cells.

Individual animals are selected by confirming expression of the transgene product in at least one or more non-neuronal cells at the protein level or the mRNA level. The offsprings of the animal inhering the gene from the selected individual have the transgene product in at least one or more non-neuronal cells. Then, the homozygous animals having the transgene on both the homologous chromosomes are obtained. By mating the male with the female homozygous animal, all the offsprings stably retain the gene. Further, the animals can be bred under normal breeding conditions while confirming the offsprings have the gene.

Production of a transgenic animal by gene introduction into a fertilized egg or an embryonic stem cell can be performed by methods described in Manipulating Mouse Embryo, 2^{nd} ed.; Gene Targeting, A Practical Approach, IRL Press at Oxford University Press (1993); Bio Manual Series 8, Gene Targeting, Production of Mutant Mouse using ES cell, YODOSHA (1995); Development Engineering Experimental Manual, How to Produce Transgenic Mice, KODANSHA (1987) and the like. Production of a transgenic animal by gene introduction and nuclear transplantation into mammalian cells can be performed by methods reported by Cibelli et al. (Science, 280, 1256, 1998), Schnieke et al. (Science, 278, 2130, 1997), Baguisi et al. (Nature Biotechnology, 17, 456, 1999) and the like. Production of a transgenic animal by gene introduction into a sperm can be performed by methods reported by Perry et al. (Science, 284, 1180, 1999) and by Wakayama et al. (Nature Biotechnology, 16, 639, 1998). Production of a transgenic animal by gene introduction into an unfertilized egg and *in vitro* fertilization can be performed by a method reported by Chan et al. (Proc. Natl. Acad. Sci. USA, 95, 14028, 1998). For example, a transgenic animal can be produced by the following techniques.

### (a) Production of a transgenic animal by gene introduction into a fertilized egg

A fertilized egg to be used for transferring a foreign gene (for example, DNA encoding a foreign reporter protein for monitoring changes in intracellular calcium ion concentration) different from an endogenous gene of a subject transgenic animal into the fertilized egg of the subject non-human mammal or the progenitor thereof, is obtained by mating a male non-human mammal of the same species (preferably, a mouse, rat or the like, particularly preferably an inbred male mouse, Wister male rat or the like) with a female non-human mammal (preferably a female mouse, female rat or the like, particularly preferably an inbred female mouse, Wister female rat or the like). A fertilized egg can also be obtained by natural mating. However, a preferred method involves artificially regulating the estrous cycle of a female non-human mammal (preferably a female mouse, female rat or the like, particularly preferably an inbred female mouse, Wister female rat or the like), and then mating the female non-human mammal with a male non-human mammal (preferably a male mouse, male rat or the like, particularly preferably an inbred male mouse, Wister male rat or the like). A preferred method for artificially regulating the estrous cycle of a female non-human mammal involves, for example, a first step of administering follicle stimulating hormone (pregnant mare serum gonadotropin, generally abbreviated as PMSG), followed by luteinizing hormone (human chorionic gonadotropin, generally abbreviated as hCG) by, for example, peritoneal injection. A preferred dosage of hormone and a dosage interval vary depending on the types of non-human mammals. When an inbred mouse and Wister rat are used, they are preferably 8-week old or more after breeding for about 1 week under a condition of a light period of approximately 12 hours (for example, 7:00 to 19:00). When a non-human mammal is a female mouse (preferably an inbred female mouse), normally, a preferred method involves administering luteinizing hormone about 48 hours after administration of follicle stimulating hormone, and mating the female mouse with a male mouse to obtain a fertilized egg. The dosage of follicle stimulating hormone is approximately 2 to 20 IU/mouse, and preferably approximately 5 IU/mouse. The dosage of luteinizing hormone is approximately 0 to 10 IU/mouse, and preferably approximately 5 IU/mouse. When a non-human mammal is a female rat (preferably a Wister female rat), normally, a preferred method involves, administering luteinizing hormone about 48 hours after administration of follicle stimulating hormone, and mating the female rat with a male rat to obtain a fertilized egg. The dosage of follicle stimulating hormone is approximately 20 to 50 IU/rat, and preferably approximately 30 IU/rat. The dosage of luteinizing hormone is approximately 0 to 10 IU/rat, and preferably approximately 5 IU/rat.

Subsequently, a foreign gene (for example, DNA encoding a foreign reporter protein for monitoring changes in intracellular calcium ion concentration) is introduced into the obtained fertilized egg by, for example, a microinjection method or retrovirus method, and then the fertilized egg is artificially transplanted into a female non-human mammal to implant, thereby obtaining a non-human mammal having DNA with an introduced foreign gene integrated therein. When a gene is introduced by the microinjection method, it is preferred to use a fertilized egg which is at about 12 to 24 hours after fertilization and contains a male pronucleus formed therein. A foreign gene that is introduced by the microinjection method may be either in a cyclic or a linear form. Preferably, a foreign gene is linearized before introduction in such a way that the portions of structural gene to be expressed and expression regulatory region are not disrupted. When a gene is introduced by a method using retrovirus, a fertilized egg at a developmental stage before the compacted morula (generally, before eight-cell-stage) is preferably used. When a fertilized egg is transplanted into a female non-human mammal, a preferred method involves mating with a vasoligated male non-human mammal to induce fertility of a pseudopregnant female non-human mammal, and artificially transplanting the obtained fertilized egg into the pseudopregnant female for implantation. A pseudopregnant female non-human mammal having an induced fertility can also be obtained by natural mating. However, a pseudopregnant female non-human mammal can also be obtained by administering luteinizing hormone releasing hormone (hereinafter, abbreviated as LHRH) or its analogue, and mating with a male non-human mammal. The dosage of LHRH or its analogue and the timing of mating with a male non-human mammal after administration vary depending on the types of non-human mammals. When a non-human mammal is a female rat (preferably a Wister female rat), normally, the female rat is preferably mated with a male rat about 4 days after administration of LHRH or its analogue (for example, [3,5-Dil-Tyr⁵]-LH-RH, [Gln⁸]-LH-RH, [D-Ala⁶]-LH-RH, des-Gly¹⁰, [D-His(Bzl)⁶]-LH-RH) Ethylamide and the like). The dosage of LHRH or its analogue is normally about 10 to 60 µg/rat, preferably about 40 µg/rat.

### (b) Production of a transgenic animal by gene introduction into an embryonic stem cell

A non-human chimera mammal having DNA with an introduced foreign gene incorporated therein is obtained by introducing a foreign gene (for example, DNA encoding a foreign reporter protein for monitoring changes in intracellular calcium ion concentration) into the chromosome of an embryonic stem cell of a non-human mammal (for example, a mouse, rat, pig, monkey or the like); allowing a fertilized egg of a non-human mammal to incorporate the obtained embryonic stem cell by an aggregation chimera method or injection chimera method; and artificially transplanting the fertilized egg into a female non-human mammal for implantation. A known technique for gene introduction (for example, a calcium phosphate method, electric pulse method, lipofection method, aggregation method, microinjection method, particle gun method, DEAE-dextran method, and virus vector method) can be employed to introduce a foreign gene into an embryonic stem cell. A foreign gene to be introduced may be in either a cyclic or a linear form. Preferably, a foreign gene is linearized before introduction in such a way that the portions of structural gene to be expressed and expression regulatory region are not disrupted. When a fertilized egg of a non-human mammal is allowed to incorporate an embryonic stem cell by the aggregation chimera method, generally, a fertilized egg at a developmental stage before eight-cell-stage is preferably used. When a fertilized egg of a non-human mammal is allowed to incorporate an embryonic stem cell by the injection chimera method, a fertilized egg at a developmental stage generally ranging from eight-cell-stage to blastcyst stage is preferably used. When a fertilized egg is transplanted into a female non-human mammal, a preferred method involves mating with a vasoligated male non-human mammal to induce fertility of a pseudopregnant female non-human mammal, and artificially transplanting the obtained fertilized egg into the pseudopregnant female for implantation. A pseudopregnant non-human mammal can be obtained by the abovementioned known methods.

By mating an individual (preferably, male) having a high chimeric ratio selected from non-human chimera mammals having DNA in which a foreign gene (for example, DNA encoding a foreign reporter protein for monitoring changes in intracellular calcium ion concentration) is integrated, with a non-human mammal (preferably, female), there can be obtained an individual having the foreign gene (for example, DNA encoding a foreign reporter protein for monitoring changes in intracellular calcium ion concentration) introduced into the chromosome of the cells of the whole body. Further, by mating the male individual with the female individual, there can be obtained a homozyous transgenic non-human mammal having the foreign gene introduced into both of the homologous chromosomes.

By combinating with the Cre-loxP system, expression time, expression level and the like of a introduced foreign gene (for example, DNA encoding a foreign reporter protein for monitoring changes in intracellular calcium ion concentration) can be controlled more actively. Known examples of such a case include a case in which a target gene has been deleted only in a certain region of the brain using a promoter expressed in the region (Cell, 87, 1317, 1996) and a case in which a target gene has been deleted organ-specifically at a time of interest using adeno virus expressing Cre (Science, 278, 5335, 1997). Therefore, a foreign gene (for example, DNA encoding a foreign reporter protein for monitoring changes in intracellular calcium ion concentration) on the chromosome can also be controlled to express at any time and in any amount.

### (c) Production of a transgenic animal by gene introduction and nuclear transplantation into mammalian cells

A foreign gene (for example, DNA encoding a foreign reporter protein for monitoring changes in intracellular calcium ion concentration) is introduced into the chromosome of cells of a non-human mammal (preferably, mouse, sheep, goat, cow or the like); the obtained cell nucleus is initialized (this manipulation restores the state at which the nucleus can repeat development again); development is started by a method which involves injecting the nucleus into an enucleated and unfertilized egg of a non-human mammal; the egg that has started development is artificially transplanted into a female non-human mammal to implant; thereby obtaining a non-human mammal having DNA with the foreign gene integrated therein. Known methods for gene introduction (for example, a calcium phosphate method, electric pulse method, lipofection method, aggregation method, microinjection method, particle gun method, DEAE-dextran method, and virus vector method) can be used for introducing a foreign gene into a cell. A foreign gene to be introduced may be in either a cyclic or a linear form. Preferably, a foreign gene is linearized before introduction in such a way that the portions of structural gene to be expressed and expression regulatory region are not disrupted. A method to initialize the nucleus of a cell varies depending on the types of non-human mammals. When a non-human mammal is a sheep, goat or cow, preferably, cells with foreign genes introduced therein are cultured in medium (for example, M2 medium) containing 5 to 30%, preferably 10%, fetal calf serum, and then in oligotrophic medium containing 0 to 1 %, preferably 0.5%, fetal calf serum for 3 to 10 days, preferably 5 days, thereby inducing the cell cycle to resting phase (G0 phase or G1 phase) for initialization. When a non-human mammal is a mouse or the like, preferably, the nucleus of a cell with a foreign gene introduced therein is injected into an enucleated and unfertilized egg of a non-human mammal of the same species, followed by culturing for several hours, preferably about 1 to 6 hours, so as to initialize the nucleus. The initialized nucleus can start development in the enucleated and unfertilized egg. A method to start development of the initialized nucleus in the enucleated and unfertilized egg varies depending on the types of non-human mammals. When a non-human mammal is a sheep, goat, cow or the like, preferably, an egg is activated to start development by transplanting the initialized nucleus whose cell cycle has been induced to resting phase (G0 phase or G1 phase) into an enucleated and unfertilized egg of a non-human mammal of the same species by an electric fusion method or the like. When a non-human mammal is a mouse or the like, preferably, development is started by stimulating an unfertilized egg, which was injected with the nucleus of a cell having a foreign gene introduced therein, with an egg activator (for example, strontium) and treating with an inhibitor (for example, cytochalasin B) for cell division for suppression of extrusion of second polar body. The egg that has started its development is artificially transplanted to a female non-human mammal of the same species by a known method to implant, so that a non-human mammal having DNA with a foreign gene (for example, DNA encoding a foreign reporter protein for monitoring changes in intracellular calcium ion concentration) integrated therein can be obtained.

### (d) Production of a transgenic animal by gene introduction into a sperm

A sperm of a non-human mammal (for example, a mouse) is allowed to incorporate a foreign gene (for example, DNA encoding a foreign reporter protein for monitoring changes in intracellular calcium ion concentration); the obtained sperm is introduced into an unfertilized egg by a microfertilization method; and the egg that has started development is artificially transplanted into a female non-human mammal to implant, thereby obtaining a non-human mammal having DNA with the foreign gene incorporated by the sperm integrated into the chromosome. When a sperm is allowed to incorporate a foreign gene, a preferred method involves incubating a sperm with disrupted cell membrane in a medium (for example, a CZB medium or NIM medium) containing DNA with a concentration of approximately 0.5 to 100 ng/ml (preferably, 5 to 10 ng/ml) for several minutes (preferably, approximately 1 min). Preferred methods for disrupting the cell membrane of a sperm are any of the following: a method which involves treating sperms with a surfactant (for example, Triton)(-100) at a low concentration (for example, 0.05%); a method which involves freeze-drying sperms suspended in a medium (for example, CZB medium) containing 10% fetal calf serum but no EDTA; and a method which involves freezing and thawing the above sperm suspension. Although sperms with disrupted cell membranes have lost their autonomous fertility following such a treatment, embryogenesis is promoted by fertilization by a microfertilization method using a micromanipulator or the like. When microfertilization is performed, a preferred method involves injecting a sperm head with a foreign gene incorporated therein into the cytoplasm of an unfertilized egg at the metaphase of the meiotic second division of a female non-human mammal of the same species. The egg that has started development is artificially transplanted by a known method into a female non-human mammal of the same species to implant, so that a non-human mammal having DNA with the foreign gene (for example, DNA encoding a foreign reporter protein for monitoring changes in intracellular calcium ion concentration) integrated therein can be obtained.

### (e) Production of a transgenic animal by gene introduction into an unfertilized egg and in vivo fertilization

An unfertilized egg of a non-human mammal (preferably, a mouse, rat, cow or the like) is infected with retrovirus with a foreign gene (for example, DNA encoding a foreign reporter protein for monitoring changes in intracellular calcium ion concentration) integrated therein so as to introduce the foreign gene; the obtained egg is allowed to develop by an *in vivo* fertilization method; the fertilized egg that has started development is artificially transplanted into a female non-human mammal to implant, thereby obtaining a non-human mammal having DNA with the introduced foreign gene integrated into the chromosome. A retrovirus with a foreign gene integrated therein can be prepared by a method described in Manipulating Mouse Embryo, 2^{nd} ed., or the like. When an unfertilized egg is infected with a recombinant retrovirus, an unfertilized egg from which the zona pellucida has been removed is preferably used. Preferred methods for removing the zona pellucida of an unfertilized egg are any of the following: a method which involves physically removing the membrane using a micromanipulator or the like; and a method which involves removing the membrane by chemical lysis of the membrane using a chemical substance (for example, acidic Tyrode). Eggs that have started development following *in vivo* fertilization are artificially transplanted by a known method into a female non-human mammal of the same species to implant, so that a non-human mammal having DNA with the foreign gene (for example, DNA encoding a foreign reporter protein for monitoring changes in intracellular calcium ion concentration) integrated therein can be obtained.

### 2. Application of the transgenic non-human mammal of the present invention

In the non-human mammal of the present invention having DNA encoding a foreign reporter protein for monitoring changes in intracellular calcium ion concentration, the DNA encoding the reporter protein is highly expressed in at least one or more non-neuron cells (for example, cells in one or more tissues or organs selected from the group consisting of epithelial tissue, connective tissue, fat tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail). Therefore, the transgenic non-human mammal of the present invention is useful in pharmacological evaluation and activity evaluation for physiologically active substances (for example, drug), novel gene products having unknown functions or the like in these tissues or organs.

Changes in intracellular calcium concentration can be measured in pathological model animals which have been produced by inducing heart diseases (for example, acute heart failure, chronic heart failure and myocarditis), respiratory diseases, arthropathy (for example, rheumatoid arthritis and osteoarthritis), renal diseases (for example, kidney failure, glomerular nephritis, and IgA nephropathy), arterial sclerosis, psoriasis, hyperlipidemia, allergic diseases (for example, asthma, allergic rhinitis and atopic dermatitis), bone diseases (for example, osteoporosis, rachitis, osteohalisteresis and hypocalcemia), blood disease, cerebrovascular injury, traumatic encephalopathy, infectious disease, dementia, chronic inflammatory disease, or the like in the transgenic non-human mammal of the present invention. Thus, one can elucidate a pathological mechanism of these diseases, study a method of treatment, and screen candidate compounds for the purpose of developing therapeutic agents, preventive agents and diagnostic agents. In this case, in vivo functions of endogenous physiologically active substances and/or receptors thereof which induce changes in intracellular calcium ion concentration may be further elucidated. Thus, the transgenic non-human mammal of the present invention is also expected to be used as an experimental model for elucidating these mechanisms.

Examples of application of the transgenic non-human mammal of the present invention are as follows.

### (1) A method for detecting in vivo changes in intracellular calcium ion concentration of a living transgenic non-human mammal of the present invention; and a method for screening an agent using the method

Using a living transgenic non-human mammal of the present invention, in vivo changes in intracellular calcium ion concentration can be measured by detecting a luminescence of DNA encoding a reporter protein introduced. An example of a method to detect luminescence of photoprotein from a living body is a method of Contag et al. which involves detecting luminescence of photoprotein from a living animal using an ICCD camera (Molecular Microbiology, 18, 593, 1995; Nature Medicine, 4, 245, 1998). Instead of ICCD, a cooled CCD camera with higher sensitivity can also be used. The method for detecting in vivo changes in intracellular calcium ion concentration of a living mammal enables examination of in vivo physiological changes and drug response, and evaluation and screening of physiologically active substances under an environment closer to the in vivo state. Specifically, a test substance is administered to a transgenic non-human mammal of the present invention, and then changes in intracellular calcium ion concentration are detected using a constitutively-expressing foreign reporter protein, so that a substance involved in the regulation of intracellular calcium ion concentration can be evaluated and screened in an in vivo environment.

### (2) A method for detecting changes in intracellular calcium ion concentration of tissues, organs and cells excised from the transgenic non-human mammal of the present invention, and a method for screening an agent using the method.

Changes in intracellular calcium ion concentration of cultured tissues, organs and cells following excision from the transgenic non-human mammal of the present invention can be measured by detecting luminescence from the reporter protein introduced. Examples of a method for culturing in vitro tissues, organs and cells include known methods described in Cultivation of Animal Tissue, Modem Biology Series 23, KYORITSU SHUPPAN CO., LTD. (1974); Cell Culture Manual, Kodansha Scientific (1982); and Use of Isolated Liver Cells and Kidney Tubules in Metabolic Studies, Elaevier/North-Holland Publishing Co., New York·Amsterdam·Oxford (1976). Examples of a method for detecting luminescence from photoprotein of cultured tissues, organs and cells include the above-mentioned method of Contag et al., a method using a cooled CCD camera, and a method using a luminometer (for example, a luminometer manufactured by EG&G BERTHOLD). The method for measuring changes in intracellular calcium ion concentration in cultured tissues, organs and cells enables evaluation and screening of physiologically active substances in tissues and organs which maintain a complex intercellular network or in the state of primary culture cell which is thought to more easily reflect *in vivo* function. Specifically, a test substance is allowed to contact with tissues, organs or cells prepared from the transgenic non-human mammal of the present invention, and changes in intracellular calcium ion concentration are detected using a constitutively-expressing foreign reporter protein, so that a substance involved in the regulation of intracellular calcium ion concentration can be evaluated and screened in an environment closer to the in vivo state.

### (3) A method for detecting changes in intracellular calcium ion concentration of cells obtained by inducing differentiation of embryonic stem cells to be used in production of the transgenic non-human mammal of the present invention, and a method for screening an agent using the method.

Changes in intracellular calcium ion concentration of cells obtained by inducing differentiation of embryonic stem cells to be used in production of the transgenic non-human mammal of the present invention can be measured by detecting luminescence from the reporter protein introduced. In *in vitro* culturing, it is known that endodermal cells, ectodermal cells, mesodermal cells, blood cells, endothelial cells, cartilage cells, skeletal muscle cells, smooth muscle cells, cardiac muscle cells, neuronal cells, glial cells, epidermal cells, melanocytes and keratinocytes are induced from embryonic stem cells (P. D. Rathjen et al.; Reprod. Fertil. Dev., 10, 31, 1998). An example of a method for obtaining cells induced to differentiate from the embryonic stem cells is a method described in the above-mentioned literature of P. D. Rathjen et al. Examples of a method for detecting luminescence from photoprotein of cells following induction of differentiation include the above-mentioned method of Contag et al., a method using a cooled CCD camera, and a method using a luminometer (for example, a luminometer manufactured by EG&G BERTHOLD). By the method for measuring changes in intracellular calcium ion concentration of cells induced to differentiate from embryonic stem cells, physiologically active substances can also be evaluated and screened in cells which are difficult to excise from a living body, and cells which exist in vivo only in small number.

Regarding application of the transgenic non-human mammal of the present invention described in (I) to (3) above, types of agents subjected to the method for screening an agent are not specifically limited, so far as they are substances that can have some physiological activities. Examples of such a substance can be any substance, such as cytokines, low molecular weight compounds, hormones, antibodies, peptides or peptide mimics, antisense oligonucleotides, and the like.

### (4) Production of a gene-modified animal using an embryonic stem cell, egg, sperm and nucleus of the transgenic non-human mammal of the present invention, and use of the gene-modified animal produced.

Using an embryonic stem cell, egg, sperm and nucleus of the transgenic non-human mammal of the present invention, a gene-modified animal which contains DNA encoding a foreign reporter protein for monitoring changes in intracellular calcium ion concentration, and has a further modified gene on the chromosome, can be obtained. A method for obtaining a gene-modified animal using an embryonic stem cell, egg, sperm and nucleus is, for example, the abovementioned known method. Examples of a method for further modifying a gene on the chromosome include a method for constructing a gene, which is known to cause a disease when its function is disrupted, using a knockout technique by homologous recombination or using a technique for introduction of a dominant-negative mutant gene or the like. Such a gene-modified animal is useful in analysis of the relation between changes in intracellular calcium ion concentration and pathological conditions shown by the gene-modified animal. A gene-modified mammal which contains DNA encoding a foreign reporter protein for monitoring changes in intracellular calcium ion concentration, and has a further modified gene on the chromosome, can be used for the development of medical care, medicaments and the like.

(5) Production of a gene-modified animal by mating the transgenic non-human mammal of the present invention with an animal of the same species but of another line, and use of the gene-modified animal produced.

By mating the transgenic non-human mammal of the present invention with an animal of the same species but of another line (for example, a human disease model animal), a gene-modified mammal which contains DNA encoding a foreign reporter protein for monitoring changes in intracellular calcium ion concentration, and shows a phenotype (for example, symptom analogous to human pathological conditions) can be obtained. Examples of a method of mating include a known *in vitro* fertilization in addition to natural mating. As a disease model animal, many disease model animals are known irrespective of the type of disease, congenital or acquired. These gene-modified animals are useful in analysis of the relation between changes in intracellular calcium ion concentration and a phenotype (for example, symptom analogous to human pathological conditions) shown by the gene-modified animal. Therefore, the gene-modified mammal which contains DNA encoding a foreign reporter protein for monitoring changes in intracellular calcium ion concentration, and shows a phenotype (for example, symptom analogous to human pathological conditions) can be used for the development of medical care, medicaments and the like.

### 3. Construction of the DNA of the present invention

The DNA of the present invention can be constructed by ligating, using appropriate linker DNAs, each DNA sequence of loxP, promoter, hprt gene, loxP and promoter by standard genetic engineering techniques described in Molecular Cloning 2^{nd} ed., or Current Protocols in Molecular Biology, or the like.

A linker DNA is preferably DNA having any restriction enzyme recognition site and a length of several bp to several kbp, preferably 10 bp to 1 kbp, and more preferably 10 bp to 100 bp.

As DNA of a promoter portion, any DNA having promoter activity can be used. Examples of such DNA include a commercially available promoter DNA and a commercially available promoter DNA which is modified based on a nucleotide sequence of a known DNA having promoter activity. Preferred examples of such DNA include a PGK promoter and a CAG promoter.

As a nucleotide sequence of a loxP portion, any nucleotide sequence which is recognized by Cre recombinase to cause recombination can be used. An example of such a nucleotide sequence is a nucleotide sequence described in Nucleic Acids Res., 25, 868, (1997), or Nucleic Acids Res., 23, 485, (1995) or the like.

As hprt gene, any sequence which can be transcribed, translated and expressed as HPRT protein when the sequence is ligated downstream of an appropriate promoter can be used. The sequence of hprt gene is known. For example, it can be obtained from Lexicon Genetics and the like.

Each DNA sequence composing the present invention can also be prepared based on nucleotide sequence information of known DNA by chemical synthesis with a DNA synthesizer, such as DNA synthesizer model 392 (Perkin Elmer), using the phosphoramidite method.

An example of a nucleotide sequence of the DNA of the present invention that can be obtained by the above method is the nucleotide sequence of SEQ ID NO: 1.

### 4. Use of the DNA of the present invention

When the DNA of the present invention is integrated into the chromosome of a mammal, it is expected to stably show promoter activity higher than the respective promoter activity of loxP, promoter and hprt gene composing the DNA of the present invention. Further, a gene ligated downstream of the DNA of the present invention can be highly expressed in at least one or more non-neuronal tissues (for example, cells of one or more tissues or organs selected from the group consisting of epithelial tissue, connective tissue, fat tissue, cartilage tissue, bone tissue, muscle tissue, intraoral tissue, blood tissue, bone marrow, cardiovascular organ, lymphatic organ, teeth, digestive gland, liver, gallbladder, pancreas, respiratory organ, urinary organ, genital organ, endocrine organ, sense organ and nail). Such properties of the DNA of the present invention are suitable and useful for, for example, expressing a foreign reporter protein (for example, a foreign reporter protein for monitoring changes in intracellular calcium ion concentration, a foreign reporter protein for labeling cells, or any other protein) in the above tissues of animals for pharmacological evaluation.

A transgenic non-human mammal having DNA encoding a foreign reporter protein for monitoring changes in intracellular calcium ion concentration and located downstream of the DNA of the present invention can be constructed according to the method described in 1 above using the DNA of the present invention prepared by the method described in 3 above as a promoter. A specific example is a transgenic non-human mammal having DNA encoding apoaequorin or cameleon protein. Examples of applications of these transgenic non-human mammals include those described in 2 above.

A transgenic non-human mammal having DNA encoding a foreign reporter protein for labeling cells and located downstream of the DNA of the present invention can be produced according to the method described in 1 above using the DNA of the present invention prepared by the method described in 3 above as a promoter. A specific example is a transgenic non-human mammal having DNA encoding fluorescent protein, or photo- or chromogenic protein. Examples of fluorescent protein that can be used herein include green fluorescent proteins (GFP) derived from *Aequorea* (*Aequorea Victoria*), EGFP, EBFP, EYFP, GFPuv, which are commercially available from Clontech; red fluorescent proteins, DsRed derived from sea anemone (*Discosoma striata*); GFP Emerald, GFP Topaz, GFP Sapphire and GFP S65T which are available from Aurora Biosciences; fluorescent proteins described in Nature Biotech., 18, 313, (2000) or Nature Cell Biol., 2, 25, (2000), or the like. Examples of DNA encoding a photo- or a chromogenic protein that can be used herein include luciferase derived from firefly (*Photinus pyralis*) and commercially available from Promega; luciferase derived from Renilla (*Renilla reniformis*); chloramphenicol acetyltransferase; alkaline phosphatase (SEAP) which is derived from human placenta and commercially available from Clontech; β-galactosidase; and the like. An example of application of these transgenic non-human mammals is a method for detecting physiological changes which comprises labeling tissues, organs or cells of transgenic non-human mammals with a foreign reporter protein; transplanting them to another individual which does not immunologically reject the tissues, organs or cells; and then detecting physiological changes, such as behavior, differentiation, malignant transformation and the like in the transplanted tissues, organs or cells using the foreign reporter protein. Another example of application of the transgenic non-human mammal is a method for detecting physiological changes which comprises transplanting embryonic stem cells used to produce transgenic non-human mammals or cells induced to differentiate from the embryonic stem cells into another individual which does not immunologically reject the cells; and detecting physiological changes, such as behavior, differentiation, malignant transformation and the like in the transplanted cells using the foreign reporter protein introduced. Specific examples of such methods include those described in Proc. Natl. Acad. Sci. USA, 94, 14809, (1997), Cell Transplant., 5, 131, (1996), Exp. Neurol., 149, 28, (1998), Science, 285, 754, (1999), or the like.

By producing a transgenic non-human mammal having DNA encoding any protein and located downstream of the DNA of the present invention, the physiological functions of the protein can be efficiently examined in vivo.

Using cells and cell nuclei of a transgenic non-human mammal having DNA encoding a foreign reporter protein and located downstream of the DNA of the present invention, a clone of the transgenic non-human mammal and a gene-modified mammal having DNA encoding a foreign reporter protein and a further modified gene on the chromosome can be produced. Examples of cells used herein include embryonic stem cells, eggs or sperms. Production methods and applications of the above transgenic or cloned non-human mammals and gene-modified mammals are as described in detail in 2 (4).

### BEST MODE FOR CARRYING OUT THE INVENTION

The present invention will be described in more detail with reference to the following examples; however, the present invention is not limited to these examples.

### Example 1: Construction of apoaequorin expression plasmid

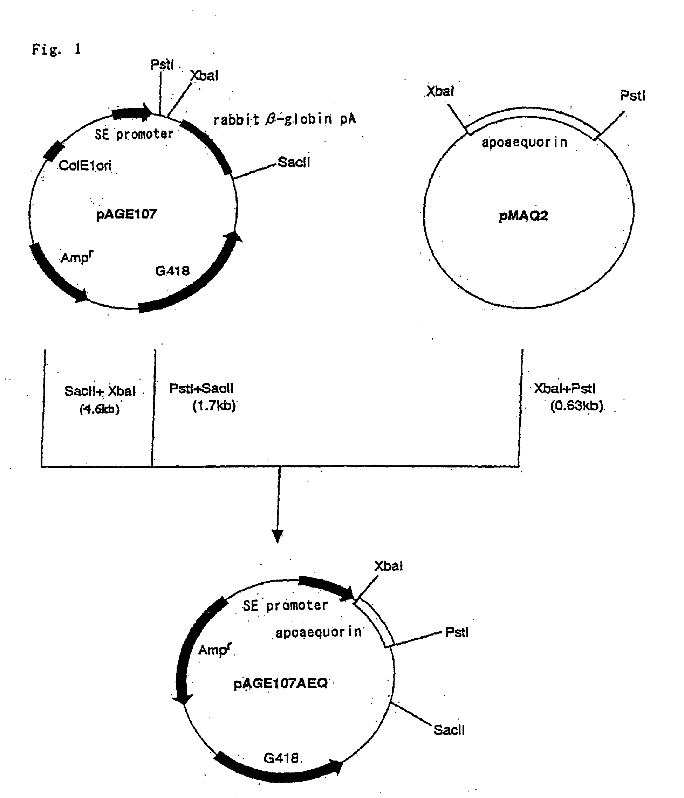

### 1) Construction of plasmid pAGE107AEQ having apoaequorin gene downstream of SV 40 early promoter (see Fig. 1)

2 µg of plasmid pAGE107 (Japanese Published Unexamined Patent Application No. 91/22979, Cytotechnology, 3, 133, 1990) was dissolved in 30 µl of Universal Buffer M (Takara Shuzo) and 10 units of *Xba*I and 10 units of *Sac*II were added thereto, followed by digestion at 37°C for 2 hours. The reaction solution was subjected to agarose gel electrophoresis to collect DNA fragment of about 4.6 kb containing an ampicillin (Ap) resistant gene using QIAEX II Gel Extraction Kit (QIAGEN) (DNA is hereinafter collected from agarose gel in accordance with this method).

2 µg of plasmid pAGE107 was dissolved in 30 µl of Universal Buffer H (Takara Shuzo) and 10 units of *Pst*I and 10 units of *Sac*II were added thereto, followed by digestion at 37°C for 2 hours. The reaction solution was subjected to agarose gel electrophoresis to collect DNA fragment of about 1.7 kb containing a polyadenylation signal of rabbit β-globin.

On the other hand, 2 µg of plasmid pMAQ2 (Molecular Probes) was dissolved in 30 µl of Universal Buffer H (Takara Shuzo) and 10 units of *Xba*I and 10 units of *Pst*I were added thereto, followed by digestion at 37°C for 2 hours. The reaction solution was subjected to agarose gel electrophoresis to collect DNA fragment of about 0.63 kb containing apoaequorin.

The above-obtained 0.1 µg of *Xba*I*-Sac*II fragment (4.6 kb) derived from plasmid pAGE107, 0.1 µg of *Pst*I-*Sac*II fragment (1.7 kb) derived from plasmid pAGE107, and 0.1 µg of *Xba*I*-Pst*I fragment (0.63 kb) derived from plasmid pMAQ2 were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using the reaction solution in accordance with the method by Cohen et al. (Proc. Natl. Acad. Sci. USA, 69, 2110, 1972) (*Escherichia coli* is hereinafter transformed in accordance with this method) to obtain an ampicillin resistant (Amp^{r}) strain. According to a conventional method (Nucleic Acids Res., 7, 1513, 1979), plasmid DNA was isolated from the transformant (this method is hereinafter employed in isolation of plasmid). The construction of the obtained plasmid was confirmed by digestion with a restriction enzyme. This plasmid is hereinafter referred to as pAGE107AEQ.

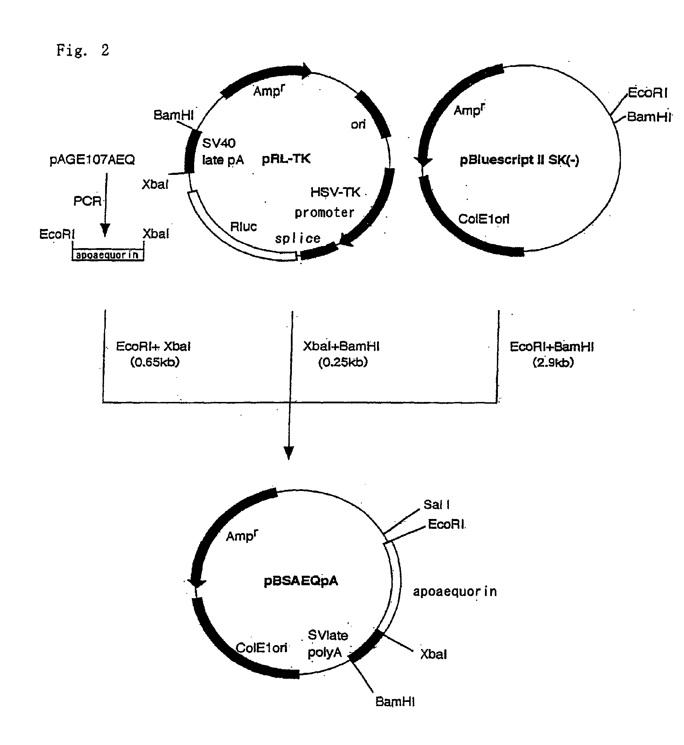

### 2) Construction of plasmid pBSAEQpA having apoaequorin gene (see Fig. 2)

27mer primer 1 having a nucleotide sequence shown by SEQ ID NO: 2 and 28mer primer 2 having a nucleotide sequence shown by SEQ ID NO: 3 were prepared based on the nucleotide sequence of apoaequorin gene cDNA described in the report by Inouye et al. (Proc. Natl. Acad. Sci. USA, 82, 3154, 1985; Japanese Published Unexamined Patent Application No. 86/135586). In accordance with a conventional method, a reaction solution was prepared using plasmid pAGE107-AEQ. PCR was carried out in the following conditions: the reaction solution was reacted at 95°C for 5 minutes, then a cycle of reaction at 95°C for 1 minute, at 55°C for 2 minutes, and at 72°C for 4 minutes was repeated 30 times, and at last, the reaction was carried out at 72°C for 10 minutes, and the solution was stored at 4°C overnight. This PCR reaction solution was extracted with phenol/chloroform, and an amplified DNA fragment was collected by ethanol precipitation. The collected DNA fragment was dissolved in 30 µl of Universal Buffer H (Takara Shuzo) and 10 units of *Xba*I and 10 units of *Eco*RI were added thereto, followed by digestion at 37°C for 2 hours. The reaction solution was subjected to agarose gel electrophoresis to collect DNA fragment of about 0.65 kb containing apoaequorin.

Subsequently, 2 µg of plasmid pRL-TK (Promega) was dissolved in 30 µl of Universal Buffer H (Takara Shuzo) and 10 units of *Xba*I and 10 units of *Bam*HI were added thereto, followed by digestion at 37°C for 2 hours. The reaction solution was subjected to agarose gel electrophoresis to collect DNA fragment of about 0.25 kb containing a polyadenylation signal of an SV 40 late gene.

2 µg of plasmid pBluescript II SK(-) (Stratagene) was dissolved in 30 µl of Universal Buffer H (Takara Shuzo) and 10 units of *Eco*RI and 10 units of *Bam*HI were added thereto, followed by digestion at 37°C for 2 hours. The reaction solution was subjected to agarose gel electrophoresis to collect DNA fragment of about 2.9 kb containing an ampicillin (Ap) resistant gene.

The above-obtained 0.1 µg of DNA fragment (0.65 kb) containing apoaequorin, 0.1 µg of *Xba*I*-Bam*HI fragment (0.25 kb) derived from plasmid pRL-TK, and 0.1 µg of *Eco*RI-*Bam*HI fragment (2.9 kb) derived from plasmid pBluescript II SK(-) were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using the reaction solution in accordance with the method by Cohen et al. to obtain an ampicillin resistant (Amp^{r}) strain. According to a conventional method, plasmid DNA was isolated from the transformant. The construction of the obtained plasmid was confirmed by digestion with a restriction enzyme. A nucleotide sequence was determined in accordance with a conventional method using DNA Sequencer 377 (Perkin Elmer), and it was confirmed that a known apoaequorin cDNA sequence was contained in the plasmid. This plasmid is hereinafter referred to as pBSAEQpA.

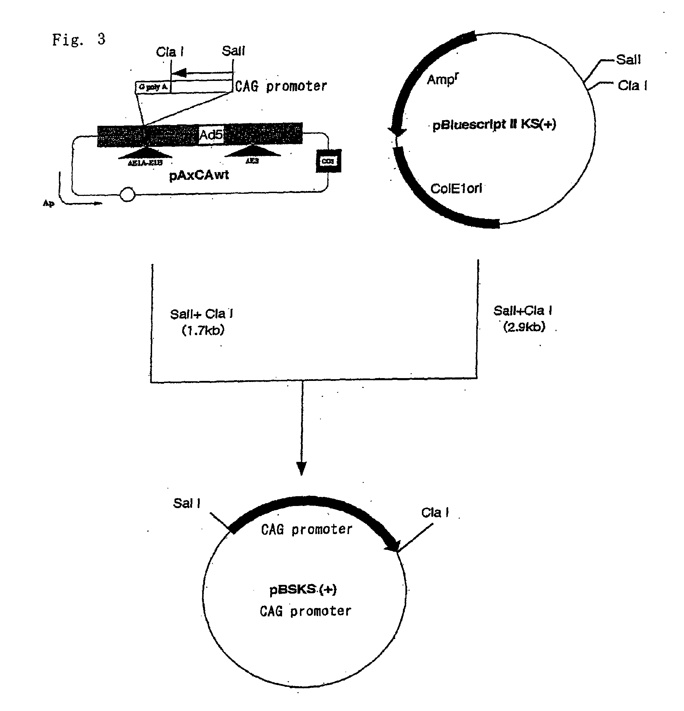

### 3) Construction of plasmid pBSKS(+)CAG promoter having CAG promoter (see Fig. 3)

2 µg of cosmid pAxCAwt (Nucleic Acids Research, 23, 3817, 1995) was dissolved in 30 µl of Universal Buffer H (Takara Shuzo) and 10 units of *Sal*I and 10 units of *Cla*I were added thereto, followed by digestion at 37°C for 2 hours. The reaction solution was subjected to agarose gel electrophoresis to collect DNA fragment of about 1.7 kb containing a CAG promoter using QIAEX II Gel Extraction Kit (QIAGEN).

Subsequently, 2 µg of plasmid pBluescript II KS (+) (Stratagene) was dissolved in 30 µl of Universal Buffer H (Takara Shuzo) and 10 units of *Sal*I and 10 units of *Cla*I were added thereto, followed by digestion at 37°C for 2 hours. The reaction solution was subjected to agarose gel electrophoresis to collect DNA fragment of about 2.9 kb containing an ampicillin (Ap) resistant gene using QIAEX II Gel Extraction Kit (QIAGEN).

The above-obtained 0.1 µg of *Sal*I*-Cla*I fragment (1.7 kb) derived from cosmid pAxCAwt and 0.1 µg of *Sal*I*-Cla*I fragment (2.9 kb) derived from plasmid pBluescript II KS (+) were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using a reaction solution in accordance with the method by Cohen et al. to obtain an ampicillin resistant (Amp^{r}) strain. According to a conventional method, plasmid DNA was isolated from the transformant. The construction of the obtained plasmid was confirmed by digestion with a restriction enzyme. This plasmid is hereinafter referred to as pBSKS(+)CAG promoter.

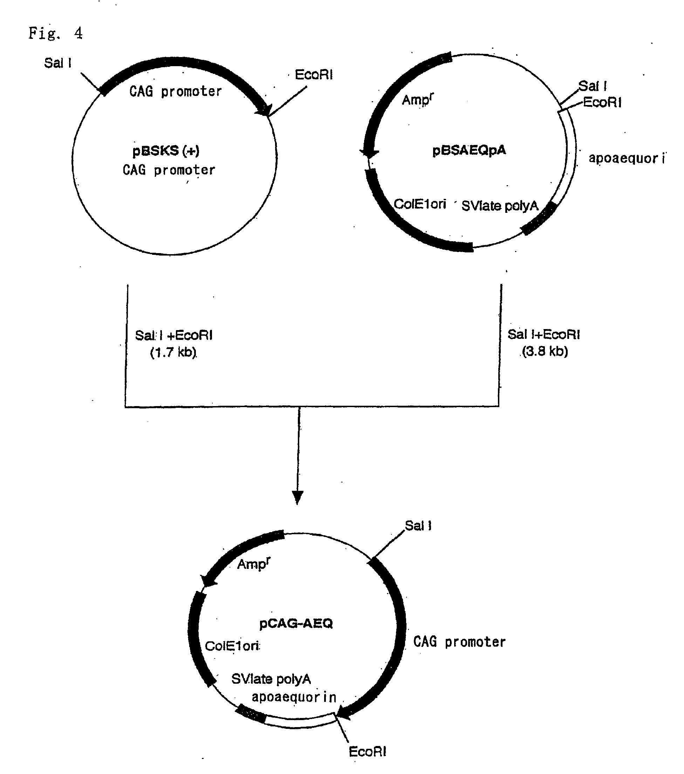

### 4) Construction of plasmid pCAG-AEQ having apoaequorin gene downstream of CAG promoter (see Fig. 4)

2 µg of plasmid pBSKS(+)CAG promoter was dissolved in 30 µl of Universal Buffer H (Takara Shuzo) and 10 units of *Sal*I and 10 units of *Eco*RI were added thereto, followed by digestion at 37°C for 2 hours. The reaction solution was subjected to agarose gel electrophoresis to collect DNA fragment of about 1.7 kb containing a CAG promoter using QIAEX II Gel Extraction Kit (QIAGEN).

Subsequently, 2 µg of plasmid pBSAEQpA was dissolved in 30 µl of Universal Buffer H (Takara Shuzo) and 10 units of *Sal*I and 10 units *of Eco*RI were added thereto, followed by digestion at 37°C for 2 hours. The reaction solution was subjected to agarose gel electrophoresis to collect DNA fragment of about 3.8 kb containing an apoaequorin gene using QIAEX II Gel Extraction Kit (QIAGEN).

The above-obtained 0.1 µg of *Sal*I*-Eco*RI fragment (1.7 kb) derived from plasmid pBSKS(+)CAG promoter and 0.1 µg of *Sal*I-*Eco*RI fragment (3.8 kb) derived from plasmid pBSAEQpA were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using the reaction solution in accordance with the method by Cohen et al. to obtain an ampicillin resistant (Amp^{r}) strain. According to a conventional method, plasmid DNA was isolated from the transformant. The construction of the obtained plasmid was confirmed by digestion with a restriction enzyme. This plasmid is hereinafter referred to as pCAG-AEQ.

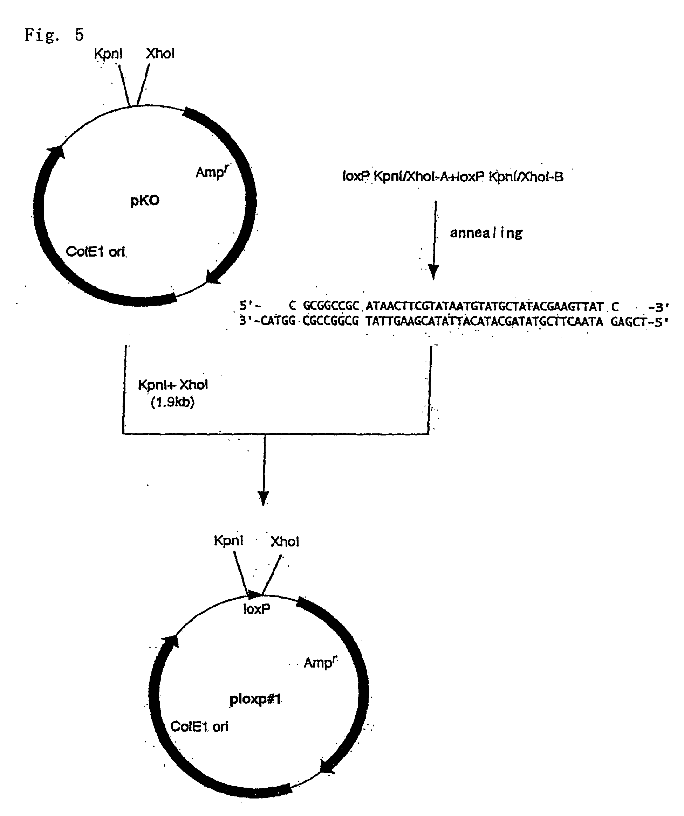

### 5) Construction of plasmid ploxp#1 (see Fig. 5)

2 µg of plasmid pKO (Lexicon Genetics Inc.) was dissolved in 30 µl of Universal Buffer M (Takara Shuzo) and 10 units of *Kpn*I and 10 units of *Xho*I were added thereto, followed by digestion at 37°C for 2 hours. The reaction solution was subjected to agarose gel electrophoresis to collect DNA fragment of about 1.97 kb containing an ampicillin (Ap) resistant gene.

Subsequently, 44mer single stranded synthetic DNA loxPKpnI/XhoI-A having a nucleotide sequence shown by SEQ ID NO: 4 containing a loxP sequence described in the report by Araki et al. (Nucleic Acids Res., 25, 868, 1997) and 52mer single stranded synthetic DNA loxPKpnI/XhoI-B having a nucleotide sequence shown by SEQ ID NO: 5 were prepared and annealed to provide double stranded DNA.

0.1 µg of the double stranded DNA and 1.0 µg of *Kpn*I-*Xho*I fragment (1.97 kb) derived from plasmid pKO that had been obtained above were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using the reaction solution in accordance with the method by Cohen et al. to obtain an ampicillin resistant (Amp^{r}) strain. According to a conventional method, plasmid DNA was isolated from the transformant. The construction of the obtained plasmid was confirmed by digestion with a restriction enzyme. This plasmid is hereinafter referred to as ploxp#1.

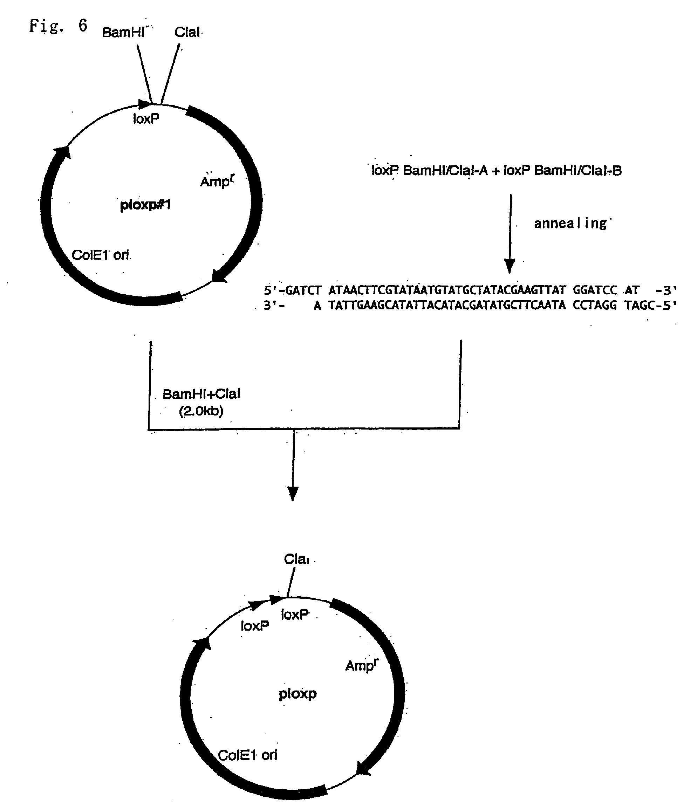

### 6) Construction of plasmid ploxp (see Fig. 6)

2 µg of plasmid ploxp#1 was dissolved in 30 µl of Universal Buffer H (Takara Shuzo) and 10 units of *Bam*HI and 10 units of *Cla*I were added thereto, followed by digestion at 37°C for 2 hours. The reaction solution was subjected to agarose gel electrophoresis to collect DNA fragment of about 2.02 kb containing an ampicillin (Ap) resistant gene.

Subsequently, 47mer single stranded synthetic DNA loxPBamHI/ClaI-A having a nucleotide sequence shown by SEQ ID NO: 6 containing a loxP sequence and 45mer single stranded synthetic DNA loxPBamHI/ClaI-B having a nucleotide sequence shown by SEQ ID NO: 7 were prepared and annealed to provide double stranded DNA.

0.1 µg of the double stranded DNA and 1.0 µg of *Bam*HI*-Cla*I fragment (2.02 kb) derived from plasmid ploxP#1 that had been obtained above were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using the reaction solution in accordance with the method by Cohen et al. to obtain an ampicillin resistant (Amp^{r}) strain. According to a conventional method, plasmid DNA was isolated from the transformant. The construction of the obtained plasmid was confirmed by digestion with a restriction enzyme. This plasmid is hereinafter referred to as ploxp.

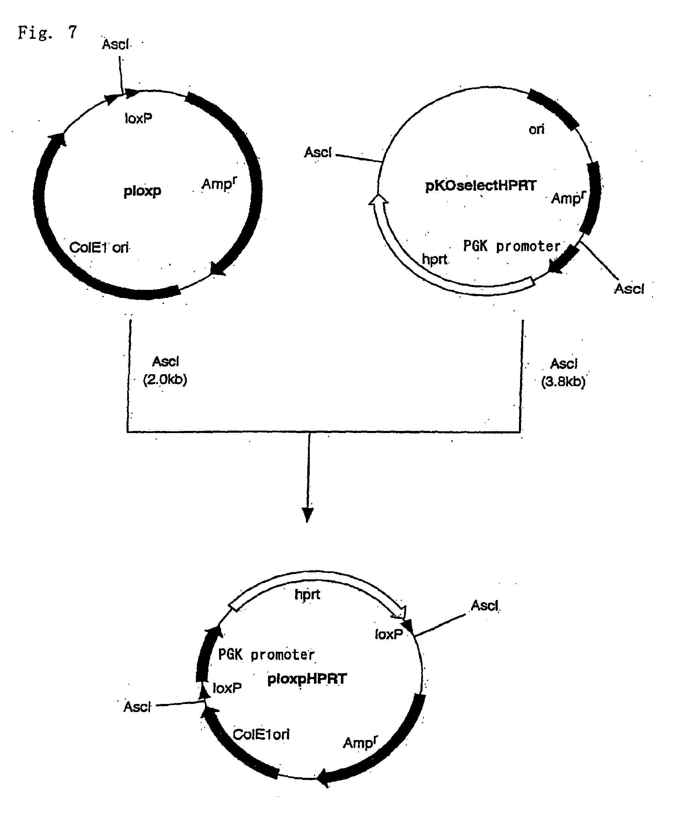

### 7) Construction of plasmid ploxpHPRT (see Fig. 7)

2 µg of plasmid ploxp was dissolved in 30 µl of NEB Buffer 4 (New England Biolabs) and 10 units *of Asc*I were added thereto, followed by digestion at 37°C for 2 hours. The reaction solution was subjected to agarose gel electrophoresis to collect DNA fragment of about 2.07 kb containing an ampicillin (Ap) resistant gene. The collected DNA fragment was dissolved in 30 µl of Alkaline Phosphatase Buffer (Takara Shuzo) and 44 units of alkaline phosphatase derived from bovine intestine (Takara Shuzo) were added so as to conduct reaction at 37°C for 30 minutes. After extraction with phenol/chloroform, dephosphorylated DNA fragment of about 2.07 kb was collected by ethanol precipitation.

Subsequently, 2 µg of plasmid pKOselectHPRT (Lexicon Genetics Inc.) was dissolved in 30 µl of NEB Buffer 4 (New England Biolabs) and 10 units of *Asc*I were added thereto, followed by digestion at 37°C for 2 hours. The reaction solution was subjected to agarose gel electrophoresis to collect DNA fragment of about 3.8 kb containing a hypoxanthine phosphoribosyltransferase (hprt) gene.

The above-obtained 0.1 µg *of Asc*I fragment (2.07 kb) derived from plasmid ploxp and 0.1 µg of *Asc*I fragment (3.8 kb) derived from plasmid pKOselectHPRT were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using the reaction solution in accordance with the method by Cohen et al. to obtain an ampicillin resistant (Amp^{r}) strain. According to a conventional method, plasmid DNA was isolated from the transformant. The construction of the obtained plasmid was confirmed by digestion with a restriction enzyme. This plasmid is hereinafter referred to as ploxpHPKT.

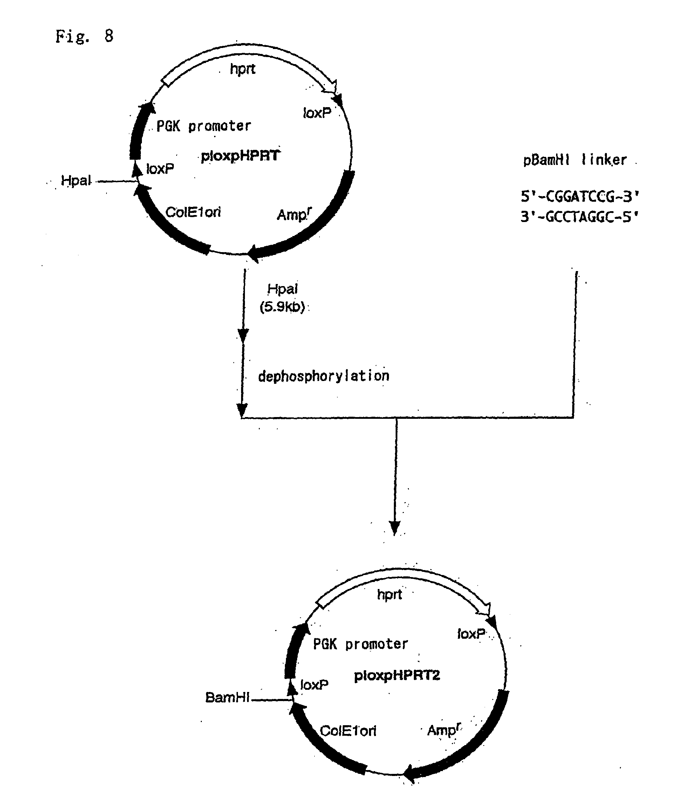

### 8) Construction of plasmid ploxpHPRT2 (see Fig. 8)

2 µg of plasmid ploxpHPKT was dissolved in 30 µl of Universal Buffer K (Takara Shuzo) and 10 units of *Hpa*I were added thereto, followed by digestion at 37°C for 2 hours. The reaction solution was subjected to agarose gel electrophoresis to collect DNA fragment of about 2.07 kb containing an ampicillin (Ap) resistant gene. The collected DNA fragment was dissolved in 30 µl of Alkaline Phosphatase Buffer (Takara Shuzo) and 44 units of alkaline phosphatase derived from bovine intestine (Takara Shuzo) were added to conduct reaction at 37°C for 30 minutes. After extraction with phenol/chloroform, dephosphorylated DNA fragment of about 5.9 kb was collected by ethanol precipitation.

The above-obtained 0.1 µg of *Hpa*I fragment (5.9 kb) derived from plasmid ploxpHPRT and 0.1 µg of 8mer synthetic linker pBamHI (8mer) (5'-pd(CGGATCCG)-3', Takara Shuzo) were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using the reaction solution in accordance with the method by Cohen et al. to obtain an ampicillin resistant (Amp^{r}) strain. According to a conventional method, plasmid DNA was isolated from the transformant. The construction of the obtained plasmid was confirmed by digestion with a restriction enzyme. This plasmid is hereinafter referred to as ploxpHPRT2.

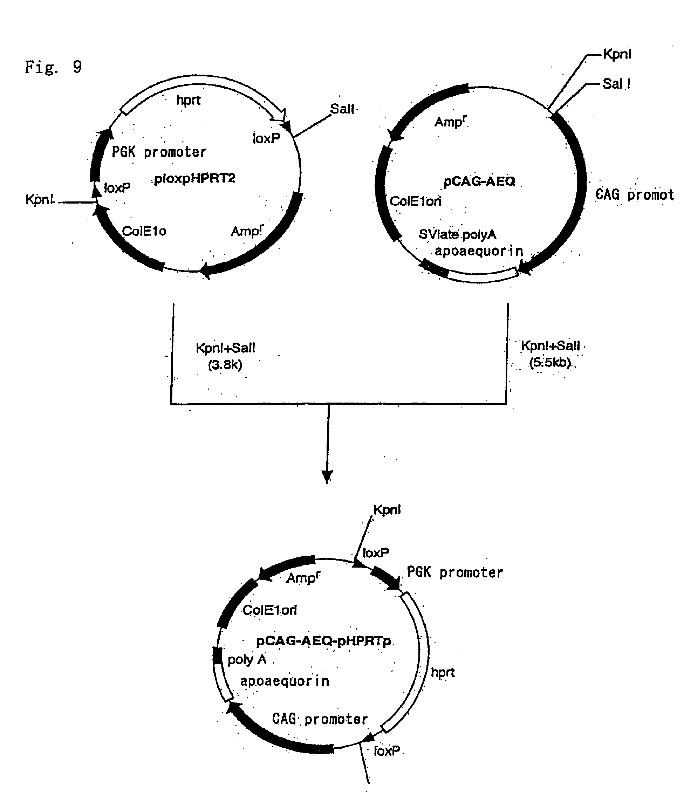

### 9) Construction of plasmid pCAG-AEQ-pHPRTp having hprt gene downstream of PGK promoter and having apoaequorin gene ligated to CAG promoter further downstream (see Fig. 9)

2 µg of plasmid ploxpHPRT2 was dissolved in 30 µl of Universal Buffer L (Takara Shuzo) and 10 units of *Kpn*I were added thereto, followed by digestion at 37°C for 2 hours. Thereafter, 4 µl of 10 x Universal Buffer H (Takara Shuzo) and 10 units of *Sal*I were added so as to bring the total amount to 40 µl, followed by digestion at 37°C for an additional 2 hours. The reaction solution was subjected to agarose gel electrophoresis to collect DNA fragment of about 3.8 kb containing an hprt gene.

Subsequently, 2 µg of plasmid pCAG-AEQ was dissolved in 30 µl of Universal Buffer L (Takara Shuzo) and 10 units of *Kpn*I were added thereto, followed by digestion at 37°C for 2 hours. Thereafter, 4 µl of 10 x Universal Buffer H (Takara Shuzo) and 10 units of *Sal*I were added so as to bring the total amount to 40 µl, followed by digestion at 37°C for additional 2 hours. The reaction solution was subjected to agarose gel electrophoresis to collect DNA fragment of about 5.5 kb containing an ampicillin (Ap) resistant gene.

0.1 µg of *Kpn*I*-Sal*II fragment (3.8 kb) derived from plasmid ploxpHPRT2 and 0.1 µg of *Kpn*I-*Sal*II fragment (5.5 kb) derived from plasmid pCAG-AEQ, which had been obtained above, were dissolved in 30 µl of T4 ligase buffer. 100 units of T4 DNA ligase were added thereto to perform ligation reaction at 12°C for 16 hours.

*Escherichia coli* DH5 (Toyobo Co.) was transformed using the reaction solution in accordance with the method by Cohen et al. to obtain an ampicillin resistant (Amp^{r}) strain. According to a conventional method, plasmid DNA was isolated from the transformant. The construction of the obtained plasmid was confirmed by digestion with a restriction enzyme. This plasmid is hereinafter referred to as pCAG-AEQ-pHPRTp.

A *Kpn*I*-Eco*RI fragment (5.8 kb) containing a CAG promoter of plasmid pCAG-AEQ-pHPRTp was shown in SEQ ID NO: 1.

### Example 2: Introduction of apoaequorin expression plasmid into mammalian cells and examination of the transient expression level of apoaequorin

Plasmid pBSAEQpA, pAGE107AEQ, or pCAG-AEQ containing DNA coding for apoaequorin constructed in Example 1 was introduced into a Chinese hamster ovarian cell that is deficient in a dihydrofolate reductase (dhfr) gene (CHOdhfr(-)DG44) (Biochemistry, 36, 10901, 1997; Cell, 33, 405, 1983), and an apoaequorin gene was transiently expressed to compare the levels of expression.

Apoaequorin expression plasmid was introduced into CHOdhfr(-) cell DG44 by a lipofection method. Specifically, the cells were inoculated onto a 6-well microplate for tissue culturing (IWAKI GLASS CO., LTD., code 3810-006) using 2ml of IMDM (GIBCO BRL) containing 10% fetal bovine serum at a density of 3 x 10⁵ cells/well. The cells were cultured for 24 hours at 37°C in 5% CO₂ and then washed several times with OPTI-MEM (GIBCO BRL). 100 µl of OPTI-MEM containing 2 µg of plasmid and 100 µl of OPTI-MEM containing 6 µl of Lipofectamine reagent (GIBCO BRL) were moderately mixed and reacted at room temperature for 30 minutes. The total amount was then brought to 1 ml with OPTI-MEM and added to the washed cells, followed by culturing for 4 hours. Four hours later, 1 ml of IMDM containing 20% fetal bovine serum was added and cultured for additional 24 hours, thereby a gene was introduced.

Subsequently, the cells into which the gene had been introduced were washed once with IMDM, and 1 ml of IMDM containing 10 µmol/l coelenterazine (Molecular Probes) was added, followed by culturing for 4 hours. Four hours later, cells were detached using 0.05% trypsin solution, suspended in a modified Krebs-Ringer buffer [20 mmol/l HEPES solution (pH 7.4) containing 125 mmol/l NaCl, 5 mmol/l KCl, 1 mmol/l MgSO₄, 1 mmol/l CaCl₂, 1 mmol/l Na₂HPO₄, and 5.5 mmol/l glucose] at a density of 1 x 10⁶ cells/ml, and inoculated onto a 96-well white plate for cell culturing (Sumitomo Bakelite, product No. MS-8096W) at 100 µl/well. Using Microplate Luminometer LB96P (EG & G BERTHOLD), 100 µl of modified Krebs-Ringer buffer containing 2 µmol/l calcium ionophore A23187 (Research Biochemical International) was added to each well in which cells had been inoculated and luminescence was measured every three seconds immediately after the addition.

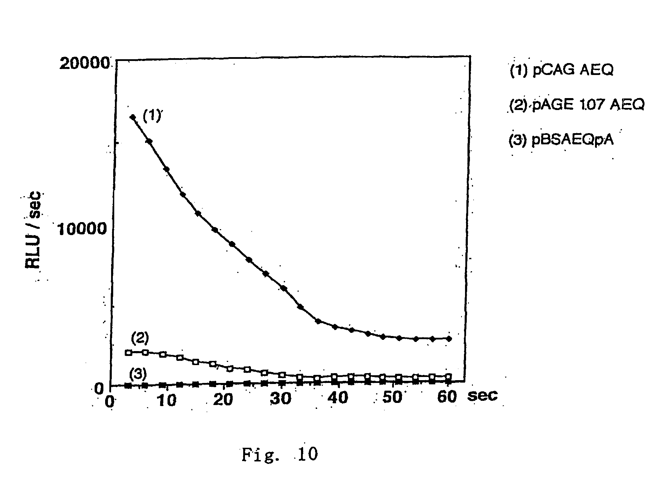

In accordance with the above method, luminescence of aequorin stimulated by A23187 of the cell in which plasmid pAGE107-AEQ, plasmid pCAG-AEQ, or plasmid pBSAEQpA was introduced into CHOdhfr(-)cell DG44 was used as an index to compare the promoter activity among each plasmid. The result is shown in Fig. 10. It was shown that a CAG promoter had stronger transcription activity than an SV40 early gene promoter that has been commonly used in expression in mammalian cells.

### Example 3: Preparation of stable transformant by introduction of apoaequorin expression plasmid into mammalian cells and examination of level of apoaequorin expression

A stable transformant wherein plasmid pCAG-AEQ-pHPRTp had been introduced into mouse myeloma cell P3. X63/Ag8.U1 (hereinafter abbreviated to P3U1) (Curr. Top. Microbiol. Immunol., 81, 1, 1978) was obtained. Further, Cre recombinase was transiently expressed in the cell to provide a mutant from which an hprt gene expression unit had been removed. Then, the level of apoaequorin expression was compared between before and after removal of an hprt expression unit.

Plasmid pCAG-AEQ-pHPRTp was introduced into P3U1 cell by electroporation. Specifically, the cells were suspended in a K-PBS buffer (137 mmol/l KCl, 2.7 mmol/l NaCl, 8.1 mmol/l Na₂HPO₄, 1.5 mmol/l KH₂PO₄, and 4 mmol/l MgCl₂) at a density of 8 x 10⁶ cells/ml, and 200 µl of this cell suspension and 4 µl of plasmid pCAG-AEQ-pHPRTp, which had been adjusted to 1 µg/µl, were mixed in a fusion chamber Gene Pulser Cuvette (gap distance of 2 mm) (BIORAD; catalog no. 165-2086). A cell fusing machine, Gene Pulser (BIORAD), was used to introduce genes at a pulse voltage of 0.25 kv with a capacitance of 125 µF, and the cells were suspended in 10 ml of RPMI 1640 medium (GIBCO BRL) containing 10% fetal bovine serum, followed by culturing at 37°C in 5% CO₂ for 24 hours. After culturing, 200 µl of HAT-Media Supplement (50 x) (Boehringer Mannheim Biochemica) was added and culturing was continued for an additional 6 days to obtain an aminopterin-resistant cell. The number of viable cells of the obtained drug-resistant cells was measured, and RPMI 1640 medium containing 2% HT-Media Supplement (50 x) (Boehringer Mannheim Biochemica) and 10% fetal bovine serum was used to dilute into 5 cells/ml, and inoculated onto a 96-well plate at 100 µl/well. One week later, cells in a well in which a single colony was formed were collected. Southern hybridization described in 3) of Example 4 was employed to measure the number of copies of introduced gene in accordance with a method described in "Hasseikogaku jikken manuaru, toransujenikkumausu no tsukurikata (Development Engineering Experiment Manual; How to produce a transgenic mouse (Kodansha Ltd., 1987))", and a cell into which one copy of introduced gene had been inserted was selected. This cell is hereinafter referred to as clone 21.

Subsequently, clone 21 cells, suspended in a PBS buffer at 1.1 x 10⁷ cell/ml, and 25 µl of plasmid pBS185 (GIBCO BRL) containing Cre recombinase genes, which had been adjusted to 1 µg/µl, were mixed in a fusion chamber Gene Pulser Cuvette (gap distance of 4 mm) (BIORAD; catalog no. 165-2088). A cell fusing machine Gene Pulser (BIORAD) was used to introduce genes at a pulse voltage of 0.23 kv with a capacitance of 500 µF, and the cells were suspended in 10 ml of RPMI 1640 medium (GIBCO BRL) containing 10% fetal bovine serum, followed by culturing at 37°C in 5% CO₂ for 24 hours. After 24 hours of culturing, 6-thioguanine (2-amino-6-mercaptopurine; Sigma) was added in an amount of 10 µmol/l, and culturing was continued for 6 days to obtain a 6-thioguanine-resistant cell. This drug-resistant cell is hereinafter referred to as clone 21 Δhprt.

The obtained cells were suspended in RPMI 1640 medium containing 10 µmol/l coelenterazine at 1 x 10⁶ cell/ml, followed by culturing for 4 hours. After culturing, the cells were collected by centrifugadon at 1,000 x g for 5 minutes, washed twice using a modified Krebs-Ringer buffer, suspended using a modified Krebs-Ringer buffer at 1 x 10⁶ cell/ml, and inoculated onto a 96-well white plate for cell culturing (Sumitomo Bakelite, Product No. MS-8096W) at 100 µl/well. Using Microplate Luminometer LB96P (EG & G BERTHOLD), 100 µl of modified Krebs-Ringer buffer containing 2 µmol/l calcium ionophore A23187 (Research Biochemical International) was added to each well in which cells had been inoculated and the luminescence was measured every three seconds immediately after the addition.

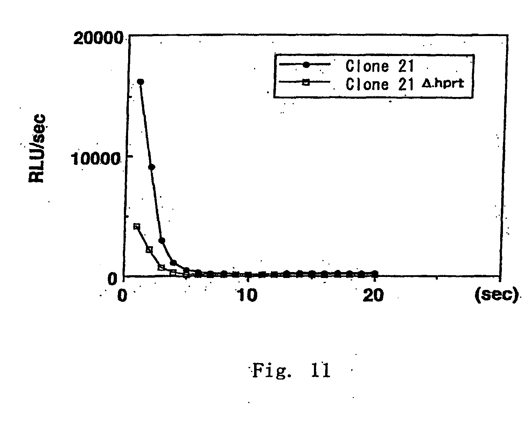

In accordance with the above method, the level of apoaequorin expression by clone 21 which is a plasmid pCAG-AEQ-pHPRTp transformant cell was compared with that by mutant clone 21 Δhprt in which an hprt gene expression unit is removed from clone 21. The result is shown in Fig. 11. It was shown that Clone 21 cell containing an hprt gene expression unit and having an hprt gene downstream of PGK promoter, expressed an apoaequorin gene at a higher level than clone 21 Δhprt containing no hprt gene expression unit.

### Example 4: Production of transgenic mouse having hprt gene downstream of PGK promoter and apoaequorin gene linked to CAG promoter further downstream

### 1) Injection of DNA into fertilized egg and implantation of the fertilized egg

An 8-week-old BDF1 mouse was purchased for ovulation and bred for 1 week under a condition of a light period of 12 hours, i.e., from 8:00 to 20:00.

Follicle stimulating hormone (pregnant mare serum gonadotrophin, hereinafter abbreviated to PMSG) was peritoneally injected (5 IU/mouse) at 17:00 one day after breeding.

After peritoneal injection of luteinizing hormone (human chorionic gonadotropin, hereinafter abbreviated to HCG, 5 IU/mouse) at 17:00 on the third day, the mouse was subjected to cohabitation in pair with an 8-week or older male BDF1 mouse for the purpose of mating.

A copulatory plug of bred female rat was examined at 9:00 on the 4th day and the rat, the copulatory plug of which had been examined, was sacrificed at 13:30, and then ovum collection was initiated. DNA fragment of about 6.6 kb containing an hprt gene ligated to PGK promoter and an apoaequorin gene ligated to CAG promoter (hereinafter abbreviated to "a transgene") was prepared by cleaving plasmid pCAG-AEQ-pHPRTp, obtained in 9) of Example 1, with restriction enzymes *Pvu*I and *Sac*I, performing agarose gel electrophoresis, collecting the fragment in 1 mmol/l Tris (pH 8.0) solution containing 0.1 mmol/l EDTA with QIAEX II Gel Extraction Kit (QIAGEN), and adjusting the concentration into 10 µg to 100 µg/ml using a PBS solution. Pronucleus-formative eggs were selected from a fertilized eggs, and the prepared 1 to 2 µl of DNA solution of the transgene was poured into the fertilized male procucleus of a BDF1 mouse egg at the single cell stage from 14:30, while observing under a microscope. Subsequently, the egg cell was cultured in a conventional modified Whitten's medium.

Two-cell stage embryo was confirmed at 13:30 on the 5th day and then transplanted to a oviduct of the pseudopregnant female MCH mouse to implant in accordance with the method described in "Manipulating Mouse Embryo (2nd. Ed.)". The pseudopregnant female MCH mouse (10-week or older) was provided by subjecting a female MCH mouse in preestrus to estrus to cohabitation with a vasoligated male MCH mouse (10-week or older) in pair and mating at 17:00 on the 4th day, and examination of copulatory plug of the female mouse at 9:00 on the 5th day.

### 2) Detection of transgene from offspring

Genome DNA, which was collected from a tail of 2-week old offspring, was used to select a mouse having an apoaequorin gene by PCR. Specifically, 27mer primer 1 having a nucleotide sequence shown by SEQ ID NO: 2 containing a nucleotide sequence of apoaequorin gene cDNA and 28mer primer 2 having a nucleotide sequence shown by SEQ ID NO: 3 were used to carry out PCR. Genome DNA used in the analysis was extracted from an about 0.5 cm fragment of the tail in accordance with a method described in "Manipulating Mouse Embryo (2nd Ed.)". The obtained nucleic acid pellet was washed once in 70% ethanol and dried, resuspended in 300 µl of 10 mmol/l Tris (pH 8.0) containing 200 µg/ml RNaseA and 1 mmol/l EDTA to obtain a tail genome DNA preparation. PCR was carried in the following conditions: 1 µl of the tail genome DNA preparation was diluted to fifty-fold with sterilized water, and reacted at 94°C for 5 minutes with primer 1 and primer 2. Subsequently, a cycle of reaction at 94°C for 1 minute, at 64°C for 2 minutes, and at 72°C for 3 minutes was repeated 30 times, and then finally reacted at 72°C for 10 minutes, and the solution was stored at 4°C overnight. The reaction product was subjected to electrophoresis through 1.0% agarose GTC (FMC Bioproduct) gel to select a mouse exhibiting a DNA band of about 600 bp. Among a total of 21 offsprings, a DNA band having about 600 bp was found in 5 offsprings. This confirmed that five mice of individual Nos. 98075-02-2 (female), 98075-04-5 (male), 98075-04-6 (male), 98075-04-7 (female), and 98075-04-9 (female) contained a transgene at least in their tail cell.

### 3) Identification of germ line transgenic mouse

Five individuals in which the presence of a transgene was found in tail genome DNA were bred to 8-week-old and mated with an 8-week or older C57BL/6J mouse in pair to obtain the offspring. A mouse having an apoaequorin gene was selected by PCR using genome DNA collected from the obtained offspring's tail.

Genome DNA was prepared in accordance with the method described in 2) of Example 4. PCR was carried out using 27mer primer 1 and 28mer primer 2 containing a nucleotide sequence of apoaequorin gene cDNA. PCR was carried out in the following conditions: 1 µl of the tail genome DNA preparation was diluted to fifty-fold with sterilized water and reacted at 94°C for 5 minutes with primer 1 and primer 2. Subsequently, a cycle of reaction at 94°C for 1 minute, at 64°C for 2 minutes, and at 72°C for 3 minutes was repeated 30 times, and then reacted at 72°C for 10 minutes at last, and the solution was stored at 4°C overnight. The reaction product was subjected to electrophoresis through 1.0% agarose GTC (FMC Bioproduct) gel to select a mouse exhibiting a DNA band of about 600 bp.

Nine out of a total of 17 offsprings derived from individual No. 98075-02-2, nine out of a total of 15 offsprings derived from individual No. 98075-04-5, and 13 out of a total of 18 offsprings derived from individual No. 98075-04-6 exhibited a DNA band having about 600 bp. No band was observed in the offspring derived from individual Nos. 98075-04-7 and 98075-04-9.

Subsequently, each of these three PCR positive offsprings was subjected to Southern hybridization using a tail genome DNA preparation and a radioisotope-labeled DNA probe containing an apoaequorin gene sequence. The fragment obtained by cleaving DNA prepared from a tail of each individual with *Eco*RI and the *Eco*RI*-Xba*I fragment (0.6 kb) containing apoaequorin gene derived from plasmid pBSAEQpA, were labeled with ³²P-dCTP (Deoxycytidine 5'-triphosphate (dCTP), [α-³²P]; NEN), and used as a probe to perform Southern hybridization. A commercially available T7 QuickPrime Kit (Pharmacia Biotech) was used for labeling. 10 µg of each DNA of three PCR positive individuals was completely cleaved with a restriction enzyme *Eco*RI, subjected to electrophoresis through 0.8% agarose gel, and transferred to a nylon filter (Hybond™-N+; Amarsham) in accordance with a method described by Southern (J. Mol. Biol., 98, 503, 1975). This filter was hybridized with a labeled probe overnight and was washed twice for 5 minutes and once for 10 minutes at 65°C with a solution containing 2 x SSC, 0.1% SDS, and was superimposed on a X-ray film (Kodak Scientific Imaging Film X-OMAT™ AR; Kodak), followed by exposure to radiation at - 80°C overnight and development on a subsequent day.

As a result of the Southern hybridization, in all the three examined mice a signal was detected at the position of 4.8 kbp. This indicates that these three individuals had a transgene injected therein. The offspring of three PCR positive individuals was subjected to Southern hybridization, and as a result, a total of three individuals, i.e., an offspring of each of the individuals had a transgene introduced therein. These results confirmed that germ line transgenic mice were three mice of individual Nos. 98075-02-2 (female), 98075-04-5 (male), and 98075-04-6 (male).

### Example 5: Analysis on transgene expression of prepared transgenic mouse

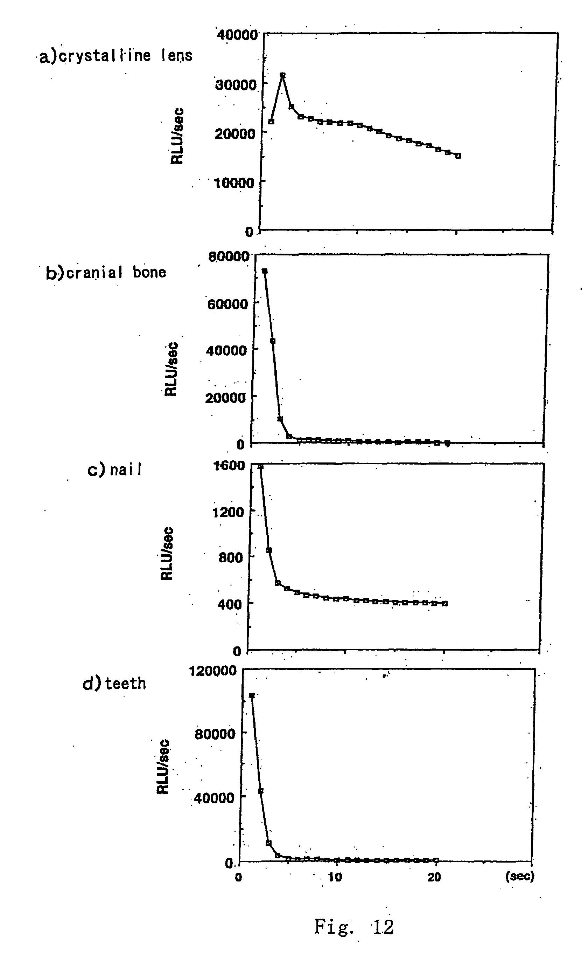

Tissues and organs were extracted from each offspring of the three individuals in which the presence of a transgene was confirmed in germ line, and the apoaequorin expression was tested. After the offspring was sacrificed, thymus, testis, seminal vesicle, spleen, abdominal muscle, diaphragm, kidney, crystalline lens, lung, pancreas, adipose tissue, skin, liver, cranial bone, esophagus, blood vessel, heart, hair, tail, ear, blood, bone marrow, teeth, tongue, stomach, small intestine, colon, urinary bladder, penis, ovary, utenus, oviduct, nail, thyroid gland, and adrenal gland were excised. These excised organs (excluding blood, bone marrow, and hair) were shredded to a cube with a dimension of about 1 to 2 mm in a PBS solution containing 10 units/ml heparin sodium and thoroughly washed by exchanging solution. Blood was collected from the eyeground in an amount of about 0.5 ml and suspended in 1.0 ml of PBS solution containing 10 units/ml heparin sodium, and then collected by centrifugation at 1,000 x g for 5 minutes. Bone marrow was extracted from femur in accordance with a method by Dexter et al. (J. Cell. Physiol., 91, 335, 1977), suspended in 5.0 ml of PBS solution containing 10 units/ml heparin sodium, and then collected by centrifugation at 1,000 x g for 5 minutes. Hair was pulled out of body and cut at the portion about 5 mm away from the hair root, and was then collected. Subsequently, in each well of a 96-well white plate for cell culturing (Sumitomo Bakelite, Product No. MS-8096W) comprising 50 µl of RPMI 1640 medium containing 10 µmol/l coelenterazine (Molecular Probes) per well, several samples of various organs prepared were added (all the collected cells regarding blood and bone marrow, and about 100 collected hairs containing hair root regarding hair) and cultured at 37°C for 5 hours. After culturing, 50 µl of PBS solution containing 0.07% Triton X-100 was added using a microplate luminometer LB96P (EG & G BERTHOLD), and luminescence for 20 seconds immediately after the addition was assayed. As a result, luminescence was observed in all the samples prepared from male and female offsprings derived from individual No. 98075-04-6, i.e., thymus, testis, seminal vesicle, spleen, abdominal muscle, diaphragm, kidney, crystalline lens, lung, pancreas, adipose tissue, skin, liver, cranial bone, esophagus, blood vessel, heart, hair root, tail, ear, blood, bone marrow, teeth, tongue, stomach, small intestine, colon, urinary bladder, penis, ovary, uterus, oviduct, nail, thyroid gland, and adrenal grand. As representatives, the assay results of crystalline lens, cranial bone, nail, and teeth are shown in Fig. 12.

In the offspring containing a transgene of individual No. 98075-04-6, neither morphological abnormality nor physiological abnormality was observed. Further, mating with C57BL/6J realized production of offsprings having a transgene in accordance with Mendel's law. This demonstrates that there is no adverse effect such as toxicity caused by the introduction of foreign apoaequorin.

### Example 6: Assay of physiologically active substance using prepared transgenic mouse

Tissues or organs were prepared from male and female offsprings derived from individual No. 98075-04-6 and a responsiveness toward physiologically active substances was examined. After the offspring was sacrificed, thymus, spleen, blood vessel, heart, uterus, adipose tissue, testis, and kidney were excised. These excised organs were shredded to a cube with a dimension of about 1 to 2 mm in a PBS solution containing 10 units/ml heparin sodium and thoroughly washed while exchanging solutions. Subsequently, three samples of prepared various organs were added to a 5 ml tube (No. 55.476, Rohren-Tubes; Sarstedt) comprising 200 µl of RPMI 1640 medium containing 10 µmol/l coelenterazine (Molecular Probes) therein and cultured at 37°C for 5 hours. After culturing, 200 µl of the following various physiologically active substances dissolved in RPMI 1640 medium were sequentially added and the luminescence was measured using a luminometer, AutoLumat LB 953 (BERTHOLD), every second immediately after the addition.

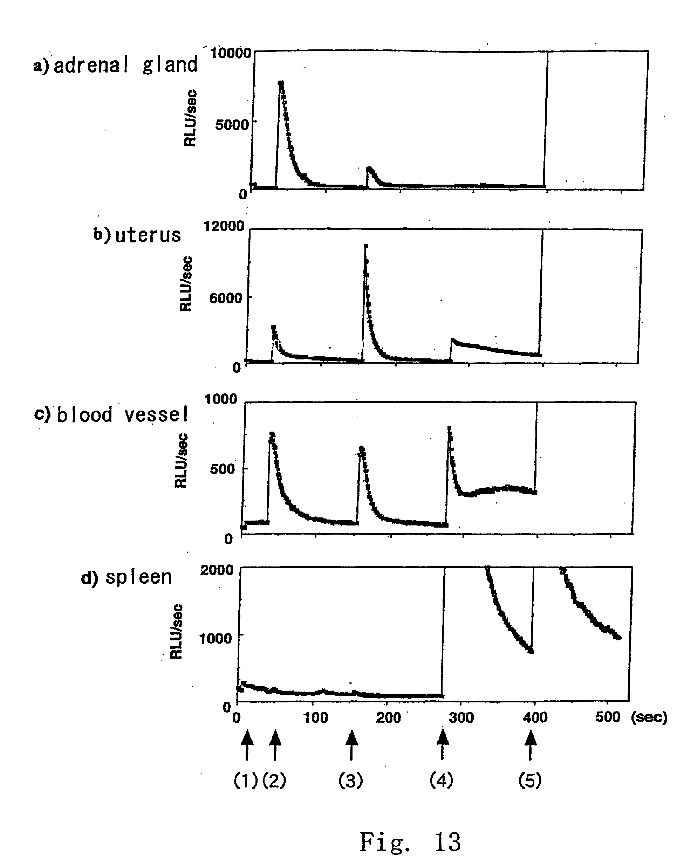

RPMI 1640 medium, angiotensin II (final concentration of 1 µmol/l), bradykinin (final concentration of 10 µmol/l), A23187 (final concentration of 1 µmol/l), and Triton X-100 (final concentration of 2%) were added to each of adrenal gland, uterus, blood vessel, and spleen in that order and the results are shown in Fig. 13.

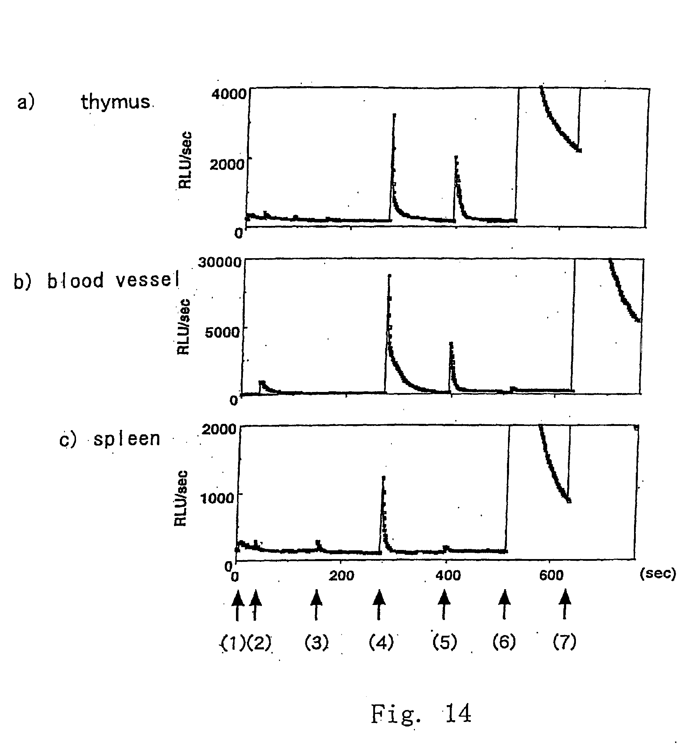

RPMI 1640 medium, carbachol (final concentration of 10 µmol/l), α-methylserotonin (final concentration of 10 µmol/l), ATP (final concentration of 100 µmol/l), phenylephrine (final concentration of 20 µmol/l), A23187 (final concentration of 1 µmol/l), and Triton X-100 (final concentration of 2%) were added to each of thymus, blood vessel, and spleen in that order and the results are shown in Fig. 14.

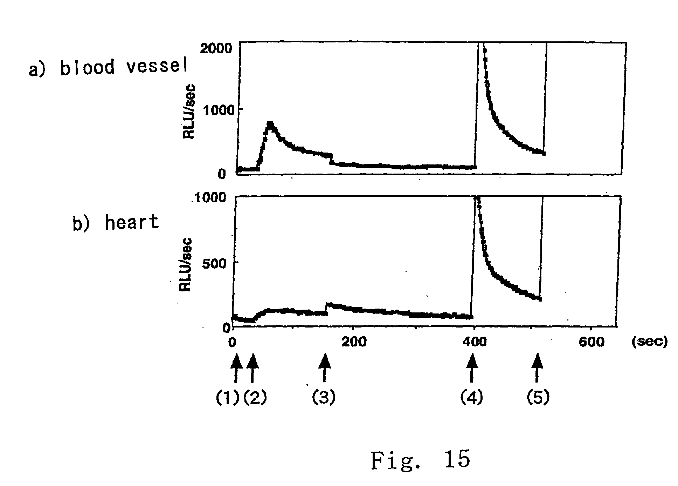

RPMI 1640 medium, endothelin (final concentration of 10 µmol/l), calcitonin (final concentration of 10 µmol/l), A23187 (final concentration of 1 µmol/l), and Triton X-100 (final concentration of 2%) were added to each of blood vessel and heart in that order and the results are shown in Fig. 15.

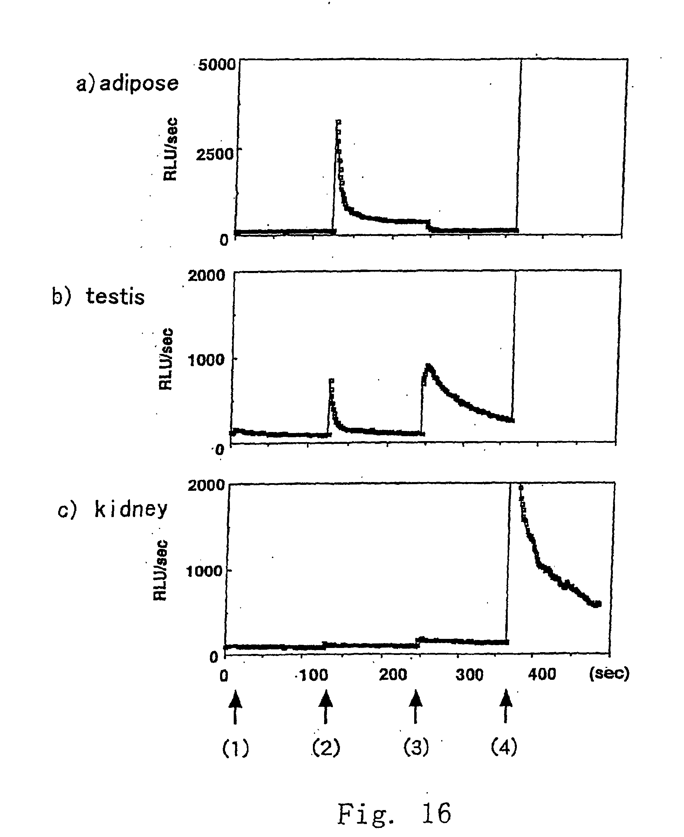

RPMI 1640 medium, phenylephrine (final concentration of 20 µmol/l), A23187 (final concentration of 1 µmol/l), and Triton X-100 (final concentration of 2%) were added to each of adipose tissue, testis, and kidney in that order and the results are shown in Fig. 16.

Angiotensin II (product code: A-151), bradykinin (product code: B-120), carbachol (product code: C-107), α-methylserotonin (product code: M-110), ATP (product code: A-141), phenylephrine (product code: A-133), A23187 (product code: C-161), endothelin (product code: E-134), and Calcitonin (product code: C-210) which were used in the experiment were purchased from Research Biochemicals International.

As a result, strong luminescence was observed in blood vessel, uterus, and adrenal gland with the addition of angiotensin II (final concentration of 1 µmol/l) and with the addition of bradykinin (final concentration of 10 µmol/l) (Fig. 13). When phenylephrine, as an agonist of adrenalin α1 receptor, was added at a final concentration of 20 µmol/l, strong luminescence was observed in thymus, blood vessel, adipose tissue, and testis (Fig. 14, Fig. 16). When carbachol, as an agonist of muscarine receptor, was added at a final concentration of 10 µmol/l, luminescence was observed in blood vessel (Fig. 14). When ATP as an agonist of P2Y receptor was added at a final concentration of 100 µmol/l, strong luminescence was observed in thymus, blood vessel, and spleen (Fig. 14). When endothelin was added at a final concentration of 10 nmol/l, strong luminescence was observed in blood vessel (Fig. 15).

### Example 7: Production of transgenic mouse having hprt gene downstream of PGK promoter and apoaequorin gene ligated to CAG promoter further downstream, using mouse embryonic stem cell

### 1) Culture of mouse embryonic stem cell

Mouse embryonic stem cell AB 2.2 (Lexicon Genetics, catalog No. C 100, hereinafter referred to as "AB 2.2 cell") was cultured on a feeder plate at 37°C in 5% CO₂ using DMEM (Lexicon Genetics; catalog No. M205, hereinafter referred to as "M15 medium") containing 15% fetal bovine serum (Lexicon Genetics, catalog No. B100), 10⁻⁴ mol/l beta-mercaptoethanol (Lexicon Genetics; catalog No. M260), 2 mmol/l L-glutamine, 50 units/ml penicillin, and 50 µg/ml streptomycin (Lexicon Genetics; catalog No. M250). The feeder plate was prepared by culturing mitomycin C-treated mouse embryo fibroblast (Lexicon Genetics; catalog No. C200, hereinafter referred to as "STO cell") in a gelatin-coated dish for cell culturing. Specifically, the feeder plate was prepared by covering a culture face of the dish for cell culturing with 0.1% gelatin solution (Lexicon Genetics; catalog No. M210) for 2 hour or longer to coat the dish with gelatin, thawing the purchased STO cells, suspending them in DMEM (Lexicon Genetics, hereinafter referred to as "STO medium") containing 7% fetal bovine serum (Lexicon Genetics), 2 mmol/l L-glutamine (Lexicon Genetics), 50 units/ml penicillin, and 50 µg/ml streptomycin (Lexicon Genetics) at the density of 4.4 x 10⁵ cells/ml, inoculating onto a 96-well plate in an amount of 0.07 ml/well, 2 ml onto a 3 cm dish, 4 ml onto a 6 cm dish, 12 ml onto a 8.5 cm dish, and 0.5 ml onto a multi-dish and a 24-well plate, respectively, and culturing at 37°C in 5% CO₂ for 48 hour or longer. AB 2.2 cells were suspended in M15 medium and then inoculated onto the feeder plate to initiate the cultureing. 0.1 ml/well M15 medium was used for a 96-well plate, 0.5 ml thereof was used for a multidish and a 24-well plate, 2 ml thereof was used for a 3 cm dish, 4 ml thereof was used for a 6 cm dish, and 10 ml thereof was used for a 8.5 cm dish, and the cells were passaged before they became confluent. Two hours before passage, M15 medium was exchanged. After washing twice with a PBS buffer (Lexicon Genetics), 0.25% trypsin solution (Lexicon Genetics; M220) containing 0.025 ml/well of 0.04% EDTA on a 96-well plate, 0.25 ml thereof on a multidish and a 24-well plate, 1 ml thereof on a 3 cm dish, 2 ml thereof on a 6 cm dish, and 2 ml thereof on a 8.5 cm dish, was added and allowed to stand for 15 minutes at 37°C in 5% CO₂. Thereafter, an equal amount of M15 medium was added thereto and the mixture was vigorously stirred forty times using a plastic pipette to disperse cells. Cells precipitated by centrifugation were then resuspended in M15 medium and inoculated onto a new feeder plate for passage.

### 2) Introduction of apoaequorin expressing gene pCAG-AEQ-pHPRTp into mouse embryonic stem cells and selection of a strain expressing apoaequorin at high level

pCAG-AEQ-pHPRTp was introduced into AB 2.2 cells by electroporation. The purchased 3.0 x 10⁶ of AB 2.2 cells were inoculated onto a 6 cm dish and cultured for 48 hours. The cultured cells were then transferred to a 8.5 cm dish for subculturing and was further cultured for 24 hours. Twenty four hours later, the cultured cells were washed twice with 7 ml of PBS, 2 ml of trypsin solution (Lexicon Genetics) was added thereto, and the cells were allowed to stand for 15 minutes at 37°C in 5% CO₂. 2 ml of M15 medium was then added and vigorously pipetted forty times using a 3 ml transfer pipette (Falcon, catalog No. 7575), 1.0 x 10⁷ cells, precipitated by centrifugation, was suspended in 0.9 ml PBS buffer (Lexicon Genetics), and 22.7 µl of 1.1 µg linear pCAG-AEQ-pHPRTp was mixed in a fusion chamber, Gene Pulser Cuvette (gap distance of 4 mm, BIORAD, catalog No. 165-2088). The linear pCAG-AEQ-pHPRTp was provided by cleaving plasmid pCAG-AEQ-pHPRTp prepared in 9) of Example 1 with a restriction enzyme *Asc*I, extracting the plasmid with phenol/chloroform, performing ethanol precipitation, and dissolving it in 1 mmol/l Tris (pH 8.0) solution containing 0.1 mmol/l EDTA. Using a cell fusing machine, Gene Pulser (BIORAD), genes were introduced at a pulse voltage of 0.23 kv with a capacitance of 500 µF, and the cells were suspended in 50 ml of M15 medium, inoculated onto five sheets of 8.5 cm feeder plates at 10 ml/plate, and then cultured for 24 hours. Twenty four hours later, the cultured cells were further cultured for 8 days in M15 medium containing 1 x concentrate HAT Supplement (100 x, Gibco BRL, catalog No. 3992) to form drug-resistant colonies. These colonies were collected on a 96-well feeder plate at 1 colony/well, culturing was continued and then a replica was prepared. The colony was washed twice with PBS buffer (Lexicon Genetics), and collected with the use of a plastic sterilized chip by directly picking up onto a round-bottomed 96-well plate (Falcon, catalog No. 3077) onto which 30 µl of trypsin solution was added at 1 colony/well. Subsequently, the cells were allowed to stand for 15 minutes or longer at 37°C in 5% CO₂, and a cell suspension was prepared by adding 70 µl/well M15 medium and dispersing the cells through forty times of vigorous pipetting with a plastic sterilized chip. The cell suspension was inoculated onto a 96-well feeder plate in which 100 µl of M15 medium was added in each well and was cultured for 3 days. Three days later, a replica plate was prepared and further cultured for 3 days. The replica plate was provided by washing the above-mentioned 96-well plate twice with 100 µl of PBS solution per well, adding 25 µl of trypsin solution and being allowed to stand for 15 minutes or longer at 37°C in 5% CO₂, adding 25 µl of M15 medium and dispersing the cells through forty times of vigorous pipetting with a plastic sterilized chip, collecting 100 µl from a cell suspension prepared by adding and mixing 50 µl of DMEM (hereinafter refereed to as "2 x freezing medium") containing 20% DMSO (Sigma, catalog No. D2650) and 40% fetal bovine serum (Lexicon Genetics), and then transferring to a white plate for cell culturing (Sumitomo Bakelite, product No. MS-8096W). The plate was provided by previously coating with gelatin and adding 100 µl of M15 per well. The plate containing remaining cell suspension was cryopreserved at -80°C as a master plate. Three days later, the medium was removed and M15 medium containing 10 µmol/l coelenterazine was added at 100 µl/well, followed by culturing for 4 hours. Four hours later, 100 µl of modified Krebs-Ringer buffer containing 2 µmol/l calcium ionophore A23187 (Research Biochemical International) was added to each well and luminescence was immediately measured for 5 seconds. The level of apoaequorin expression was compared among various drug-resistant cells assayed in accordance with the above method to select a cell with the highest level of apoaequorin expression, AB 2.2 clone No. 1A7.

Subsequently, an injection stock into mouse blastcyst of AB 2.2 clone No. 1A7 was prepared. The cryopreserved master plate was thawed, a cell suspension containing 1A7 was inoculated in a well on a 24-well feeder plate to which 2 ml of M15 medium had been added, and the medium was exchanged after 24 hours of culturing, followed by further culturing for three days. Three days later, the cultured cells were transferred to a well of the 24-well feeder plate for passage, further cultured for 2 days, and then transferred to a 3 cm dish for passage. Twenty four hours later, the cells, which had been precipitated by centrifugation after detached from the dish as in the passage method, were suspended in 0.5 ml of M15 medium, and 0.1 ml of suspension prepared by adding and mixing 0.5 ml of 2 x freezing medium was added in a tube for cell freezing, and then the cells were cryopreserved at -80°C. Thus, an injection stock in a mouse blastocyst was prepared.

### 3) Injection of mouse embryonic stem cell into blastocyst and implantation of the blastocyst

AB 2.2 clone 1A7 expressing the established apoaequorin at high level, was cultured in a multidish (Nunc, catalog No. 176740) when injecting into mouse blastocyst. The injection stock for mouse blastocyst was thawed and then suspended in 10 ml of M15 medium and the cells, which had been precipitated by centrifugation, were inoculated onto a multidish to initiate culturing. The cell was transferred to a multidish again 24 hours later for subculturing, and after the additional 24 hours of culturing, the cell was washed twice with 0.5 ml of PBS (Lexicon Genetics), and 0.25 ml of trypsin solution (Lexicon Genetics) was added thereto. The mixture was allowed to stand for 15 minutes at 37°C in 5% CO₂, 0.75 ml of M15 medium was added thereto, and vigorously pipetted forty times using a 3 ml transfer pipet (Falcon, catalog No. 7575) to prepare a suspension. Fetal bovine serum (Lexicon Genetics) was added thereto and mixed. The thus obtained cell suspension was used for injection into mouse blastocyst. The mouse blastocyst was obtained by naturally mating a female C57B1/6J mouse, which was subjected to superovulation, with a male mouse of the same lineage and perfusing the inside of uterus excised four days later with M15 medium. This mouse blastocyst was allowed to stand at 37°C in 5% CO₂ until the blastocoele was sufficiently expanded, and transferred into M15 medium containing 20 mmol/l HEPES, which had been cooled to about 4°C. While observing under an inverted microscope (Nikon Corp.) equipped with microinjector (NARISHIGE CO., LTD.) and micromanipulator (NARISHIGE CO., LTD.), 10 to 15 AB 2.2 clone 1A7 was microinjected into the blastocoele through the injection needle operation. The blastcyst was allowed to stand at 37°C in 5% CO₂ until the blastocoele was expanded. In accordance with a method described in "Manipulating the Mouse Embryo, A Laboratory Manual", Second Edition, Cold Spring Harbor Laboratory Press (1994), the blastocoele was transplanted to an uterus portion on the oviduct side of a pseudopregnant female MCH mouse to implant. A female MCH mouse (10-week or older) in preestrus to estrus was subjected to cohabitation and mating in 1 : 1 with a 10-week or older vasoligated male MCH mouse at 17:00 three days before the transplantation, followed by examination of the copulatory plug at 9:00 on the following morning. Then, it was used for the above purpose two days later.

### 4) Identification of germ line chimera mouse

Among chimera individuals in which brown coat was developed in black coat because of the injected 1A7, male individuals having more than 50% chimera rate were bred to 8-week-old and mated with an 8 week or older female C57BL/6J individual in 1 : 1, thereby obtaining the offspring. Among the offsprings, genome DNA collected from a tail of the individuals having brown coat was used to examine the presence or absence of a transgene in accordance with a method described in 3) of Example 4. Genome DNA was prepared in accordance with a method described in 2) of Example 4. Brown offspring was obtained from five individuals out of six male individuals and the offspring having a transgene was obtained from two individuals. From this result, the above two individuals, named "Kuro" and "Ryu" respectively, were identified as the germ line chimera mice.

### 5) Analysis of transgene expression in transgenic mouse prepared from mouse embryonic stem cell and assay of physiologically active substance using the mouse

Tissues or organs were prepared respectively from offspring of "Ryu" and the apoaequorin expression was examined in accordance with the method described in Example 5. As a result, luminescence was observed in all the samples, specifically, thymus, testis, seminal vesicle, spleen, abdominal muscle, diaphragm, kidney, crystalline lens, lung, pancreas, adipose tissue, skin, liver, cranial bone, esophagus, blood vessel, heart, hair root, tail, ear, blood, bone marrow, teeth, tongue, stomach, small intestine, colon, urinary bladder, penis, ovary, uterus, oviduct, nail, thyroid gland, and adrenal gland, obtained from the offsprings of "Ryu".

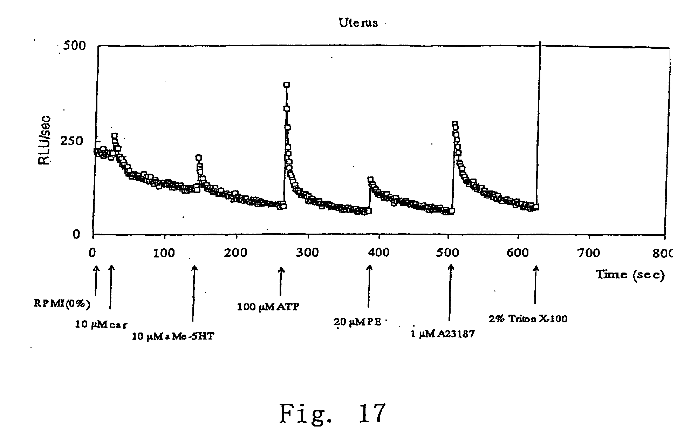

Subsequently, tissues or organs were prepared from male and female offspring derived from the mouse in accordance with a method described in Example 6, and responsiveness toward physiologically active substances was examined. As representatives, RPMI 1640 medium, carbachol (final concentration of 20 µmol/l), α-methylserotonin (final concentration of 20 µmol/l), ATP (final concentration of 100 µmol/l), phenylephrine (final concentration of 20 µmol/l), A23187 (final concentration of 1 µmol/l), and Triton X-100 (final concentration of 2%) were added to uterus in that order, and the results are shown in Fig. 17. Strong liminescence was observed with the addition of 20 µmol/l carbachol, 20 µmol/l α-methylserotonin, 100 µmol/l ATP, or 20 µmol/l phenylephrine.

### Example 8: Analysis of stable transformant having apoaequorin gene introduced downstream of DNA of the present invention

In accordance with the method described in Example 3, 10 stable transformants, wherein plasmid pCAG-AEQ-pHPRTp had been introduced into mouse myeloma cell P3U1, were optionally provided, and Cre recombinase was transiently expressed in these provided cells to prepare a mutant from which the expression unit of an hprt gene were removed. Thus, the level of apoaequorin expression before the removal of the hprt expression unit was compared with that after the removal.

In each of the transformants, decreased apoaequorin expression level was observed by the removal of an hprt gene expression unit. In most transformants, the level of expression was descreased by 5 to 10 times, and in one transformant, there was almost no apoaequorin expression after the removal.

In accordance with the method described in Example 3, plasmid pCAG-AEQ-pHPRTp or pCAG-AEQ was introduced into mouse myeloma cell P3U1. Twenty four hours later, the level of transient apoaequorin expression was assayed and compared. There was no significant difference between P3U1 cell into which pCAG-AEQ had been introduced and P3U1 cell into which pCAG-AEQ-pHPRTp had been introduced.

These results suggest that the upstream region of an apoaequorin gene of pCAG-AEQ-pHPRTp plasmid, that is, a nucleotide sequence region in loxP, a PGK promoter, an hprt gene, loxP, and a CAG promoter, shown by SEQ ID NO: 1 has a function to strongly express genes existing downstream on the chromosome.

### Example 9: Preparation of transgenic mouse containing gene in which GFP gene has been ligated to downstream of DNA of the present invention

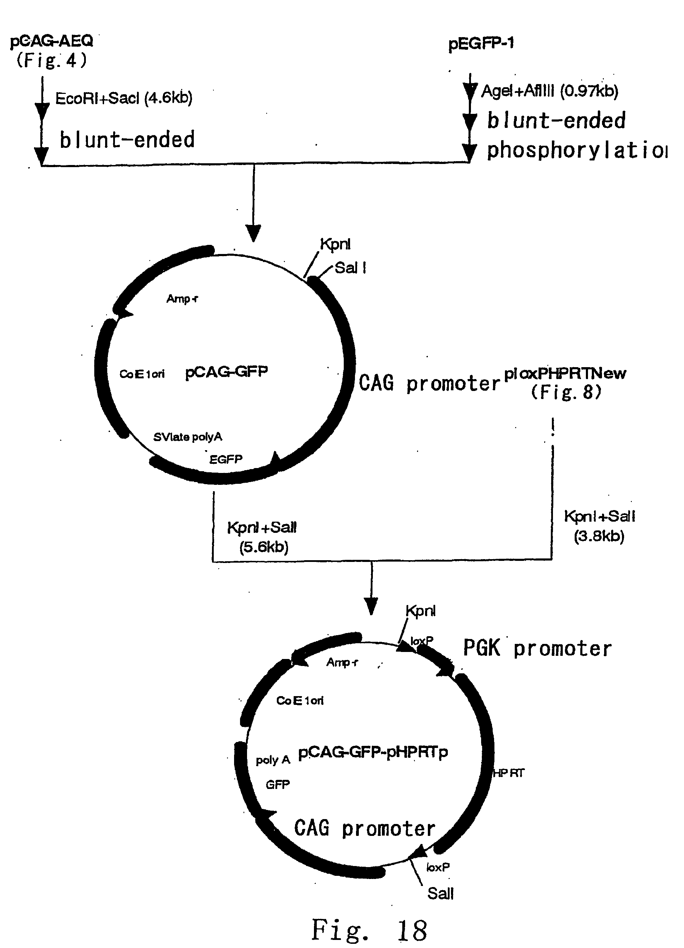

### 1) Construction of plasmid pCAG-GFP-pHPRTp containing hprt gene downstream of PGK promoter and GFP gene ligated to CAG promoter further downstream (see Fig. 18)

3 µg of plasmid pCAG-AEQ, which had been prepared in accordance with the method described in 6) of Example 1, was dissolved in 20 µl of Universal Buffer L (Takara Shuzo) and 10 units of *Sac*I were added thereto, followed by digestion at 37°C for 2 hours. Thereafter, 4 µl of 10 x Universal Buffer H (Takara Shuzo) and 10 units of *Eco*RI were added so as to bring the total amount to 40 µl, and digestion was then carried out at 37°C for an additional 2 hours. The reaction solution was subjected to agarose gel electrophoresis, and DNA fragment of about 4.6 kb containing an ampicillin (Ap) resistant gene was then collected using QIAEX II Gel Extraction Kit (QIAGEN).

2 µg of plasmid pEGFP-1 (Clontech, catalog No. 6086-1) was dissolved in 30 µl of NE Buffer 1 (New England Biolabs) and 10 units of *Age*I were added thereto, followed by digestion at 25°C for 2 hours. Thereafter, 4 µl of 10 x NE Buffer 3 (New England Biolabs) and 10 units of *Afl*III were added so as to bring the total amount to 40 µl, and digestion was then carried out at 37°C for 2 hours. The reaction solution was subjected to agarose gel electrophoresis, and GFP gene of about 0.97 kb and a DNA fragment containing a polyadenylation signal for an SV 40 late gene were then collected.

1.0 µg of *Eco*RI*-Sac*I fragment (4.6 kb) derived from plasmid pCAG-AEQ and 1.0 µg of *Age*I-*Afl*III fragment (0.97 kb) derived from plasmid pEGFP-1, which had been prepared above, were respectively processed into blunt-end in accordance with a method described in an attached manual using DNA Blunting Kit (Takara Shuzo, catalog No. 6025). Each DNA was dissolved in 9 µl of reaction buffer (Takara Shuzo) appendant to the kit and allowed to stand for 5 minutes at 70°C. Thereafter, 1 unit of T4 DNA polymerase (Takara Shuzo) was added so as to bring the total amount to 10 µl and reacted at 37°C for 5 minutes. Immediately after that, the reaction mixture was vigorously stirred using a vortex mixer to inactivate the enzyme, thereby terminating the reaction. Subsequently, the reaction solution of *Age*I*-Afl*III fragment derived from plasmid pEGFP-1 was extracted with phenol/chloroform, 5 µl thereof was then collected, 2.5 µl of T4 polynucleotide kinase buffer (Takara Shuzo) and 10 units of T4 polynucleotide kinase (Takara Shuzo) were added so as to bring the total amount to 25 µl, and the 5' terminus of DNA was phosphorylated at 37°C for 30 minutes. The reaction solution was allowed to stand at 65°C for 10 minutes to terminate the reaction. 0.1 µg of blunt-end DNA fragment (4.6 kb) derived from plasmid pCAG-AEQ and 0.1 µg of phosphorylated blunt-end DNA fragment (0.97 kb) derived from pEGFP-1, which had been prepared as described above, were dissolved in 30 µl of T4 ligase buffer. 100 units of T4 DNA ligase were added thereto and a ligation reaction was carried out at 12°C for 16 hours. The reaction solution was used to transform *Escherichia coli* DH5 (Toyobo Co.) in accordance with a method by Cohen et al. to obtain an ampicillin resistant (Ampr) strain. Plasmid DNA was isolated from this transformant in accordance with a conventional method. The construction of the obtained plasmid was confirmed through digestion by a restriction enzyme. This plasmid is hereinafter referred to as pCAG-GFP.

2 µg of plasmid ploxpHPRTnew prepared in accordance with a method described in 8) of Example 1 was dissolved in 30 µl of Universal Buffer L (Takara Shuzo), and 10 units of *Kpn*I were added thereto, followed by digestion at 37°C for 2 hours. Thereafter, 4 µl of 10 x Universal Buffer H (Takara Shuzo) and 10 units of *Sal*I were added so as to bring the total amount to 40 µl, and digestion was then carried out at 37°C for an additional 2 hours. The reaction solution was subjected to agarose gel electrophoresis and about 3.8 kb of DNA fragment containing an hprt gene was then collected.

Separately, 2 µg of plasmid pCAG-GFP was dissolved in 30 µl of Universal Buffer L (Takara Shuzo), and 10 units of *Kpn*I were added thereto, followed by digestion at 37°C for 2 hours. Thereafter, 4 µl of 10 x Universal Buffer H (Takara Shuzo) and 10 units of *Sal*I were added so as to bring the total amount to 40 µl, and digestion was then carried out at 37°C for an additional 2 hours. The reaction solution was subjected to agarose gel electrophoresis and DNA fragment of about 5.6 kb containing an ampicillin (Ap) resistant gene was then collected.

0.1 µg of *Kpn*I*-Sal*I fragment (3.8 kb) derived from plasmid ploxpHPRTnew and 0.1 µg of *Kpn*I*-Sal*I fragment (5.6 kb) derived from plasmid pCAG-GFP, which had been obtained above, were dissolved in 30 µl of T4 ligase buffer. 100 units of T4 DNA ligase were added, and a ligation reaction was carried out at 12°C for 16 hours. The reaction solution was used to transform *Escherichia coli* DH5 (Toyobo Co.) in accordance with a method by Cohen et al. to provide an ampicillin resistant (Ampr) strain. Plasmid DNA was isolated from this transformant in accordance with a conventional method. The construction of the obtained plasmid was confirmed through digestion by a restriction enzyme. This plasmid is hereinafter referred to as pCAG-GFP-pHPRTp.

### 2) Preparation of transgenic mouse having hprt gene downstream of PGK promoter and GFP gene ligated to CAG promoter further downstream

In accordance with the method described in Example 7, the prepared GFP expression gene pCAG-GFP-pHPRTp was introduced into mouse embryonic stem cell AB 2.2. Among the obtained drug-resistant cells, a cell expressing GFP at high level was selected, and the selected cell was injected into mouse blastocyst, and then transferred into uterus of a pseudopregnant female mouse. By identifying a germ line chimera mouse among the produced chimera mice, a transgenic mouse 3C5' having a gene in which a GFP gene was ligated to a nucleotide sequence shown by SEQ ID NO: 1 was prepared. The offspring of the prepared transgenic mouse 3C5' expressed a GFP gene at high level, and thus, skin looked green even under a fluorescent lamp. Therefore, it could be determined whether the offspring was transgenic or not without the need to detect a transgene using genome DNA.

Tissues and organs were excised from the offspring of prepared transgenic mouse 3C5' to test whether the fluorescence of GFP was observed. The fluorescence was detected using Lumino Image Analyzer LAS-1000 (Fuji Photo Film). After the offspring was sacrificed, thymus, testis, seminal vesicle, spleen, abdominal muscle, diaphragm, kidney, crystalline lens, lung, pancreas, adipose tissue, skin, liver, cranial bone, esophagus, blood vessel, heart, hair root, tail, ear, blood, bone marrow, teeth, tongue, stomach, small intestine, colon, urinary bladder, penis, ovary, uterus, oviduct, nail, thyroid gland, and adrenal gland were extracted. These organs were placed on a tray, which had been placed in a dark box, and the fluorescence of the organs was assayed with a cooled CCD camera. Blue-SQW-LED (Nichia Corporation) emitting blue light at around 470 nm was used as an excitation light source, and the organs were photographed by mounting a fluorescent filter Y515-Di (Fuji Photo Film) in front of a camera lens. As a result, the fluorescence of GFP was observed in all the excised samples. After the abdominal operation on the offspring, a portable ultraviolet lamp, Model UVGL-58 (UVP Inc.), was used to apply ultraviolet to the organs. As a result, the emission of green fluorescence from each organ could be visually observed.

### INDUSTRIAL APPLICABILITY

The non-human mammal comprising DNA encoding a foreign reporter protein for monitoring changes in intracellular calcium ion concentration according to the present invention, expresses the DNA in at least one tissue or organ selected from the group consisting of epithelial tissue, connective tissue, adipose tissue, cartilage tissue, bone tissue, muscular tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ, and nail, and can be used for evaluation of the pharmacological effect and the activity of physiologically active substances (for example, drugs), novel gene products having unknown functions and the like in these tissues and organs. In particular, the non-human mammalians of the present invention can be used in assaying, for example, the drug responsiveness in cells for which *in vitro* culturing while maintaining the physiological function is difficult, and in tissues or organs having a complex intercellular network. Also, there can be induced, for example, heart diseases (for example, acute heart failure, chronic heart failure, and myocarditis), respiratory disorder, articular diseases (for example, rheumatoid arthritis and osteoarthritis), renal diseases (for example, kidney failure, glomerular nephritis, and IgA nephropathy), arteriosclerosis, psoriasis, hyperlipidemia, allergic diseases (for example, asthma, allergic rhinitis, and atopic dermatitis), bone diseases (for example, osteoporosis, rachitis, osteohalisteresis, and hypocalcemia), blood diseases, cerebrovascular disorder, traumatic encephalopathy, infectious disease, dementia, and chronic inflammatory disease into the transgenic non-human mammal of the present invention, and the alteration in intracellular calcium level can be assayed in such pathological animal models. Thus, the non-human mammal of the present invention can be useful for the elucidation of pathology and mechanisms of these diseases, the study of therapeutic methods, and the screening of candidate compounds for the research and development of therapeutic agents.

## Claims

1. A transgenic non-human mammal which has DNA encoding a foreign reporter protein for monitoring changes in intracellular calcium ion concentration, and expresses the reporter protein in one or more types of non-neuronal cells.

2. The transgenic non-human mammal according to claim 1 wherein the non-neuronal cells are cells which compose a site selected from the group consisting of epithelial tissue, connective tissue, adipose tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail.

3. The transgenic non-human mammal according to claim 1 or 2 wherein the DNA encoding a foreign reporter protein is DNA selected from the group consisting of the following (a), (b), (c) and (d):
(a) DNA encoding apoaequorin;
(b) DNA encoding a protein which comprises an amino acid sequence derived from the amino acid sequence of apoaequorin by deletion, substitution, or addition of one or more amino acids, and which is capable of emitting light upon binding with calcium ion;
(c) DNA encoding a protein which comprises an amino acid sequence having 50% or more homology with the amino acid sequence of apoaequorin, and which is capable of emitting light upon binding with calcium ion;
(d) DNA capable of hybridizing to any one of the DNAs of (a) to (c) under stringent conditions, and encoding a protein which is capable of emitting light upon binding with calcium ion;
(e) DNA encoding cameleon;
(f) DNA encoding a protein which comprises an amino acid sequence derived from the amino acid sequence of cameleon by deletion, substitution, or addition of one or more amino acids, and which is capable of causing fluorescence resonance energy transfer (FRET) upon binding with calcium ion;
(g) DNA encoding a protein which comprises an amino acid sequence having 50% or more homology with the amino acid sequence of cameleon, and which is capable of causing fluorescence resonance energy transfer (FRET) upon binding with calcium ion;
(h) DNA capable of hybridizing to any one of the DNAs of (e), (f) and (g) under stringent conditions, and encoding a protein which is capable of causing fluorescence resonance energy transfer (FRET) upon binding with calcium ion.

4. The transgenic non-human mammal according to any one of claims 1 to 3 wherein the DNA encoding a foreign reporter protein is ligated to DNA having promoter activity that exists upstream of the DNA.

5. The transgenic non-human mammal according to claim 4 wherein the DNA having promoter activity is any one of the following DNAs of (a) to (i):
(a) DNA having SE promoter sequence;
(b) DNA having CAG promoter sequence;
(c) DNA having PGK promoter sequence;
(d) DNA having a sequence in which hprt gene is inserted between two types of promoters;
(e) DNA in which loxP, promoter, hprt gene, loxP and promoter are linked in this order;
(f) DNA having a nucleotide sequence represented by SEQ ID NO: 1;
(g) DNA comprising a nucleotide sequence derived from the nucleotide sequence of any one of the DNAs of (a) to (f) by deletion, substitution, or addition of one or more nucleotides and having promoter activity;
(h) DNA comprising a nucleotide sequence which has 50% or more homology with the nucleotide sequence of any one of the DNAs of (a) to (f) and having promoter activity;
(i) DNA capable of hybridizing to any one of the DNAs of (a) to (f) under stringent conditions and having promoter activity;

6. The transgenic non-human mammal according to any one of claims 1 to 5, which is a non-human mammal selected from the group consisting of a mouse, rat, guinea pig, hamster, rabbit, dog, cat, sheep, pig, goat, cow and monkey.

7. The transgenic non-human mammal according to any one of claims 1 to 6, which is a non-human mammal having a mutated gene on the chromosome.

8. A method for producing the transgenic non-human mammal according to any one of claims 1 to 7, which comprises the steps of constructing a transgene which comprises DNA encoding a foreign reporter protein and DNA having promoter activity located upstream of the DNA; microinjecting the transgene into a fertilized egg of a non-human mammal; transplanting the obtained egg to the oviduct or uterus of a pseudopregnant female non-human mammal; breeding the transplanted animal; and from the offsprings, selecting individuals having the transgene.

9. A method for producing the transgenic non-human mammal according to any one of claims 1 to 7, which comprises the steps of constructing a transgene which comprises DNA encoding a foreign reporter protein and DNA having promoter activity located upstream of the DNA; introducing the transgene into an embryonic stem cell of a non-human mammal; introducing the embryonic stem cell into a fertilized egg of a non-human mammal; transplanting the obtained egg to the oviduct or uterus of a pseudopregnant female non-human mammal; breeding the transplanted animal; and from the offsprings, selecting individuals having the transgene.

10. A method for producing the transgenic non-human mammal according to any one of claims 1 to 7, which comprises the steps of constructing a transgene which comprises DNA encoding a foreign reporter protein and DNA having promoter activity located upstream of the DNA; introducing the transgene into a cell of a non-human mammal; introducing the nucleus of the cell into an unfertilized and enucleated egg of a mammal; transplanting the obtained egg to the oviduct or uterus of a pseudopregnant female non-human mammal; breeding the transplanted animal; and from the offsprings, selecting individuals having the transgene.

11. A method for producing the transgenic non-human mammal according to any one of claims 1 to 7, which comprises the steps of constructing a transgene which comprises DNA encoding a foreign reporter protein and DNA having promoter activity located upstream of the DNA; allowing a sperm of a non-human mammal to incorporate the transgene; allowing the sperm to fertilize an unfertilized egg; transplanting the obtained egg to the oviduct or uterus of a pseudopregnant female non-human mammal; breeding the transplanted animal; and from the offsprings, selecting individuals having the transgene.

12. A method for producing the transgenic non-human mammal according to any one of claims 1 to 7, which comprises the steps of constructing a transgene which comprises DNA encoding a foreign reporter protein and DNA having promoter activity located upstream of the DNA; integrating the transgene into a retrovirus vector; infecting an egg cell with virus containing the retrovirus vector; transplanting the obtained egg to the oviduct or uterus of a pseudopregnant female non-human mammal; breeding the transplanted animal; and from the offsprings, selecting individuals having the transgene.

13. An embryonic stem cell of a non-human mammal in which DNA encoding a foreign reporter protein is introduced, which is used for producing the transgenic non-human mammal according to any one of claims 1 to 7.

14. A cell of the transgenic non-human mammal according to any one of claims 1 to 7.

15. The cell according to claim 14, which is a cell selected from the group consisting of an embryonic stem cell, an egg and a sperm.

16. A nucleus of the cell according to any one of claims 13 to 15.

17. A method for detecting changes in intracellular calcium ion concentration by using a foreign reporter protein, wherein the transgenic non-human mammal according to any one of claims 1 to 7 expressing the foreign reporter protein, or the tissue, organ or cell of the animal, is used.

18. The method according to claim 17, wherein the transgenic non-human mammal is a living transgenic non-human mammal.

19. A method for detecting changes in intracellular calcium ion concentration by using a foreign reporter protein introduced into an embryonic stem cell, which comprises inducing differentiation of the embryonic stem cell according to claims 13 or 15, and detecting changes in intracellular calcium ion concentration of the cell obtained by the induction of differentiation.

20. A method for screening a substance involved in the regulation of intracellular calcium ion concentration, which comprises contacting a test substance with the transgenic non-human mammal according to any one of claims 1 to 7 expressing a foreign reporter protein, or the tissue, organ or cell of the animal; and detecting changes in intracellular calcium ion concentration using the foreign reporter protein.

21. A method for producing a transgenic non-human mammal wherein the cell according to claims 14 or 15 is used.

22. A method for producing a cloned non-human mammal wherein the nucleus of the cell of claim 16 is used.

23. A transgenic or cloned non-human mammal which is produced by the method according to claim 21 or 22.

24. A method for producing a gene-modified mammal, which comprises mating the transgenic non-human mammal according to any one of claims 1 to 7 with an animal belonging to the same species but to a different strain from the transgenic non-human mammal.

25. A gene-modified mammal which is obtained by the method of claim 24.

26. A method for detecting changes in intracellular calcium ion concentration using a foreign reporter protein, wherein the mammal according to claim 23 or 25 expressing the foreign reporter protein, or the tissue, organ or cell of the animal is used.

27. The method according to claim 26, wherein the mammal is a living mammal.

28. A method for screening a substance involved in the regulation of intracellular calcium ion concentration, which comprises contacting a test substance with the mammal according to claims 23 or 25 expressing a foreign reporter protein, or the tissue, organ or cell of the animal; and detecting changes in intracellular calcium ion concentration using the foreign reporter protein.

29. DNA selected from the following (a) to (f):
(a) DNA having a sequence in which hprt gene is inserted between two types of promoters;
(b) DNA in which loxP, promoter, hprt gene, loxP and promoter are ligated in this order;
(c) DNA having a nucleotide sequence represented by SEQ ID NO: 1;
(d) DNA comprising a nucleotide sequence derived from the nucleotide sequence of any one of the DNAs of (a) to (c) by deletion, substitution, or addition of one or more nucleotides and having promoter activity;
(e) DNA comprising a nucleotide sequence which has 50% or more homology with the nucleotide sequence of any one of the DNAs of (a) to (c) and having promoter activity;
(f) DNA capable of hybridizing to any one of the DNAs of (a) to (c) under stringent conditions and having promoter activity.

30. The DNA according to claim 29, wherein the DNA having a sequence in which hprt gene is inserted between two types of promoters is DNA having a sequence in which hprt gene is inserted between PGK promoter and CAG promoter.

31. The DNA according to claim 29, wherein the DNA in which loxP, promoter, hprt gene, loxP and promoter are ligated in this order is DNA in which loxP, PGK promoter, hprt gene, loxP and CAG promoter are ligated in this order.

32. The DNA according to any one of claims 29 to 31, which has, when integrated into a chromosome of a mammal, promoter activity higher than the respective promoter activity of loxP, promoter, and hprt gene that compose the DNA.

33. The DNA according to claim 32 which has, when integrated into a chromosome of a mammal, promoter activity higher than the respective promoter activity of loxP, promoter, and hprt gene that compose the DNA, in a cell composing a site selected from the group consisting of epithelial tissue, connective tissue, adipose tissue, cartilage tissue, bone tissue, muscle tissue, intraoral tissue, blood tissue, bone marrow, cardiovascular organ, lymphatic organ, teeth, digestive tract, liver, gallbladder, pancreas, respiratory organ, urinary organ, genital organ, endocrine organ, sense organ and nail.

34. A transgenic non-human mammal, which has DNA wherein DNA encoding a foreign protein is ligated downstream of the DNA according to any one of claims 29 to 33.

35. The transgenic non-human mammal according to claim 34, wherein the DNA encoding a foreign protein is DNA selected from the following (a) or (b):
(a) DNA encoding a foreign reporter protein for monitoring changes in intracellular calcium ion concentration;
(b) DNA encoding a foreign reporter protein for labeling a cell.

36. The transgenic non-human mammal according to claim 35 wherein the DNA encoding a foreign reporter protein for monitoring changes in intracellular calcium ion concentration is DNA selected from the group consisting of the following (a), (b), (c), (d), (e), (f), (g) and (h):
(a) DNA encoding apoaequorin;
(b) DNA encoding a protein which comprises an amino acid sequence derived from the amino acid sequence of apoaequorin by deletion, substitution, or addition of one or more amino acids, and which is capable of emitting light upon binding with calcium ion;
(c) DNA encoding a protein which comprises an amino acid sequence having 50% or more homology with the amino acid sequence of apoaequorin, and which is capable of emitting light upon binding with calcium ion;
(d) DNA capable of hybridizing to any one of the DNAs of (a) to (c) under stringent conditions and encoding a protein which is capable of emitting light upon binding with calcium ion;
(e) DNA encoding cameleon;
(f) DNA encoding a protein which comprises an amino acid sequence derived from the amino acid sequence of cameleon by deletion, substitution, or addition of one or more amino acids, and which is capable of causing fluorescence resonance energy transfer (FRET) upon binding with calcium ion;
(g) DNA encoding a protein which comprises an amino acid sequence having 50% or more homology with the amino acid sequence of cameleon, and which is capable of causing fluorescence resonance energy transfer (FRET) upon binding with calcium ion;
(h) DNA capable of hybridizing to any one of the DNAs of (e) to (g) under stringent conditions, and encoding a protein which is capable of causing fluorescence resonance energy transfer (FRET) upon binding with calcium ion.

37. The transgenic non-human mammal according to claim 35, wherein the DNA encoding a foreign reporter protein for labeling a cell is DNA selected from the following (a) or (b):
(a) DNA encoding a fluorescent protein;
(b) DNA encoding a photo- or chromogenic protein.

38. The transgenic non-human mammal according to claim 37, wherein the DNA encoding a fluorescent protein is DNA selected from the group consisting of the following (a), (b), (c), (d) and (e):
(a) DNA encoding the green fluorescent protein (GFP) of jelly fish;
(b) DNA encoding the red fluorescent protein (DsRed) of sea anemone;
(c) DNA encoding a protein which comprises an amino acid sequence derived from the amino acid sequence of the fluorescent protein of (a) or (b) by deletion, substitution, or addition of one or more amino acids, and which is capable of emitting fluorescence;
(d) DNA encoding a protein which comprises an amino acid sequence having 50% or more homology with the amino acid sequence of the fluorescent protein of (a) or (b), and which is capable of emitting fluorescence;
(e) DNA capable of hybridizing to any one of the DNAs of (a) to (d) under stringent conditions and encoding a protein which is capable of emitting fluorescence.

39. The transgenic non-human mammal according to claim 37, wherein the DNA encoding a photo- or chromogenic protein is DNA selected from the group consisting of the following (a), (b), (c), (d), (e), (f), (g) and (h):
(a) DNA encoding luciferase of firefly;
(b) DNA encoding luciferase of *Renilla*;
(c) DNA encoding alkaline phosphatase:
(d) DNA encoding β-galactosidase;
(e) DNA encoding chroramphenicol acetyltransferase;
(f) DNA encoding a protein which comprises an amino acid sequence derived from the amino acid sequence of the photo- or chromogenic protein according to any one of (a) to (e) by deletion, substitution, or addition of one or more amino acids, and which is capable of catalyzing light emission or color development by acting on any substrate;
(g) DNA encoding a protein which comprises an amino acid sequence having 50% or more homology with the amino acid sequence of the photo- or chromogenic protein according to any one of (a) to (e), and which is capable of catalyzing light emission or color development by acting on any substrate;
(h) DNA capable of hybridizing to any one of the DNAs of (a) to (g) under stringent conditions and encoding a protein which is capable of catalyzing light emission or color development by acting on any substrate.

40. The transgenic non-human mammal according to any one of claims 34 to 39, which is selected from the group consisting of a mouse, rat, guinea pig, hamster, rabbit, dog, cat, sheep, pig, goat, cow and monkey.

41. The transgenic non-human mammal according to any one of claims 34 to 40, which is a non-human mammal having a mutated gene on the chromosome.

42. A method for producing the transgenic non-human mammal according to any one of claims 34 to 41, which comprises the steps of constructing a transgene which comprises DNA having DNA encoding a foreign protein ligated downstream of the DNA according to any one of claims 29 to 33; microinjecting the transgene into a fertilized egg of a non-human mammal; transplanting the obtained egg to the oviduct or uterus of a pseudopregnant female non-human mammal; breeding the transplanted animal; and from the offsprings, selecting individuals having the transgene.

43. A method for producing the transgenic non-human mammal according to any one of claims 34 to 41, which comprises the steps of constructing a transgene which comprises DNA having DNA encoding a foreign protein ligated downstream of the DNA according to any one of claims 29 to 33; introducing the transgene into an embryonic stem cell of a non-human mammal; introducing the embryonic stem cell into a fertilized egg of a non-human mammal; transplanting the obtained egg to the oviduct or uterus of a pseudopregnant female non-human mammal; breeding the transplanted animal; and from the offsprings, selecting individuals having the transgene.

44. A method for producing the transgenic non-human mammal according to any one of claims 34 to 41, which comprises the steps of constructing a transgene which comprises DNA having DNA encoding a foreign protein ligated downstream of the DNA according to any one of claims 29 to 33; introducing the transgene into a cell of a non-human mammal; introducing the nucleus of the cell into an unfertilized and enucleated egg of a mammal; transplanting the obtained egg to the oviduct or uterus of a pseudopregnant female non-human mammal; breeding the transplanted animal; and from the progeny, selecting individuals having the transgene.

45. A method for producing the transgenic non-human mammal according to any one of claims 34 to 41, which comprises the steps of constructing a transgene which comprises DNA having DNA encoding a foreign protein ligated downstream of the DNA according to any one of claims 29 to 33; allowing a sperm of a non-human mammal to incorporate the transgene; allowing the sperm to fertilize an unfertilized egg; transplanting the obtained egg to the oviduct or uterus of a pseudopregnant female non-human mammal; breeding the transplanted animal; and from the offsprings, selecting individuals having the transgene.

46. A method for producing the transgenic non-human mammal according to any one of claims 34 to 41, which comprises the steps of constructing a transgene which comprises DNA having DNA encoding a foreign protein ligated downstream of the DNA according to any one of claims 29 to 33; integrating the transgene into a retrovirus vector; infecting an egg cell with virus containing the retrovirus vector; transplanting the obtained egg to the oviduct or uterus of a pseudopregnant female non-human mammal; breeding the transplanted animal; and from the offsprings, selecting individuals having the transgene.

47. An embryonic stem cell of a non-human mammal in which DNA encoding a foreign protein is introduced, which is used for producing the transgenic non-human mammal according to any one of claims 34 to 41.

48. A cell of the transgenic non-human mammal according to any one of claims 34 to 41.

49. The cell according to claim 48 which is a cell selected from the group consisting of an embryonic stem cell, an egg and a sperm.

50. A nucleus of the cell according to any one of claims 47 to 49.

51. A method for detecting changes in intracellular calcium ion concentration using a foreign reporter protein, wherein the transgenic non-human mammal according to any one of claims 35, 36, 40 and 41 expressing the foreign reporter gene, or the tissue, organ or cell of the animal is used.

52. The method according to claim 51, wherein the transgenic non-human mammal is a living transgenic non-human mammal.

53. A method for detecting changes in intracellular calcium ion concentration using a foreign reporter protein introduced in an embryonic stem cell, which comprises inducing differentiation of the embryonic stem cell according to claims 47 or 49, and detecting changes in calcium ion concentration in the cell obtained by the induction of differentiation.

54. A method for screening a substance involved in the regulation of intracellular calcium ion concentration, which comprises contacting a test substance with the transgenic non-human mammal according to claims 35, 36, 40 or 41 expressing a foreign reporter protein, or the tissue, organ or cell of the animal; and detecting changes in intracellular calcium ion concentration using the foreign reporter protein.

55. A method for detecting physiological changes, which comprises transplanting the tissue, organ or cell of the transgenic non-human mammal according to any one of claims 34 to 41 expressing the foreign reporter protein into another individual which does not immunologically reject the transplanted tissue, organ or cell; and detecting physiological changes in the transplanted tissue, organ or cell using the foreign reporter protein.

56. A method for detecting physiological changes, which comprises transplanting an embryonic stem cell according to claims 47 or 49 or a cell induced to differentiate from the embryonic stem cell into another individual which does not immunologically reject the transplanted cell; and detecting physiological changes in the transplanted cell using the foreign reporter protein introduced in the embryonic stem cell.

57. A method for producing a transgenic non-human mammal, which uses the cell according to claims 48 or 49.

58. A method for producing a cloned non-human mammal, which uses the nucleus according to claim 50.

59. A transgenic or a cloned non-human mammal which is produced by the method according to claims 57 or 58.

60. A method for producing a gene-modified mammal, which comprises mating the transgenic non-human mammal according to any one of claims 34 to 41 with an animal belonging to the same species but to a different strain from the transgenic non-human mammal.

61. A gene-modified mammal, which is obtained by the method according to claim 60.
